# EUROPEAN PATENT SPECIFICATION

(11) **EP 2 992 016 B1**
(45) Date of publication and mention of the grant of the patent: **18.10.2017**
(21) Application number: 14724662.3
(22) Date of filing: 30.04.2014
(51) Int. Cl.: C07K 16/28, A61P 35/02, A61P 35/00

(54) **COMBINATION THERAPY OF AN AFUCOSYLATED CD20 ANTIBODY WITH A CD79B ANTIBODY-DRUG CONJUGATE**
KOMBINATIONSTHERAPIE AUS EINEM AFUCOSYLIERTEN CD20-ANTIKÖRPER UND EINEM CD79B-ANTIKÖRPER-WIRKSTOFF-KONJUGAT
POLYTHÉRAPIE À BASE D'UN ANTICORPS ANTI-CD20 AFUCOSYLÉ ET D'UN CONJUGUÉ ANTICORPS ANTI-CD79B

(30) Priority: 02.05.2013 US 201361818821 P
(43) Date of publication of application: 09.03.2016
(73) Proprietor: F. Hoffmann-La Roche AG, 4070 Basel (CH)
(72) Inventor: KLEIN, Christian, CH-8906 Bonstetten (CH); LANG, Sabine, CH-8102 Oberengstringen (CH); POLSON, Andrew G., South San Francisco California 94080 (US); UMANA, Pablo, CH-8832 Wollerau (CH)
(74) Representative: Klein, Thomas
(86) International application number: PCT/EP2014/058827
(87) International publication number: WO 2014/177615

(56) References cited:
- WO-A1-2009/012268
- Anonymous: "A Randomized, Open-Label, Multicenter, Phase II Trial Evaluating the Safety and Activity of DCDT2980S in Combination With Rituximab or DCDS4501A in Combination With Rituximab in Patients With Relapsed or Refractory B-cell Non Hodgkin's Lymphoma", , 19 February 2013 (2013-02-19), pages 1-9, XP055128138, Retrieved from the Internet: URL:http://clinicaltrials.gov/archive/NCT0 1691898/2013_02_19 [retrieved on 2014-07-10]
- PALANCA-WESSELS MARIA CORINNA ET AL: "A Phase I Study of the Anti-CD79b Antibody-Drug Conjugate (ADC) DCDS4501A Targeting CD79b in Relapsed or Refractory B-Cell Non-Hodgkin's Lymphoma (NHL)", BLOOD, vol. 120, no. 21, November 2012 (2012-11), page 56, XP002731412, & 54TH ANNUAL MEETING AND EXPOSITION OF THE AMERICAN-SOCIETY-OF-HEMATOLOGY (ASH); ATLANTA, GA, USA; DECEMBER 08 -11, 2012
- LU D ET AL: "PHARMACOKINETICS (PK) OF ANTI-CD22 AND ANTI-CD79B ANTIBODY DRUG CONJUGATES (ADCS) IN RELAPSED OR REFRACTORY B-CELL NON-HODGKIN'S LYMPHOMA (NHL) PATIENTS: RESULTS FROM PHASE I DOSE-ESCALATION STUDIES", CLINICAL PHARMACOLOGY & THERAPEUTICS, vol. 93, no. Suppl. 1, February 2013 (2013-02), pages S77-S78, XP002731413, ANNUAL MEETING OF THE AMERICAN-SOCIETY-FOR-CLINICAL-PHARMACOLOGY -AND-THERAPEUTICS (ASCPT); INDIANAPOLIS, IN, USA; MARCH 05 -09, 2013
- D. DORNAN ET AL: "Therapeutic potential of an anti-CD79b antibody-drug conjugate, anti-CD79b-vc-MMAE, for the treatment of non-Hodgkin lymphoma", BLOOD, vol. 114, no. 13, 24 September 2009 (2009-09-24), pages 2721-2729, XP055075268, ISSN: 0006-4971, DOI: 10.1182/blood-2009-02-205500
- JOHN R GASDASKA ET AL: "An afucosylated anti-CD20 monoclonal antibody with greater antibody-dependent cellular cytotoxicity and B-cell depletion and lower complement-dependent cytotoxicity than rituximab", MOLECULAR IMMUNOLOGY, PERGAMON, GB, vol. 50, no. 3, 10 January 2012 (2012-01-10), pages 134-141, XP028461489, ISSN: 0161-5890, DOI: 10.1016/J.MOLIMM.2012.01.001 [retrieved on 2012-01-18]

## Description

The present invention is directed to the combination therapy of an afucosylated CD20 antibody with a CD79b antibody-drug conjugate for the treatment of cancer.

### Background of the Invention

### Afucosylated antibodies

Cell-mediated effector functions of monoclonal antibodies can be enhanced by engineering their oligosaccharide component as described in Umaña, P., et al., Nature Biotechnol. 17 (1999) 176-180; and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A., and Morrison, S.L., Trends Biotechnol. 15 (1997) 26-32). Umaña, P., et al.. Nature Biotechnol. 17 (1999) 176-180 and WO 99/154342 showed that overexpression in Chinese hamster ovary (CHO) cells of ß(1,4)-N-acetylglucosaminyltransferase III ("GnTIII"), a glycosyltransferase catalyzing the formation of bisected oligosaccharides, significantly increases the in vitro ADCC activity of antibodies. Alterations in the composition of the N297 carbohydrate or its elimination affect also binding to Fc binding to FcγR and C1 q (Umaña, P., et al., Nature Biotechnol. 17 (1999) 176-180; Davies, J., et al., Biotechnol. Bioeng. 74 (2001) 288-294; Mimura, Y., et al., J. Biol. Chem. 276 (2001) 45539-45547; Radaev, S., et al., J. Biol. Chem. 276 (2001) 16478-16483; Shields, R.L., et al., J. Biol. Chem. 276 (2001) 6591-6604; Shields, R.L., et al., J. Biol. Chem. 277 (2002) 26733-26740; Simmons, L.C., et al., J. Immunol. Methods 263 (2002) 133-147).

Studies discussing the activities of afucosylated and fucosylated antibodies, including anti-CD20 antibodies, have been reported (e.g., Iida, S., et al., Clin. Cancer Res. 12 (2006) 2879-2887; Natsume, A., et al., J. Immunol. Methods 306 (2005) 93-103; Satoh, M., et al., Expert Opin. Biol. Ther. 6 (2006) 1161-1173; Kanda, Y., et al., Biotechnol. Bioeng. 94 (2006) 680-688; Davies, J., et al., Biotechnol. Bioeng. 74 (2001) 288-294.

### CD20 and anti CD20 antibodies

The CD20 molecule (also called human B-lymphocyte-restricted differentiation antigen or Bp35) is a hydrophobic transmembrane protein located on pre-B and mature B lymphocytes that has been described extensively (Valentine, M.A., et al., J. Biol. Chem. 264 (1989) 11282-11287; and Einfeld, D.A., et al., EMBO J. 7 (1988) 711-717; Tedder, T.F., et al., Proc. Natl. Acad. Sci. U.S.A. 85 (1988) 208-212; Stamenkovic, I., et al., J. Exp. Med. 167 (1988) 1975-1980; Tedder, T.F., et al., J. Immunol. 142 (1989) 2560-2568). CD20 is expressed on greater than 90 % of B cell non-Hodgkin's lymphomas (NHL) (Anderson, K.C., et al., Blood 63 (1984) 1424-1433) but is not found on hematopoietic stem cells, pro-B cells, normal plasma cells, or other normal tissues (Tedder, T.F., et al., J, Immunol. 135 (1985) 973- 979).

There exist two different types of anti-CD20 antibodies differing significantly in their mode of CD20 binding and biological activities (Cragg, M.S., et al., Blood 103 (2004) 2738-2743; and Cragg, M.S., et al., Blood 101 (2003) 1045-1052). Type I antibodies, as, e.g., rituximab (a non-afucosylated antibody with an amount of fucose of 85 % or higher), are potent in complement mediated cytotoxicity.

Type II antibodies, as e.g. Tositumomab (B1), 11B8, AT80 or humanized B-Ly1 antibodies, effectively initiate target cell death via caspase-independent apoptosis with concomitant phosphatidylserine exposure.

### CD79b antibody-drug conjugates

CD79 is the signaling component of the B-cell receptor consisting of a covalent heterodimer containing CD79a (Igα, mb-1) and CD79b (Igβ, B29). CD79a and CD79b each contain an extracellular immunoglobulin (Ig) domain, a transmembrane domain, and an intracellular signaling domain, an immunoreceptor tyrosine-based activation motif (ITAM) domain. CD79 is expressed on B cells and in Non-Hodgkin's Lymphoma cells (NHLs) (Cabezudo et al., Haematologica, 84:413-418 (1999); D'Arena et al., Am. J. Hematol., 64: 275-281 (2000); Olejniczak et al., Immunol. Invest., 35: 93-114 (2006)). CD79a and CD79b and sIg are all required for surface expression of the CD79 (Matsuuchi et al., Curr. Opin. Immunol., 13(3): 270-7)). The average surface expression of CD79b on NHLs is similar to that on normal B-cells, but with a greater range (Matsuuchi et al., Curr. Opin. Immunol., 13(3): 270-7 (2001)).

Given the expression of CD79b, it is beneficial to produce therapeutic antibodies to the CD79b antigen that create minimal or no antigenicity when administered to patients, especially for chronic treatment. The present invention satisfies this and other needs. The present invention provides anti-CD79b antibodies that overcome the limitations of current therapeutic compositions as well as offer additional advantages that will be apparent from the detailed description below.

The use of antibody-drug conjugates (ADC), i.e. immunoconjugates, for the local delivery of cytotoxic or cytostatic agents, i.e. drugs to kill or inhibit tumor cells in the treatment of cancer (Lambert, J. (2005) Curr. Opinion in Pharmacology 5:543-549; Wu et al (2005) Nature Biotechnology 23(9):1137-1146; Payne, G. (2003) Cancer Cell 3:207-212; Syrigos and Epenetos (1999) Anticancer Research 19:605-614; Niculescu-Duvaz and Springer (1997) Adv. Drug Del. Rev. 26:151-172; US 4975278) allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al (ed.s), pp. 475-506). Efforts to improve the therapeutic index, i.e. maximal efficacy and minimal toxicity of ADC have focused on the selectivity of polyclonal (Rowland et al (1986) Cancer Immunol. Immunother., 21:183-87) and monoclonal antibodies (mAbs) as well as drug-linking and drug-releasing properties (Lambert, J. (2005) Curr. Opinion in Pharmacology 5:543-549). Drug moieties used in antibody drug conjugates include bacterial protein toxins such as diphtheria toxin, plant protein toxins such as ricin, small molecules such as auristatins, geldanamycin (Mandler et al (2000) J. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al (1996) Proc. Natl. Acad. Sci. USA 93:8618-8623), calicheamicin (Lode et al (1998) Cancer Res. 58:2928; Hinman et al (1993) Cancer Res. 53:3336-3342), daunomycin, doxorubicin, methotrexate, and vindesine (Rowland et al (1986) *supra*). The drug moieties may affect cytotoxic and cytostatic mechanisms including tubulin binding, DNA binding, or topoisomerase inhibition. Some cytotoxic drugs tend to be inactive or less active when conjugated to large antibodies or protein receptor ligands.

The auristatin peptides, auristatin E (AE) and monomethylauristatin (MMAE), synthetic analogs of dolastatin (WO 02/088172), have been conjugated as drug moieties to: (i) chimeric monoclonal antibodies cBR96 (specific to Lewis Y on carcinomas); (ii) cAC10 which is specific to CD30 on hematological malignancies (Klussman, et al (2004), Bioconjugate Chemistry 15(4):765-773; Doronina et al (2003) Nature Biotechnology 21(7):778-784; Francisco et al (2003) Blood 102(4):1458-1465; US 2004/0018194; (iii) anti-CD20 antibodies such as rituxan (WO 04/032828) for the treatment of CD20-expressing cancers and immune disorders; (iv) anti-EphB2R antibody 2H9 for treatment of colorectal cancer (Mao et al (2004) Cancer Research 64(3):781-788); (v) E-selectin antibody (Bhaskar et al (2003) Cancer Res. 63:6387-6394); (vi) trastuzumab (HERCEPTIN®, US 2005/0238649), and (vi) anti-CD30 antibodies (WO 03/043583). Variants of auristatin E are disclosed in US 5767237 and US 6124431. Monomethyl auristatin E conjugated to monoclonal antibodies are disclosed in Senter et al, Proceedings of the American Association for Cancer Research, Volume 45, Abstract Number 623, presented March 28, 2004. Auristatin analogs MMAE and MMAF have been conjugated to various antibodies (US 2005/0238649).

Conventional means of attaching, i.e. linking through covalent bonds, a drug moiety to an antibody generally leads to a heterogeneous mixture of molecules where the drug moieties are attached at a number of sites on the antibody. For example, cytotoxic drugs have typically been conjugated to antibodies through the often-numerous lysine residues of an antibody, generating a heterogeneous antibody-drug conjugate mixture. Depending on reaction conditions, the heterogeneous mixture typically contains a distribution of antibodies with from 0 to about 8, or more, attached drug moieties. In addition, within each subgroup of conjugates with a particular integer ratio of drug moieties to antibody, is a potentially heterogeneous mixture where the drug moiety is attached at various sites on the antibody. Analytical and preparative methods may be inadequate to separate and characterize the antibody-drug conjugate species molecules within the heterogeneous mixture resulting from a conjugation reaction. Antibodies are large, complex and structurally diverse biomolecules, often with many reactive functional groups. Their reactivities with linker reagents and drug-linker intermediates are dependent on factors such as pH, concentration, salt concentration, and co-solvents. Furthermore, the multistep conjugation process may be nonreproducible due to difficulties in controlling the reaction conditions and characterizing reactants and intermediates.

Cysteine thiols are reactive at neutral pH, unlike most amines which are protonated and less nucleophilic near pH 7. Since free thiol (RSH, sulfhydryl) groups are relatively reactive, proteins with cysteine residues often exist in their oxidized form as disulfide-linked oligomers or have internally bridged disulfide groups. Extracellular proteins generally do not have free thiols (Garman, 1997, Non-Radioactive Labelling: A Practical Approach, Academic Press, London, at page 55). Antibody cysteine thiol groups are generally more reactive, i.e. more nucleophilic, towards electrophilic conjugation reagents than antibody amine or hydroxyl groups. Cysteine residues have been introduced into proteins by genetic engineering techniques to form covalent attachments to ligands or to form new intramolecular disulfide bonds (Better et al (1994) J. Biol. Chem. 13:9644-9650; Bernhard et al (1994) Bioconjugate Chem. 5:126-132; Greenwood et al (1994) Therapeutic Immunology 1:247-255; Tu et al (1999) Proc. Natl. Acad. Sci USA 96:4862-4867; Kanno et al (2000) J. of Biotechnology, 76:207-214; Chmura et al (2001) Proc. Nat. Acad. Sci. USA 98(15):8480-8484; US 6248564). However, engineering in cysteine thiol groups by the mutation of various amino acid residues of a protein to cysteine amino acids is potentially problematic, particularly in the case of unpaired (free Cys) residues or those which are relatively accessible for reaction or oxidation. In concentrated solutions of the protein, whether in the periplasm of E. coli, culture supernatants, or partially or completely purified protein, unpaired Cys residues on the surface of the protein can pair and oxidize to form intermolecular disulfides, and hence protein dimers or multimers. Disulfide dimer formation renders the new Cys unreactive for conjugation to a drug, ligand, or other label. Furthermore, if the protein oxidatively forms an intramolecular disulfide bond between the newly engineered Cys and an existing Cys residue, both Cys thiol groups are unavailable for active site participation and interactions. Furthermore, the protein may be rendered inactive or non-specific, by misfolding or loss of tertiary structure (Zhang et al (2002) Anal. Biochem. 311:1-9).

Cysteine-engineered antibodies have been designed as FAB antibody fragments (thioFab) and expressed as full-length, IgG monoclonal (thioMab) antibodies (Junutula, J.R. et al. (2008) J Immunol Methods 332:41-52; US 2007/0092940). ThioFab and ThioMab antibodies have been conjugated through linkers at the newly introduced cysteine thiols with thiol-reactive linker reagents and drug-linker reagents to prepare antibody drug conjugates (Thio ADC).

A clinical trial study published under "A Randomized, Open-Label, Multicenter, Phase II Trial Evaluating the Safety and Activity of DCDT2980S in Combination With Rituximab or DCDS4501A in Combination With Rituximab in Patients With Relapsed or Refractory B-cell Non Hodgkin's Lymphoma", 2013-02-19, pages 1-9, XP055128138 describes the treatment of refractory follicular non Hodgkin's lymphoma and refractory/relapsed diffuse large cell B-cell lymphoma with an anti CD79b antibody drug-conjugate DCDS4501A in combination with rituximab.

Palanca-Wessels et al. describe in "A Phase I Study of the Anti-CD79b Antibody-Drug Conjugate (ADC) DCDS4501A Targeting CD79b in Relapsed or Refractory B-Cell Non-Hodgkin's Lymphoma (NHL)", Blood, Vol. 120, No. 21, 2012-11-08, page 56 the treatment of refractory follicular non Hodgkin's lymphoma with an anti CD79b antibody drug-conjugate DCDS4501A. The anti CD79b antibody is conjugated to MMAE.

Lu et al. describe in "Pharmacokinetics (PK) of anti-CD22 and anti-CD79B antibody drug conjugates (ADCs) in relapsed or refractory B-cell non-Hodgkin's lymphoma (NHL) patients: results from phase i dose-escalation studies", Clinical Pharmacology & Therapeutics, Vol. 93, No. Suppl. 1, 2013-02, pages S77-S78, Annual Meeting of the American Society for Clinical Pharmacology and Therapeutics (ASCPT) the treatment of refractory B-cell non Hodgkin's lymphoma with an anti CD79b antibody drug-conjugate DCDS4501A in combination with rituximab (abstract, methods). Continuation in phase II is proposed.

WO2009/012268 describes anti CD79b antibodies having the same sequences as the presently used antibodies. It proposes their combined use of an anti CD79b antibody drug conjugate with an anti CD20 antibody such as rituximab for treating leukemia or lymphomas (paragraph 200).

### Summary of the Invention

We have now found out that the combination of an afucosylated anti-CD20 antibody with a CD79b antibody-drug conjugate showed significantly enhanced antiproliferative effects.

One aspect of the invention is an afucosylated anti-CD20 antibody with an amount of fucose of 60% or less of the total amount of oligosaccharides (sugars) at Asn297, for the treatment of cancer in combination with a CD79b antibody-drug conjugate.

Another aspect of the invention is the use of an afucosylated anti-CD20 antibody with an amount of fucose of 60% or less of the total amount of oligosaccharides (sugars) at Asn297, for the manufacture of a medicament for the treatment of cancer in combination with a CD79b antibody-drug conjugate.

Another aspect of the invention is a method of treatment of patient suffering from cancer by administering an afucosylated anti-CD20 antibody with an amount of fucose of 60% or less of the total amount of oligosaccharides (sugars) at Asn297, in combination with a CD79b antibody-drug conjugate, to a patient in the need of such treatment.

In one aspect, the amount of fucose is between 40% and 60% of the total amount of oligosaccharides (sugars) at Asn297. In another aspect, the amount of fucose is 0% of the total amount of oligosaccharides (sugars) at Asn297.

In one embodiment, the afucosylated anti-CD20 antibody is an IgG1 antibody. In another embodiment, said cancer is a CD20 expressing cancer, preferably a lymphoma or lymphocytic leukemia. In one embodiment said afucosylated anti-CD20 antibody is a humanized B-Ly1 antibody. In a specific embodiment, the anti-CD20 antibody is obinutuzumab (recommended INN, WHO Drug Information, Vol. 26, No. 4, 2012, p. 453). As used herein, obinutuzumab is synonymous for GA101. This replaces all previous versions (e.g. Vol. 25, No. 1, 2011, p.75-76), and is formerly known as afutuzumab (recommended INN, WHO Drug Information, Vol. 23, No. 2, 2009, p. 176;Vol. 22, No. 2, 2008, p. 124).

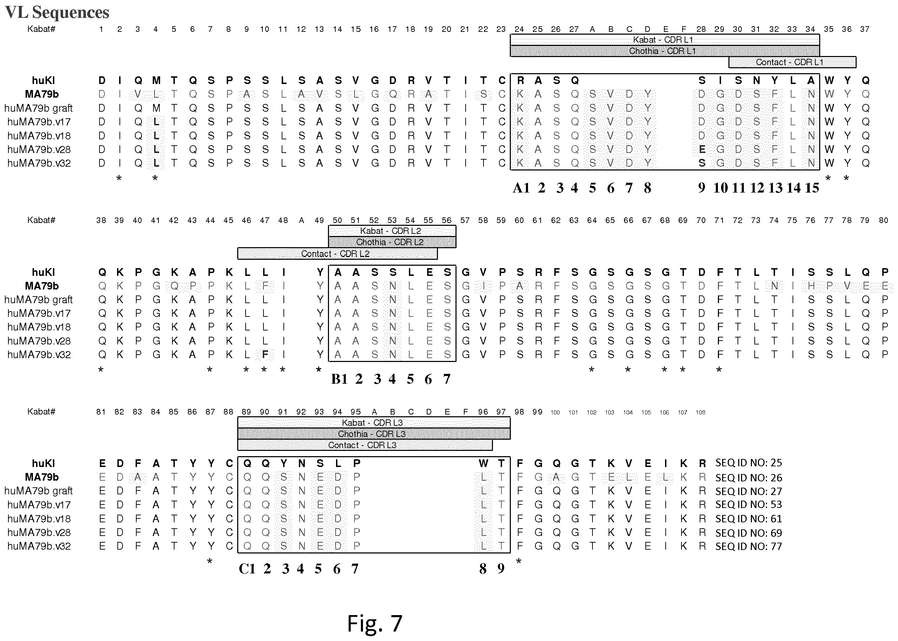

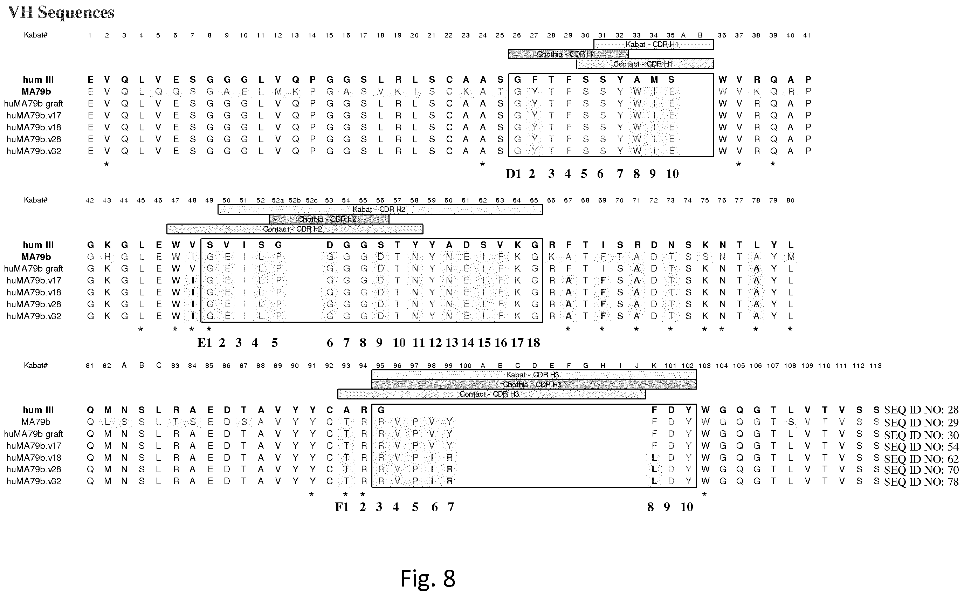

In one aspect, the CD79b antibody in the CD79b antibody-drug conjugate invention is a humanized anti-CD79b antibody wherein the monovalent affinity (e.g affinity of the antibody as a Fab fragment to CD79b) or affinity in its bivalent form of the antibody to CD79b (e.g. affinity of the antibody as an IgG fragment to CD79b) is substantially the same as, lower than, or greater than, the monovalent affinity or affinity in its bivalent form, respectively, of a murine antibody (e.g. affinity of the murine antibody as a Fab fragment or as an IgG fragment to CD79b) or a chimeric antibody (e.g. affinity of the chimeric antibody as a Fab fragment or as an IgG fragment to CD79b), comprising, consisting or consisting essentially of a light chain and heavy chain variable domain sequence as as depicted in Figure 7 (SEQ ID NO: 26) and in Figure 8 (SEQ ID NO: 29).

In one aspect, the CD79b antibody in the CD79b antibody-drug conjugate invention is a humanized anti-CD79b antibody wherein the affinity of the antibody in its bivalent form to CD79b (e.g., affinity of the antibody as an IgG to CD79b) is 0.4 nM, 0.2 nM or 0.5 nM.

In one embodiment, the CD79b antibody in the CD79b antibody-drug conjugate comprises at least one, two, three, four, five or six HVRs selected from the group consisting of:
(i) HVR-L1 comprising sequence A1-A15, wherein A1-A15 is KASQSVDYDGDSFLN (SEQ ID NO: 31)
(ii) HVR-L2 comprising sequence B1-B7, wherein B1-B7 is AASNLES (SEQ ID NO: 32)
(iii) HVR-L3 comprising sequence C1-C9, wherein C1-C9 is QQSNEDPLT (SEQ ID NO: 33)
(iv) HVR-H1 comprising sequence D1-D10, wherein D1-D10 is GYTFSSYWIE (SEQ ID NO: 34)
(v) HVR-H2 comprising sequence E1-E18, wherein E1-E18 is GEILPGGGDTNYNEIFKG (SEQ ID NO: 35) and
(vi) HVR-H3 comprising sequence F1-F10, wherein F1-F10 IS TRRVPVYFDY (SEQ ID NO: 36).

In one aspect, an antibody that binds to CD79b in the CD79b antibody-drug conjugate according to the invention comprises at least one variant HVR wherein the variant HVR sequence comprises modification of at least one residue of the sequence depicted in SEQ ID NOs: 31, 32, 33, 34, 35 or 36.

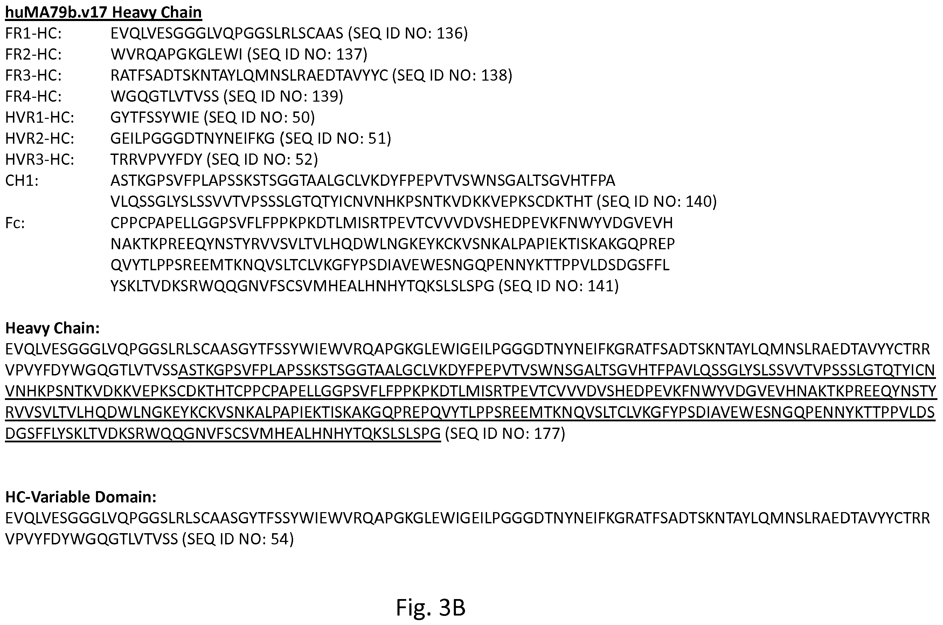

In one aspect, the invention provides an antibody in the CD79b antibody-drug conjugate according to the invention comprising a heavy chain variable domain comprising the HVR1-HC, HVR2-HC and/or HVR3-HC sequence depicted in Figure 3B (SEQ ID NO: 50-52).

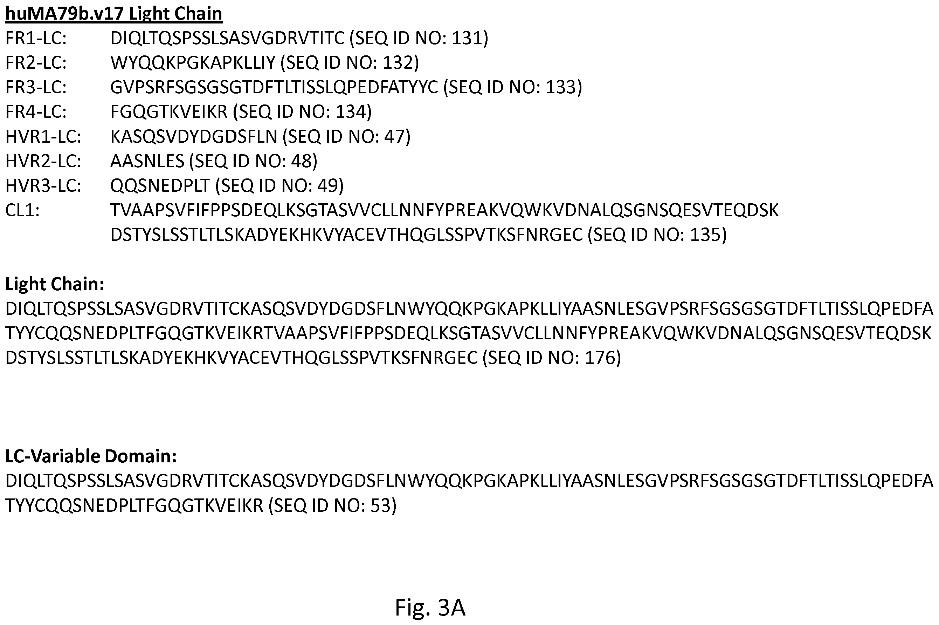

In one aspect, the invention provides an antibody in the CD79b antibody-drug conjugate according to the invention comprising a light chain variable domain comprising HVR1-LC, HVR2-LC and/or HVR3-LC sequence depicted in Figure 3A (SEQ ID NO: 47-49).

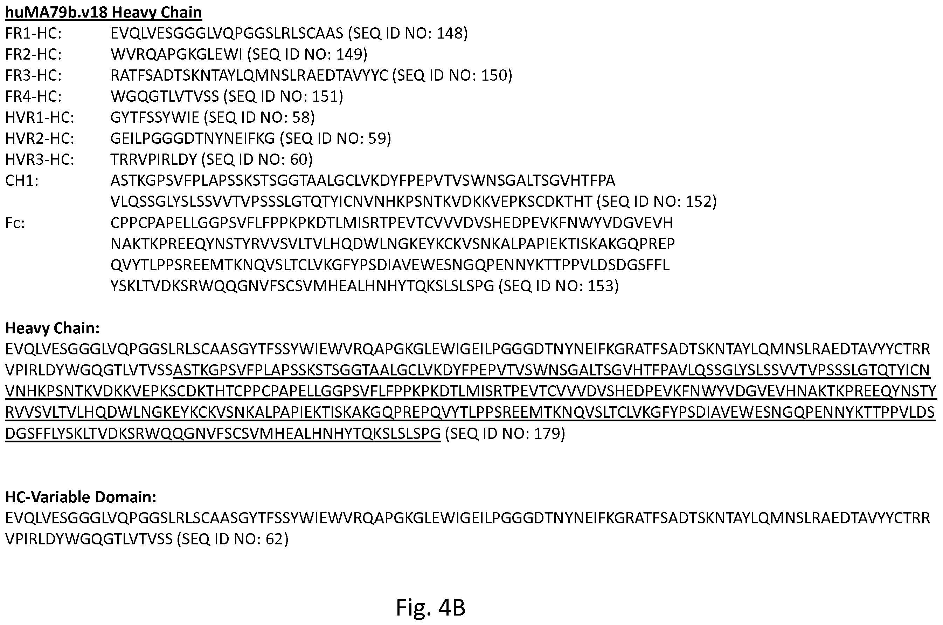

In one aspect, the invention provides an antibody in the CD79b antibody-drug conjugate according to the invention comprising a heavy chain variable domain comprising the HVR1-HC, HVR2-HC and/or HVR3-HC sequence depicted in Figure 4B (SEQ ID NO: 58-60).

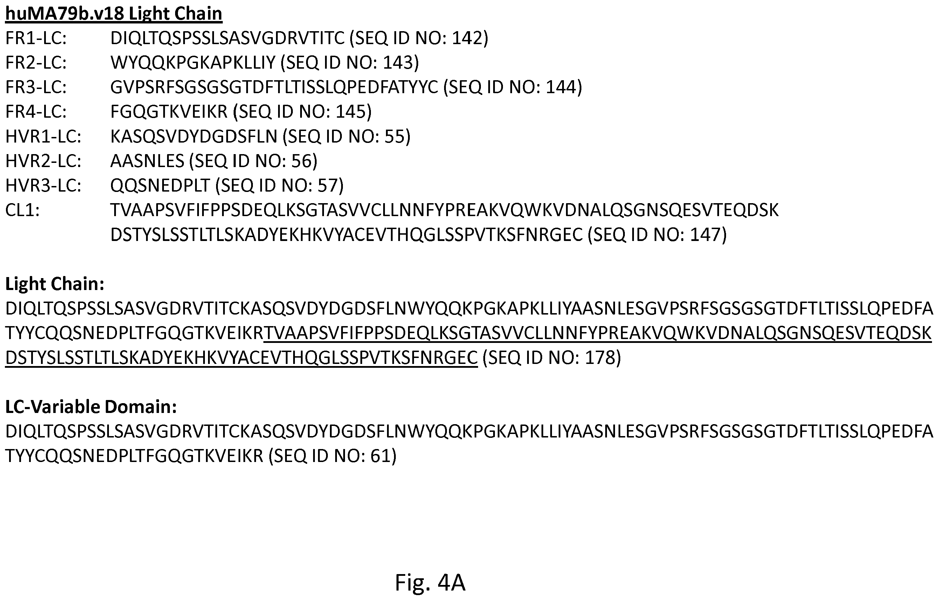

In one aspect, the invention provides an antibody in the CD79b antibody-drug conjugate according to the invention comprising a light chain variable domain comprising HVR1-LC, HVR2-LC and/or HVR3-LC sequence depicted in Figure 4A (SEQ ID NO: 55-57).

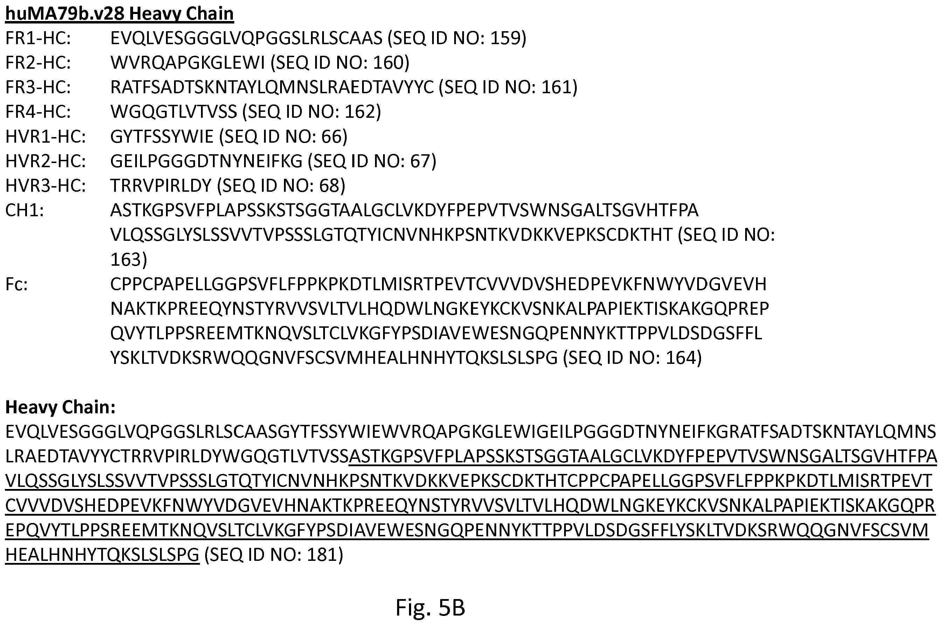

In one aspect, the invention provides an antibody in the CD79b antibody-drug conjugate according to the invention comprising a heavy chain variable domain comprising the HVR1-HC, HVR2-HC and/or HVR3-HC sequence depicted in Figure 5B (SEQ ID NO: 66-68).

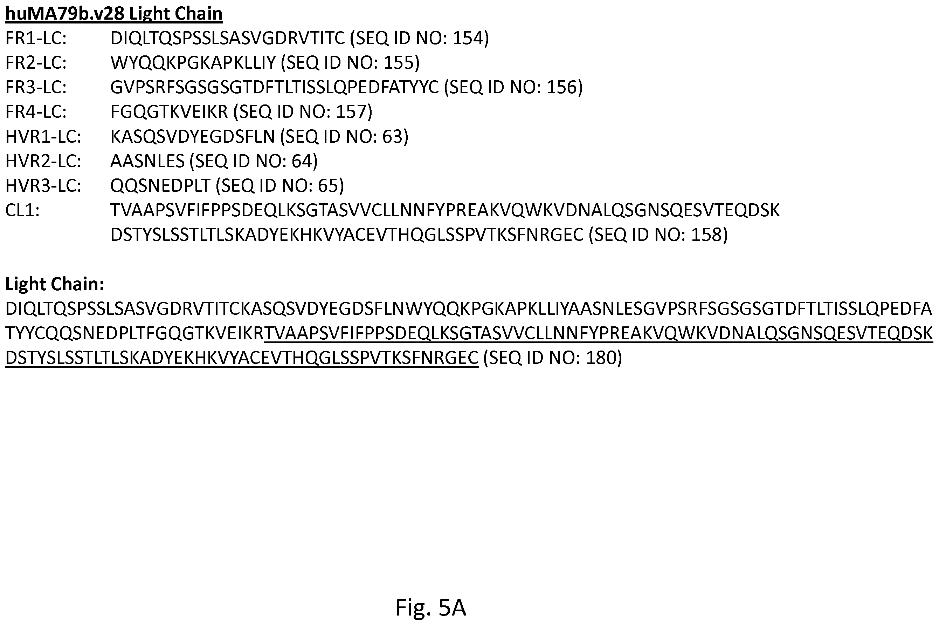

In one aspect, the invention provides an antibody in the CD79b antibody-drug conjugate according to the invention comprising a light chain variable domain comprising HVR1-LC, HVR2-LC and/or HVR3-LC sequence depicted in Figure 5A (SEQ ID NO: 63-65).

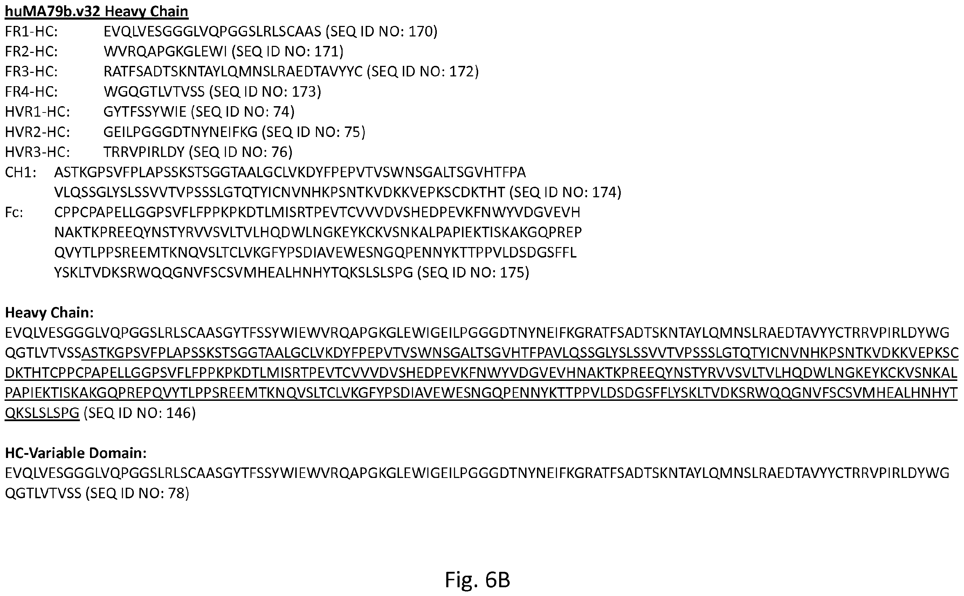

In one aspect, the invention provides an antibody in the CD79b antibody-drug conjugate according to the invention comprising a heavy chain variable domain comprising the HVR1-HC, HVR2-HC and/or HVR3-HC sequence depicted in Figure 6B (SEQ ID NO: 74-76).

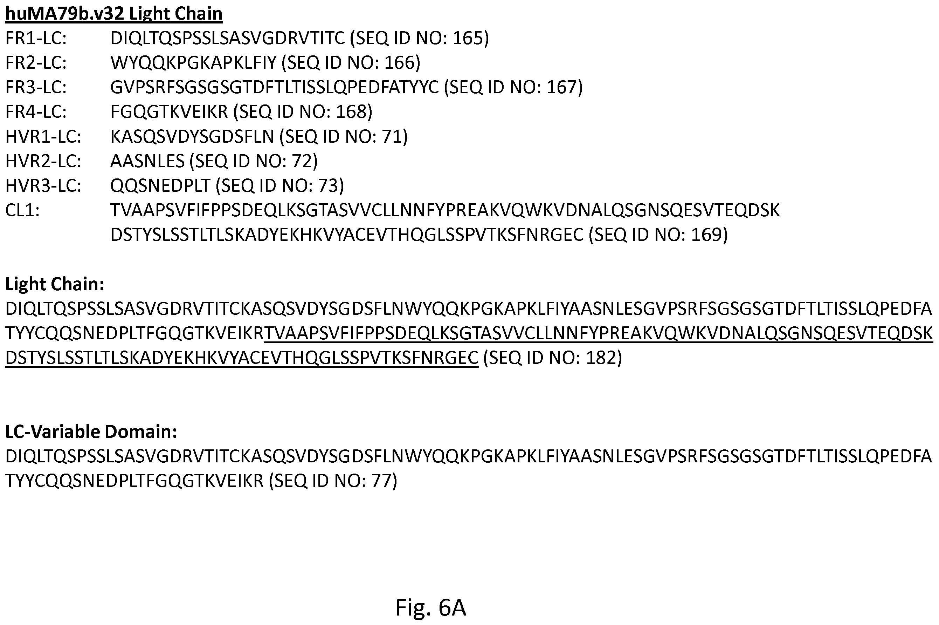

In one aspect, the invention provides an antibody in the CD79b antibody-drug conjugate according to the invention comprising a light chain variable domain comprising HVR1-LC, HVR2-LC and/or HVR3-LC sequence depicted in Figure 6A (SEQ ID NO: 71-73).

In one aspect, the invention includes an anti-CD79b antibody in the CD79b antibody-drug conjugate according to the invention comprising a heavy chain variable domain selected from SEQ ID NOs: 54, 62, 70 or 78. In another aspect, the invention includes an anti-CD79b antibody in the CD79b antibody-drug conjugate according to the invention comprising a light chain variable domain selected from SEQ ID NOs: 53, 61, 69 or 77.

In one aspect, the invention includes a cysteine engineered anti-CD79b antibody in the CD79b antibody-drug conjugate according to the invention comprising one or more free cysteine amino acids and a sequence selected from SEQ ID NOs: 83-130. The cysteine engineered anti-CD79b antibody in the CD79b antibody-drug conjugate according to the invention may bind to a CD79b polypeptide. The cysteine engineered anti-CD79b antibody in the CD79b antibody-drug conjugate according to the invention may be prepared by a process comprising replacing one or more amino acid residues of a parent anti-CD79b antibody by cysteine.

In one aspect, the invention includes a cysteine engineered anti-CD79b antibody in the CD79b antibody-drug conjugate according to the invention comprising one or more free cysteine amino acids wherein the cysteine engineered anti-CD79b antibody binds to a CD79b polypeptide and is prepared by a process comprising replacing one or more amino acid residues of a parent anti-CD79b antibody by cysteine wherein the parent antibody comprises at least one HVR sequence selected from:
(i) HVR-L1 comprising sequence A1-A15, wherein A1-A15 is KASQSVDYDGDSFLN (SEQ ID NO: 31) or KASQSVDYEGDSFLN (SEQ ID NO: 37);
(ii) HVR-L2 comprising sequence B1-B7, wherein B1-B7 is AASNLES (SEQ ID NO: 32);
(iii) HVR-L3 comprising sequence C1-C9, wherein C1-C9 is QQSNEDPLT (SEQ ID NO: 33);
(iv) HVR-H1 comprising sequence D1-D10, wherein D1-D10 is GYTFSSYWIE (SEQ ID NO: 34);
(v) HVR-H2 comprising sequence E1-E18, wherein E1-E18 is GEILPGGGDTNYNEIFKG (SEQ ID NO: 35) and
(vi) HVR-H3 comprising sequence F1-F10, wherein F1-F10 is TRRVPVYFDY (SEQ ID NO: 36) or TRRVPIRLDY (SEQ ID NO: 38).

In one aspect, the CD79b antibody in the CD79b antibody-drug conjugate comprises a variable light chain sequence selected from the group consisting of light chain human kappa I consensus sequence (labeled as "huKI"; SEQ ID NO: 25) with VL-FR1, VL-FR2, VL-FR3, VL-FR4 (SEQ ID NOs: 39-42, respectively), murine anti-CD79b antibody (labeled as "MA79b"; SEQ ID NO: 26), MA79b-grafted "humanized" antibody (labeled as "huMA79b graft"; SEQ ID NO: 27), MA79b-grated "humanized" antibody variant 17 (labeled as "huMA79b.v17"; SEQ ID NO: 53), MA79b-grafted "humanized" antibody variant 18 (labeled as "huMA79b.v18"; SEQ ID NO: 61), MA79b-grafted "humanized" antibody variant 28 (labeled as "huMA79b.v28"; SEQ ID NO: 69) and MA79b-grafted "humanized" antibody variant 32 (labeled as "huMA79b.v32"; SEQ ID NO: 77).

In one aspect, the CD79b antibody in the CD79b antibody-drug conjugate invention comprises a variable heavy chain sequence selected from the group consisting of: heavy chain human subgroup III consensus sequence (labeled as "humIII"; SEQ ID NO: 28) with VH-FR1, VH-FR2, VH-FR3, and VH-FR4 (SEQ ID NOs: 43-46), murine anti-CD79b antibody (labeled as "MA79b"; SEQ ID NO: 29), MA79b-grafted "humanized" antibody (labeled as "huMA79b graft"; SEQ ID NO: 30) (containing 71A, 73T and 78A), MA79b-grated "humanized" antibody variant 17 (labeled as "huMA79b.v17"; SEQ ID NO: 54) (containing 71A, 73T and 78A), MA79b-grafted "humanized" antibody variant 18 (labeled as "huMA79b.v18"; SEQ ID NO: 62) (containing 71A, 73T and 78A), MA79b-grafted "humanized" antibody variant 28 (labeled as "huMA79b.v28"; SEQ ID NO: 70) (containing 71A, 73T and 78A) and MA79b-grafted "humanized" antibody variant 32 (labeled as "huMA79b.v32"; SEQ ID NO: 78) (containing 71A, 73T and 78A).

In one embodiment, the CD79b antibody in the CD79b antibody-drug conjugate comprises a cysteine engineered anti-CD79b antibody comprising one or more free cysteine amino acids wherein the cysteine engineered anti-CD79b antibody binds to a CD79b polypeptide and is prepared by a process comprising replacing one or more amino acid residues of a parent anti-CD79b antibody by cysteine wherein the parent antibody comprises at least one HVR sequence selected from:
(a) HVR-L1 comprising sequence A1-A15, wherein A1-A15 is KASQSVDYDGDSFLN (SEQ ID NO: 31) or KASQSVDYEGDSFLN (SEQ ID NO: 37);
(b) HVR-L2 comprising sequence B1-B7, wherein B1-B7 is AASNLES (SEQ ID NO: 32)
(c) HVR-L3 comprising sequence C1-C9, wherein C1-C9 is QQSNEDPLT (SEQ ID NO: 33)
(d) HVR-H1 comprising sequence D1-D10, wherein D1-D10 is GYTFSSYWIE (SEQ ID NO: 34)
(e) HVR-H2 comprising sequence E1-E18, wherein E1-E18 is GEILPGGGDTNYNEIFKG (SEQ ID NO: 35) and
(f) HVR-H3 comprising sequence F1-F10, wherein F1-F10 is TRRVPVYFDY (SEQ ID NO: 36) or TRRVPIRLDY (SEQ ID NO: 38).

In one embodiment, the CD79b antibody-drug conjugate having the formula Ab-(L-D)p (Formula I), wherein
(a) Ab is the CD79b antibody as defined herein;
(b) L is a linker;
(c) D is a drug moiety.

Accordingly, the antibody may be conjugated to the drug either directly or via a linker. In Formula I, p is the average number of drug moieties per antibody, which can range, e.g., from about 1 to about 20 drug moieties per antibody, and in certain embodiments, from 1 to about 8 drug moieties per antibody. The invention includes a composition comprising a mixture of antibody-drug compounds of Formula I where the average drug loading per antibody is about 2 to about 5, or about 3 to about 4.

In one embodiment of the CD79b antibody-drug conjugate having the formula Ab-(L-D)p, L is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), N-succinimidyl 4-(N-maleimidomethyl) cyclohexane-1 carboxylate (SMCC), and N-Succinimidyl (4-iodo-acetyl) aminobenzoate (SIAB).

In one embodiment of the CD79b antibody-drug conjugate having the formula Ab-(L-D)p, D is selected from the group consisting of auristatin, dolostantin, DM1, DM3, DM4, MMAE and MMAF.

In one embodiment, said CD79b antibody-drug conjugate is anti-CD79b-MC-vc-PAB-MMAE. In a specific embodiment, the anti-CD79b antibody in said conjugate is huMA79b.v28.

In one embodiment, the afucosylated anti-CD20 antibody binds CD20 with an KD of 10⁻⁸ M to 10⁻¹³ M.

One embodiment of the invention is a composition comprising an afucosylated anti-CD20 antibody with an amount of fucose of 60% or less of the total amount of oligosaccharides (sugars) at Asn297, (in one embodiment an afucosylated humanized B-Ly1 antibody), and a CD79b antibody-drug conjugate. In one embodiment of the composition according to the invention, said anti-CD20 antibody is obinutuzumab.

In one embodiment of the composition according to the invention, said CD79b antibody in the CD79b antibody-drug conjugate comprises at least one, two, three, four, five or six HVRs selected from the group consisting of:
(i) HVR-L1 comprising sequence A1-A15, wherein A1-A15 is KASQSVDYDGDSFLN (SEQ ID NO: 31)
(ii) HVR-L2 comprising sequence B1-B7, wherein B1-B7 is AASNLES (SEQ ID NO: 32)
(iii) HVR-L3 comprising sequence C1-C9, wherein C1-C9 is QQSNEDPLT (SEQ ID NO: 33)
(iv) HVR-H1 comprising sequence D1-D10, wherein D1-D10 is GYTFSSYWIE (SEQ ID NO: 34)
(v) HVR-H2 comprising sequence E1-E18, wherein E1-E18 is GEILPGGGDTNYNEIFKG (SEQ ID NO: 35) and
(vi) HVR-H3 comprising sequence F1-F10, wherein F1-F10 IS TRRVPVYFDY (SEQ ID NO: 36).

In one aspect of the composition according to the invention, the CD79b antibody in the CD79b antibody-drug conjugate comprises a variable light chain sequence selected from the group consisting of light chain human kappa I consensus sequence (labeled as "huKI"; SEQ ID NO: 25) with VL-FR1, VL-FR2, VL-FR3, VL-FR4 (SEQ ID NOs: 39-42, respectively), murine anti-CD79b antibody (labeled as "MA79b"; SEQ ID NO: 26), MA79b-grafted "humanized" antibody (labeled as "huMA79b graft"; SEQ ID NO: 27), MA79b-grated "humanized" antibody variant 17 (labeled as "huMA79b.v17"; SEQ ID NO: 53), MA79b-grafted "humanized" antibody variant 18 (labeled as "huMA79b.v18"; SEQ ID NO: 61), MA79b-grafted "humanized" antibody variant 28 (labeled as "huMA79b.v28"; SEQ ID NO: 69) and MA79b-grafted "humanized" antibody variant 32 (labeled as "huMA79b.v32"; SEQ ID NO: 77).

In one aspect of the composition according to the invention, the CD79b antibody in the CD79b antibody-drug conjugate comprises a variable heavy chain sequence selected from the group consisting of: heavy chain human subgroup III consensus sequence (labeled as "humIII"; SEQ ID NO: 28) with VH-FR1, VH-FR2, VH-FR3, and VH-FR4 (SEQ ID NOs: 43-46), murine anti-CD79b antibody (labeled as "MA79b"; SEQ ID NO: 29), MA79b-grafted "humanized" antibody (labeled as "huMA79b graft"; SEQ ID NO: 30) (containing 71A, 73T and 78A), MA79b-grated "humanized" antibody variant 17 (labeled as "huMA79b.v17"; SEQ ID NO: 54) (containing 71A, 73T and 78A), MA79b-grafted "humanized" antibody variant 18 (labeled as "huMA79b.v18"; SEQ ID NO: 62) (containing 71A, 73T and 78A), MA79b-grafted "humanized" antibody variant 28 (labeled as "huMA79b.v28"; SEQ ID NO: 70) (containing 71A, 73T and 78A) and MA79b-grafted "humanized" antibody variant 32 (labeled as "huMA79b.v32"; SEQ ID NO: 78) (containing 71A, 73T and 78A).

In one embodiment of the composition according to the invention, one or more additional other cytotoxic, chemotherapeutic or anti-cancer agents, or compounds or ionizing radiation that enhance the effects of such agents are administered.

In one embodiment of the composition according to the invention, the CD79b antibody-drug conjugate is having the formula Ab-(L-D)p, wherein
(a) Ab is the CD79b antibody as defined herein;
(b) L is a linker;
(c) D is a drug moiety.

In one embodiment of the composition according to the invention, the CD79b antibody-drug conjugate is having the formula Ab-(L-D)p, wherein L is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), N-succinimidyl 4-(N-maleimidomethyl) cyclohexane-1 carboxylate (SMCC), and N-Succinimidyl (4-iodo-acetyl) aminobenzoate (SIAB).

In one embodiment of the composition according to the invention, the CD79b antibody-drug conjugate is having the formula Ab-(L-D)p, wherein D is selected from the group consisting of auristatin, dolostantin, DM1, DM3, DM4, MMAE and MMAF.

In one embodiment of the composition according to the invention, the CD79b antibody-drug conjugate is is anti-CD79b-MC-vc-PAB-MMAE for the treatment of cancer. In a specific embodiment, the anti-CD79b antibody in said conjugate is huMA79b.v28. An afucosylated anti-CD20 antibody with an amount of fucose of 60% or less of the total amount of oligosaccharides (sugars) at Asn297, for the treatment of cancer in combination with a CD79b antibody-drug conjugate.

One aspect of the invention is a method of treatment of patient suffering from cancer by administering an afucosylated anti-CD20 antibody with an amount of fucose of 60% or less of the total amount of oligosaccharides (sugars) at Asn297, in combination with a CD79b antibody-drug conjugate, to a patient in the need of such treatment.

In one aspect according to the invention, the method is characterized in that said cancer is a CD20 expressing cancer.

In one aspect according to the invention, the method is characterized in that said CD20 expressing cancer is a lymphoma or lymphocytic leukemia.

In one aspect according to the invention, the method is characterized in that said anti-CD20 antibody is a humanized B-Ly1 antibody.

In one aspect according to the invention, the method is characterized in that said anti-CD20 antibody is obinutuzumab.

In one aspect according to the invention, the method is characterized in that one or more additional other cytotoxic, chemotherapeutic or anti-cancer agents, or compounds or ionizing radiation that enhance the effects of such agents are administered.

In one aspect according to the invention, the method is characterized in that said CD79b antibody in the CD79b antibody-drug conjugate comprises at least one, two, three, four, five or six HVRs selected from the group consisting of:
(i) HVR-L1 comprising sequence A1-A15, wherein A1-A15 is KASQSVDYDGDSFLN (SEQ ID NO: 31)
(ii) HVR-L2 comprising sequence B1-B7, wherein B1-B7 is AASNLES (SEQ ID NO: 32)
(iii) HVR-L3 comprising sequence C1-C9, wherein C1-C9 is QQSNEDPLT (SEQ ID NO: 33)
(iv) HVR-H1 comprising sequence D1-D10, wherein D1-D10 is GYTFSSYWIE (SEQ ID NO: 34)
(v) HVR-H2 comprising sequence E1-E18, wherein E1-E18 is GEILPGGGDTNYNEIFKG (SEQ ID NO: 35) and
(vi) HVR-H3 comprising sequence F1-F10, wherein F1-F10 IS TRRVPVYFDY (SEQ ID NO: 36).

In one aspect of the method according to the invention, the CD79b antibody in the CD79b antibody-drug conjugate comprises a variable light chain sequence selected from the group consisting of light chain human kappa I consensus sequence (labeled as "huKI"; SEQ ID NO: 25) with VL-FR1, VL-FR2, VL-FR3, VL-FR4 (SEQ ID NOs: 39-42, respectively), murine anti-CD79b antibody (labeled as "MA79b"; SEQ ID NO: 26), MA79b-grafted "humanized" antibody (labeled as "huMA79b graft"; SEQ ID NO: 27), MA79b-grated "humanized" antibody variant 17 (labeled as "huMA79b.v17"; SEQ ID NO: 53), MA79b-grafted "humanized" antibody variant 18 (labeled as "huMA79b.v18"; SEQ ID NO: 61), MA79b-grafted "humanized" antibody variant 28 (labeled as "huMA79b.v28"; SEQ ID NO: 69) and MA79b-grafted "humanized" antibody variant 32 (labeled as "huMA79b.v32"; SEQ ID NO: 77).

In one aspect of the method according to the invention, the CD79b antibody in the CD79b antibody-drug conjugate comprises a variable heavy chain sequence selected from the group consisting of: heavy chain human subgroup III consensus sequence (labeled as "humIII"; SEQ ID NO: 28) with VH-FR1, VH-FR2, VH-FR3, and VH-FR4 (SEQ ID NOs: 43-46), murine anti-CD79b antibody (labeled as "MA79b"; SEQ ID NO: 29), MA79b-grafted "humanized" antibody (labeled as "huMA79b graft"; SEQ ID NO: 30) (containing 71A, 73T and 78A), MA79b-grated "humanized" antibody variant 17 (labeled as "huMA79b.v17"; SEQ ID NO: 54) (containing 71A, 73T and 78A), MA79b-grafted "humanized" antibody variant 18 (labeled as "huMA79b.v18"; SEQ ID NO: 62) (containing 71A, 73T and 78A), MA79b-grafted "humanized" antibody variant 28 (labeled as "huMA79b.v28"; SEQ ID NO: 70) (containing 71A, 73T and 78A) and MA79b-grafted "humanized" antibody variant 32 (labeled as "huMA79b.v32"; SEQ ID NO: 78) (containing 71A, 73T and 78A).

In one aspect of the method according to the invention, the CD79b antibody-drug conjugate is having the formula Ab-(L-D)p, wherein
(a) Ab is the CD79b antibody as disclosed herein;
(b) L is a linker;
(c) D is a drug moiety.

In one aspect of the method according to the invention, the CD79b antibody-drug conjugate is having the formula Ab-(L-D)p, wherein L is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), N-succinimidyl 4-(N-maleimidomethyl) cyclohexane-1 carboxylate (SMCC), and N-Succinimidyl (4-iodo-acetyl) aminobenzoate (SIAB).

In one aspect of the method according to the invention, the CD79b antibody-drug conjugate is having the formula Ab-(L-D)p, wherein D is selected from the group consisting of auristatin, dolostantin, DM1, DM3, DM4, MMAE and MMAF.

In one aspect of the method according to the invention, the CD79b antibody-drug conjugate is anti-CD79b-MC-vc-PAB-MMAE.

In one aspect of the method according to the invention, the anti-CD79b antibody in said CD79b antibody-drug conjugate is huMA79b.v28.

One embodiment according to the invention is the use of an afucosylated anti-CD20 antibody with an amount of fucose of 60% or less of the total amount of oligosaccharides (sugars) at Asn297, for the manufacture of a medicament for the treatment of cancer in combination with a CD79b antibody-drug conjugate.

One embodiment of the use according to the invention is characterized in that said cancer is a CD20 expressing cancer.

One embodiment of the use according to the invention is characterized in that said CD20 expressing cancer is a lymphoma or lymphocytic leukemia.

One embodiment of the use according to the invention is characterized in that said anti-CD20 antibody is a humanized B-Ly1 antibody.

One embodiment of the use according to the invention is characterized in that said anti-CD20 antibody is obinutuzumab.

One embodiment of the use according to the invention is characterized in that one or more additional other cytotoxic, chemotherapeutic or anti-cancer agents, or compounds or ionizing radiation that enhance the effects of such agents are administered.

One embodiment of the use according to the invention is characterized characterized in that said CD79b antibody in the CD79b antibody-drug conjugate comprises at least one, two, three, four, five or six HVRs selected from the group consisting of:
(i) HVR-L1 comprising sequence A1-A15, wherein A1-A15 is KASQSVDYDGDSFLN (SEQ ID NO: 31)
(ii) HVR-L2 comprising sequence B1-B7, wherein B1-B7 is AASNLES (SEQ ID NO: 32)
(iii) HVR-L3 comprising sequence C1-C9, wherein C1-C9 is QQSNEDPLT (SEQ ID NO: 33)
(iv) HVR-H1 comprising sequence D1-D10, wherein D1-D10 is GYTFSSYWIE (SEQ ID NO: 34)
(v) HVR-H2 comprising sequence E1-E18, wherein E1-E18 is GEILPGGGDTNYNEIFKG (SEQ ID NO: 35) and
(vi) HVR-H3 comprising sequence F1-F10, wherein F1-F10 IS TRRVPVYFDY (SEQ ID NO: 36).

In one aspect of the use according to the invention, the CD79b antibody in the CD79b antibody-drug conjugate comprises a variable light chain sequence selected from the group consisting of light chain human kappa I consensus sequence (labeled as "huKI"; SEQ ID NO: 25) with VL-FR1, VL-FR2, VL-FR3, VL-FR4 (SEQ ID NOs: 39-42, respectively), murine anti-CD79b antibody (labeled as "MA79b"; SEQ ID NO: 26), MA79b-grafted "humanized" antibody (labeled as "huMA79b graft"; SEQ ID NO: 27), MA79b-grated "humanized" antibody variant 17 (labeled as "huMA79b.v17"; SEQ ID NO: 53), MA79b-grafted "humanized" antibody variant 18 (labeled as "huMA79b.v18"; SEQ ID NO: 61), MA79b-grafted "humanized" antibody variant 28 (labeled as "huMA79b.v28"; SEQ ID NO: 69) and MA79b-grafted "humanized" antibody variant 32 (labeled as "huMA79b.v32"; SEQ ID NO: 77).

In one aspect of the use according to the invention, the CD79b antibody in the CD79b antibody-drug conjugate comprises a variable heavy chain sequence selected from the group consisting of: heavy chain human subgroup III consensus sequence (labeled as "humIII"; SEQ ID NO: 28) with VH-FR1, VH-FR2, VH-FR3, and VH-FR4 (SEQ ID NOs: 43-46), murine anti-CD79b antibody (labeled as "MA79b"; SEQ ID NO: 29), MA79b-grafted "humanized" antibody (labeled as "huMA79b graft"; SEQ ID NO: 30) (containing 71A, 73T and 78A), MA79b-grated "humanized" antibody variant 17 (labeled as "huMA79b.v17"; SEQ ID NO: 54) (containing 71A, 73T and 78A), MA79b-grafted "humanized" antibody variant 18 (labeled as "huMA79b.v18"; SEQ ID NO: 62) (containing 71A, 73T and 78A), MA79b-grafted "humanized" antibody variant 28 (labeled as "huMA79b.v28"; SEQ ID NO: 70) (containing 71A, 73T and 78A) and MA79b-grafted "humanized" antibody variant 32 (labeled as "huMA79b.v32"; SEQ ID NO: 78) (containing 71A, 73T and 78A).

One embodiment of the use according to the invention is characterized in that the CD79b antibody-drug conjugate is having the formula Ab-(L-D)p, wherein
(a) Ab is the CD79b antibody as disclosed herein;
(b) L is a linker;
(c) D is a drug moiety.

One embodiment of the use according to the invention is characterized in that the CD79b antibody-drug conjugate is having the formula Ab-(L-D)p, wherein L is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio) pentanoate (SPP), N-succinimidyl 4-(N-maleimidomethyl) cyclohexane-1 carboxylate (SMCC), and N-Succinimidyl (4-iodo-acetyl) aminobenzoate (SIAB).

One embodiment of the use according to the invention is characterized in that the CD79b antibody-drug conjugate is having the formula Ab-(L-D)p, wherein D is selected from the group consisting of auristatin, dolostantin, DM1, DM3, DM4, MMAE and MMAF.

One embodiment of the use according to the invention is characterized in that the CD79b antibody-drug conjugate is anti-CD79b-MC-vc-PAB-MMAE.

One embodiment of the use according to the invention is characterized in that the anti-CD79b antibody in said CD79b antibody-drug conjugate is huMA79b.v28.

One embodiment of the use according to the invention is characterized in that one or more additional other cytotoxic, chemotherapeutic or anti-cancer agents, or compounds or ionizing radiation that enhance the effects of such agents are administered.

### Description of the Figures

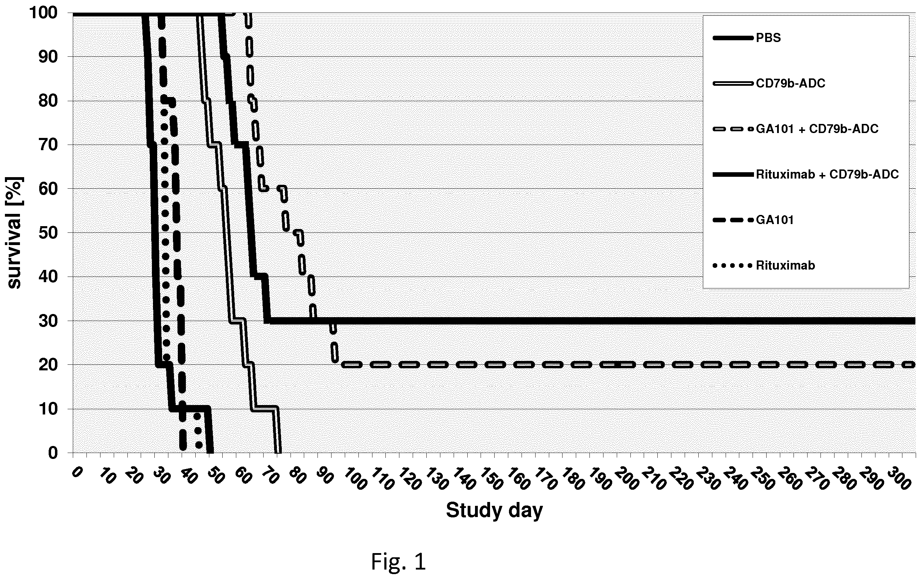

**Figure 1****:** Effect of obinutuzumab (GA101), rituximab, anti-CD79b-ADC and the combinations of CD79b with GA101 or rituximab on a disseminated Z138 mantle cell lymphoma (MCL) model in SCID beige mice

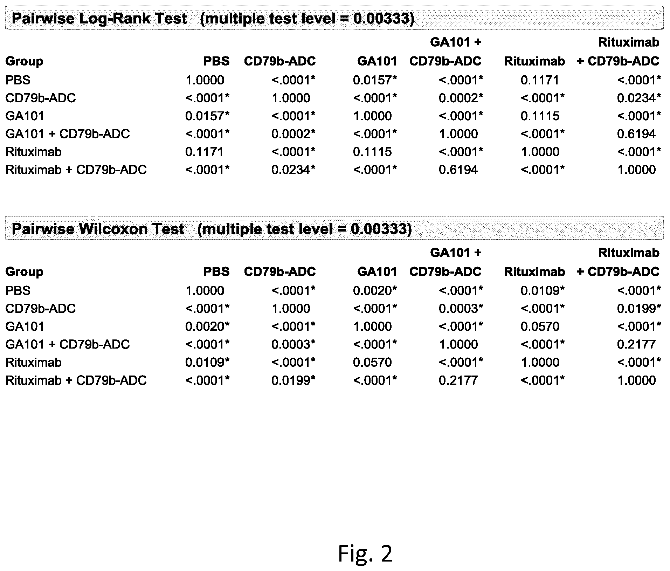

**Figure 2****:** Statistical analysis of the data in Figure 1 by Pairwise Wilcoxon and Pairwise Log-Rank test
**Figures 3A** **(light chain) and 3B (heavy chain)** show amino acid sequences of an antibody of the invention (huMA79b.v17). Figures 3A (light chain) and 3B (heavy chain) show amino acid sequences of the framework (FR), hypervariable region (HVR), first constant domain (CL or CH1) and Fc region (Fc) of one embodiment of an antibody of the invention (huMA79b.v17) (SEQ ID NOs: 131-134, 47-49, and 135 (Figure 3A) and SEQ ID NOs: 136-139, 50-52, and 140-141 (Figure 3B)). Full-length amino acid sequences (variable and constant regions) of the light and heavy chains of huMA79b.v17 are shown (SEQ ID NO: 176 (Figure 3A) and 177 (Figure 3B), respectively, with the constant domains underlined. Amino acid sequences of the variable domains are shown (SEQ ID NO: 53 (Figure 3A for light chain) and SEQ ID NO: 54 (Figure 3B for heavy chain)).
**Figures 4A** **(light chain) and 4B (heavy chain)** show amino acid sequences of an antibody of the invention (huMA79b.v18). Figures 4A (light chain) and 4B (heavy chain) show amino acid sequences of the framework (FR), hypervariable region (HVR), first constant domain (CL or CH1) and Fc region (Fc) of one embodiment of an antibody of the invention (huMA79b.v18) (SEQ ID NOs: 142-145, 55-57, and 147 (Figure 4A) and SEQ ID NOs: 148-151, 58-60, and 152-153 (Figure 4B)). Full-length amino acid sequences (variable and constant regions) of the light and heavy chains of huMA79b.v18 are shown (SEQ ID NO: 178 (Figure 4A) and 179 (Figure 4B), respectively, with the constant domains underlined. Amino acid sequences of the variable domains are shown (SEQ ID NO: 61 (Figure 4A for light chain) and SEQ ID NO: 62 (Figure 4B for heavy chain)).
**Figures 5A** **(light chain) and 5B (heavy chain)** show amino acid sequences of an antibody of the invention (huMA79b.v28). Figures 5A (light chain) and 5B (heavy chain) show amino acid sequences of the framework (FR), hypervariable region (HVR), first constant domain (CL or CH1) and Fc region (Fc) of one embodiment of an antibody of the invention (huMA79b.v28) (SEQ ID NOs: 154-157, 63-65, and 158 (Figure 5A) and SEQ ID NOs: 159-162, 66-68, and 163-164 (Figure 5B). Full-length amino acid sequences (variable and constant regions) of the light and heavy chains of huMA79b.v28 are shown (SEQ ID NO: 180 (Figure 5A) and 181 (Figure 5B), respectively, with the constant domains underlined. Amino acid sequences of the variable domains are shown (SEQ ID NO: 69 (Figure 7 for light chain) and SEQ ID NO: 70 (Figure 8 for heavy chain)).
**Figures 6A** **(light chain) and 6B (heavy chain)** show amino acid sequences of an antibody of the invention (huMA79b.v32). Figures 6A (light chain) and 6B (heavy chain) show amino acid sequences of the framework (FR), hypervariable region (HVR), first constant domain (CL or CH1) and Fc region (Fc) of one embodiment of an antibody of the invention (huMA79b.v32) (SEQ ID NOs: 165-168, 71-73, and 169 (Figure 6A) and SEQ ID NOs: 170-173, 74-76, and 174-175 (Figure 6B). Full-length amino acid sequences (variable and constant regions) of the light and heavy chains of huMA79b.v32 are shown (SEQ ID NO: 182 (Figure 6A) and 146 (Figure 6B), respectively, with the constant domains underlined. Amino acid sequences of the variable domains are shown (SEQ ID NO: 77 (Figure 6A for light chain) and SEQ ID NO: 78 (Figure 6B for heavy chain)).
**Figure 7** shows the alignment of sequences of the variable light chains for the following: light chain human kappa I consensus sequence (labeled as "huKI"; SEQ ID NO: 25) with VL-FR1, VL-FR2, VL-FR3, VL-FR4 (SEQ ID NOs: 39-42, respectively), murine anti-CD79b antibody (labeled as "MA79b"; SEQ ID NO: 26), MA79b-grafted "humanized" antibody (labeled as "huMA79b graft"; SEQ ID NO: 27), MA79b-grated "humanized" antibody variant 17 (labeled as "huMA79b.v17"; SEQ ID NO: 53), MA79b-grafted "humanized" antibody variant 18 (labeled as "huMA79b.v18"; SEQ ID NO: 61), MA79b-grafted "humanized" antibody variant 28 (labeled as "huMA79b.v28"; SEQ ID NO: 69) and MA79b-grafted "humanized" antibody variant 32 (labeled as "huMA79b.v32"; SEQ ID NO: 77). Positions are numbered according to Kabat and hypervariable regions (HVRs) grafted from MA79b to the variable light Kappa I consensus framework are boxed.
**Figure 8** shows the alignment of sequences of the variable heavy chains for the following: heavy chain human subgroup III consensus sequence (labeled as "humIII"; SEQ ID NO: 28) with VH-FR1, VH-FR2, VH-FR3, and VH-FR4 (SEQ ID NOs: 43-46), murine anti-CD79b antibody (labeled as "MA79b"; SEQ ID NO: 29), MA79b-grafted "humanized" antibody (labeled as "huMA79b graft"; SEQ ID NO: 30) (containing 71A, 73T and 78A), MA79b-grafted "humanized" antibody variant 17 (labeled as "huMA79b.v17"; SEQ ID NO: 54) (containing 71A, 73T and 78A), MA79b-grafted "humanized" antibody variant 18 (labeled as "huMA79b.v18"; SEQ ID NO: 62) (containing 71A, 73T and 78A), MA79b-grafted "humanized" antibody variant 28 (labeled as "huMA79b.v28"; SEQ ID NO: 70) (containing 71A, 73T and 78A) and MA79b-grafted "humanized" antibody variant 32 (labeled as "huMA79b.v32"; SEQ ID NO: 78) (containing 71A, 73T and 78A). Positions are numbered according to Kabat and hypervariable regions (HVRs) grafted from MA79b to the variable heavy subgroup III consensus framework are boxed.

### Detailed Description of the Invention

The invention comprises an afucosylated anti-CD20 antibody of IgG1 or IgG3 isotype with an amount of fucose of 60% or less of the total amount of oligosaccharides (sugars) at Asn297, for for the treatment of cancer in combination with a CD79b antibody-drug conjugate.

The invention comprises the use of an afucosylated anti-CD20 antibody of IgG1 or IgG3 isotype with an amount of fucose of 60% or less of the total amount of oligosaccharides (sugars) at Asn297, for the manufacture of a medicament for the treatment of cancer in combination with a CD79b antibody-drug conjugate.

In one embodiment, the amount of fucose is between 40% and 60% of the total amount of oligosaccharides (sugars) at Asn297.

The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the invention are retained. The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L. et al., Nature 332 (1988) 323-327; and Neuberger, M.S. et al., Nature 314 (1985) 268-270. Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. Human antibodies are well-known in the state of the art (van Dijk, M.A., and van de Winkel, J.G., Curr. Opin. in Chem. Biol. 5 (2001) 368-374). Based on such technology, human antibodies against a great variety of targets can be produced. Examples of human antibodies are for example described in Kellermann, S.A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form. The recombinant human antibodies according to the invention have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

As used herein, the term "binding" or "specifically binding" refers to the binding of the antibody to an epitope of the tumor antigen in an in vitro assay, preferably in an plasmon resonance assay (BIAcore, GE-Healthcare Uppsala, Sweden) with purified wild-type antigen. The affinity of the binding is defined by the terms ka (rate constant for the association of the antibody from the antibody/antigen complex), k_{D} (dissociation constant), and K_{D} (k_{D}/ka). Binding or specifically binding means a binding affinity (K_{D}) of 10⁻⁸ M or less, preferably 10⁻⁸ M to 10⁻¹³ M (in one embodiment 10⁻⁹ M to 10⁻¹³ M). Thus, an afucosylated antibody according to the invention is specifically binding to the tumor antigen with a binding affinity (K_{D}) of 10⁻⁸ mol/l or less, preferably 10⁻⁸ M to 10⁻¹³ M (in one embodiment 10⁻⁹ M to 10⁻¹³ M).

The term "nucleic acid molecule", as used herein, is intended to include DNA molecules and RNA molecules. A nucleic acid molecule may be single-stranded or double-stranded, but preferably is double-stranded DNA.

The "constant domains" are not involved directly in binding the antibody to an antigen but are involved in the effector functions (ADCC, complement binding, and CDC).

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a b-sheet conformation and the CDRs may form loops connecting the b-sheet structure. The CDRs in each chain are held in their three-dimensional structure by the framework regions and form together with the CDRs from the other chain the antigen binding site.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, MD (1991), and/or those residues from a "hypervariable loop".

The term "afucosylated antibody" refers to an antibody of IgG1 or IgG3 isotype (preferably of IgG1 isotype) with an altered pattern of glycosylation in the Fc region at Asn297 having a reduced level of fucose residues. Glycosylation of human IgG1 or IgG3 occurs at Asn297 as core fucosylated bianntennary complex oligosaccharide glycosylation terminated with up to 2 Gal residues. These structures are designated as G0, G1 (α1,6 or α1,3) or G2 glycan residues, depending from the amount of terminal Gal residues (Raju, T.S., BioProcess Int. 1 (2003) 44-53). CHO type glycosylation of antibody Fc parts is e.g. described by Routier, F.H., Glycoconjugate J. 14 (1997) 201-207. Antibodies which are recombinantely expressed in non glycomodified CHO host cells usually are fucosylated at Asn297 in an amount of at least 85%. It should be understood that the term an afucosylated antibody as used herein includes an antibody having no fucose in its glycosylation pattern. It is commonly known that typical glycosylated residue position in an antibody is the asparagine at position 297 according to the EU numbering system ("Asn297").

The "EU numbering system" or "EU index" is generally used when referring to a residue in an immunoglobulin heavy chain constant region (e.g., the EU index reported in Kabat et al., Sequences of Proteins of Immunological Interest, 5th ed., Public Health Service, National Institutes of Health, Bethesda, MD (1991)).

Thus an afucosylated antibody according to the invention means an antibody of IgG1 or IgG3 isotype (preferably of IgG1 isotype) wherein the amount of fucose is 60% or less of the total amount of oligosaccharides (sugars) at Asn297 (which means that at least 40% or more of the oligosaccharides of the Fc region at Asn297 are afucosylated). In one embodiment the amount of fucose is between 40% and 60% of the oligosaccharides of the Fc region at Asn297. In another embodiment the amount of fucose is 50% or less, and in still another embodiment the amount of fucose is 30% or less of the oligosaccharides of the Fc region at Asn297. According to the invention "amount of fucose" means the amount of said oligosaccharide (fucose) within the oligosaccharide (sugar) chain at Asn297, related to the sum of all oligosaccharides (sugars) attached to Asn 297 (e. g. complex, hybrid and high mannose structures) measured by MALDI-TOF mass spectrometry and calculated as average value (for a detailed procedure to determine the amount of fucose, see e.g. WO 2008/077546). Furthermore in one embodiment, the oligosaccharides of the Fc region are bisected. The afucosylated antibody according to the invention can be expressed in a glycomodified host cell engineered to express at least one nucleic acid encoding a polypeptide having GnTIII activity in an amount sufficient to partially fucosylate the oligosaccharides in the Fc region. In one embodiment, the polypeptide having GnTIII activity is a fusion polypeptide. Alternatively α1,6-fucosyltransferase activity of the host cell can be decreased or eliminated according to US 6,946,292 to generate glycomodified host cells. The amount of antibody fucosylation can be predetermined e.g. either by fermentation conditions (e.g. fermentation time) or by combination of at least two antibodies with different fucosylation amount. Such afucosylated antibodies and respective glycoengineering methods are described in WO 2005/044859, WO 2004/065540, WO 2007/031875, Umana, P., et al., Nature Biotechnol. 17 (1999) 176-180, WO 99/154342, WO 2005/018572, WO 2006/116260, WO 2006/114700, WO 2005/011735, WO 2005/027966, WO 97/028267, US 2006/0134709, US 2005/0054048, US 2005/0152894, WO 2003/035835, WO 2000/061739. These glycoengineered antibodies have an increased ADCC. Other glycoengineering methods yielding afucosylated antibodies according to the invention are described e.g. in Niwa, R.. et al., J. Immunol. Methods 306 (2005) 151-160; Shinkawa, T., et al., J. Biol. Chem, 278 (2003) 3466-3473; WO 03/055993 or US 2005/0249722.

Thus one aspect of the invention is an afucosylated anti-CD20 antibody of IgG1 or IgG3 isotype (preferably of IgG1 isotype) specifically binding to CD20 with an amount of fucose of 60% or less of the total amount of oligosaccharides (sugars) at Asn297, for the treatment of cancer in combination with a CD79b antibody-drug conjugate. In another aspect of the invention is the use of an afucosylated anti-CD20 antibody of IgG1 or IgG3 isotype (preferably of IgG1 isotype) specifically binding to CD20 with an amount of fucose of 60% or less of the total amount of oligosaccharides (sugars) at Asn297, for the manufacture of a medicament for the treatment of cancer in combination with a CD79b antibody-drug conjugate. In one embodiment the amount of fucose is between 60% and 20% of the total amount of oligosaccharides (sugars) at Asn297. In one embodiment the amount of fucose is between 60% and 40% of the total amount of oligosaccharides (sugars) at Asn297. In one embodiment the amount of fucose is between 0% of the total amount of oligosaccharides (sugars) at Asn297.

CD20 (also known as B-lymphocyte antigen CD20, B-lymphocyte surface antigen B1, Leu-16, Bp35, BM5, and LF5; the sequence is characterized by the SwissProt database entry P11836) is is a hydrophobic transmembrane protein with a molecular weight of approximately 35 kD located on pre-B and mature B lymphocytes (Valentine, M.A. et al., J. Biol. Chem. 264 (1989) 11282-11287; Tedder, T.F., et al., Proc. Natl. Acad. Sci. U.S.A. 85 (1988) 208-212; Stamenkovic, I., et al., J. Exp. Med. 167 (1988) 1975-1980; Einfeld, D.A., et al., EMBO J. 7 (1988) 711-717; Tedder, T.F., et al., J. Immunol. 142 (1989) 2560-2568). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members. Alternative splicing of this gene results in two transcript variants which encode the same protein.

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al., Blood 103 (2004) 2738-2743; and Cragg, M.S., et al., Blood 101 (2003) 1045-1052, see Table 1.

**Table 1: Properties of type I and type II anti-CD20 antibodies**

| **type I anti-CD20 antibodies** | **type II anti-CD20 antibodies** |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

Examples of type I anti-CD20 antibodies include e.g. rituximab, HI47 IgG3 (ECACC, hybridoma), 2C6 IgG1 (as disclosed in WO 2005/103081), 2F2 IgG1 (as disclosed and WO 2004/035607 and WO 2005/103081) and 2H7 IgG1 (as disclosed in WO 2004/056312).

The afucosylated anti-CD20 antibodies according to the invention is in one embodiment a type II anti-CD20 antibody, in another embodiment an afucosylated humanized B-Ly1 antibody.

The afucosylated anti-CD20 antibodies according to the invention have an increased antibody dependent cellular cytotoxicity (ADCC) unlike anti-CD20 antibodies having no reduced fucose.

By "afucosylated anti-CD20 antibody with increased antibody dependent cellular cytotoxicity (ADCC)" is meant an afucosylated anti-CD20 antibody, as that term is defined herein, having increased ADCC as determined by any suitable method known to those of ordinary skill in the art. One accepted in vitro ADCC assay is as follows:
1) the assay uses target cells that are known to express the target antigen recognized by the antigen-binding region of the antibody;
2) the assay uses human peripheral blood mononuclear cells (PBMCs), isolated from blood of a randomly chosen healthy donor, as effector cells;
3) the assay is carried out according to following protocol:
   i) the PBMCs are isolated using standard density centrifugation procedures and are suspended at 5 x 10⁶ cells/ml in RPMI cell culture medium;
   ii) the target cells are grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵ cells/ml;
   iii) 100 microliters of the final target cell suspension above are transferred to each well of a 96-well microtiter plate;
   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);
   vi) for the spontaneous release (SR) controls, 3 additional wells in the plate containing the labeled target cells, receive 50 microliters of RPMI cell culture medium instead of the antibody solution (point iv above);
   vii) the 96-well microtiter plate is then centrifuged at 50 x g for 1 minute and incubated for 1 hour at 4°C;
   viii) 50 microliters of the PBMC suspension (point i above) are added to each well to yield an effector:target cell ratio of 25: 1 and the plates are placed in an incubator under 5% CO₂ atmosphere at 37 C for 4 hours;
   ix) the cell-free supernatant from each well is harvested and the experimentally released radioactivity (ER) is quantified using a gamma counter;
   x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);
4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

Said "increased ADCC" can be obtained by glycoengineering of said antibodies, that means enhance said natural, cell-mediated effector functions of monoclonal antibodies by engineering their oligosaccharide component as described in Umana, P., et al., Nature Biotechnol. 17 (1999) 176-180 and US 6,602,684.

The term "complement-dependent cytotoxicity (CDC)" refers to lysis of human tumor target cells by the antibody according to the invention in the presence of complement. CDC is measured preferably by the treatment of a preparation of CD20 expressing cells with an anti-CD20 antibody according to the invention in the presence of complement. CDC is found if the antibody induces at a concentration of 100 nM the lysis (cell death) of 20% or more of the tumor cells after 4 hours. The assay is performed preferably with ⁵¹Cr or Eu labeled tumor cells and measurement of released ⁵¹Cr or Eu. Controls include the incubation of the tumor target cells with complement but without the antibody.

The "rituximab" antibody (reference antibody; example of a type I anti-CD20 antibody) is a genetically engineered chimeric human gamma 1 murine constant domain containing monoclonal antibody directed against the human CD20 antigen. This chimeric antibody contains human gamma 1 constant domains and is identified by the name "C2B8" in US 5,736,137 (Anderson et. al.) issued on April 17, 1998, assigned to IDEC Pharmaceuticals Corporation. Rituximab is approved for the treatment of patients with relapsed or refracting low-grade or follicular, CD20 positive, B cell non-Hodgkin's lymphoma. In vitro mechanism of action studies have shown that rituximab exhibits human complement--dependent cytotoxicity (CDC) (Reff, M.E., et. al., Blood 83 (1994) 435-445). Additionally, it exhibits significant activity in assays that measure antibody-dependent cellular cytotoxicity (ADCC). Rituximab is not afucosylated.

**Table 2:**

| Antibody | Amount of fucose |
|---|---|
| Rituximab (non-afucosylated) | >85 % |
| Wild type afucosylated glyco-engineered humanized B-Ly1 (B-HH6-B-KV1) (non-afucosylated) | >85 % |
| afucosylated glyco-engineered humanized B-Ly1 (B-HH6-B-KV1 GE) | 45-50 % |

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 1; variable region of the murine light chain (VL): SEQ ID NO: 2 (see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

In one embodiment, the "humanized B-Ly1 antibody" has variable region of the heavy chain (VH) selected from group of SEQ ID NO: 3 to SEQ ID NO: 19 (B-HH2 to B-HH9 and B-HL8 to B-HL17 of WO 2005/044859 and WO 2007/031875). In one specific embodiment, such variable domain is selected from the group consisting of SEQ ID NOs: 3, 4, 7, 9, 11, 13 and 15 (B-HH2, BHH-3, B-HH6, B-HH8, B-HL8, B-HL11 and B-HL13 of WO 2005/044859 and WO 2007/031875). In one specific embodiment, the "humanized B-Ly1 antibody" has variable region of the light chain (VL) of SEQ ID NO: 20 (B-KV1 of WO 2005/044859 and WO 2007/031875). In one specific embodiment, the "humanized B-Ly1 antibody" has a variable region of the heavy chain (VH) of SEQ ID NO: 7 (B-HH6 of WO 2005/044859 and WO 2007/031875) and a variable region of the light chain (VL) of SEQ ID NO: 20 (B-KV1 of WO 2005/044859 and WO 2007/031875). Furthermore in one embodiment, the humanized B-Ly1 antibody is an IgG1 antibody. According to the invention such afocusylated humanized B-Ly1 antibodies are glycoengineered (GE) in the Fc region according to the procedures described in WO 2005/044859, WO 2004/065540, WO 2007/031875, Umana, P. et al., Nature Biotechnol. 17 (1999) 176-180 and WO 99/154342. In one embodiment, the afucosylated glyco-engineered humanized B-Ly1 is B-HH6-B-KV1 GE. In one embodiment, the anti-CD20 antibody is obinutuzumab (recommended INN, WHO Drug Information, Vol. 26, No. 4, 2012, p. 453). As used herein, obinutuzumab is synonymous for GA101. This replaces all previous versions (e.g. Vol. 25, No. 1, 2011, p.75-76), and is formerly known as afutuzumab (recommended INN, WHO Drug Information, Vol. 23, No. 2, 2009, p. 176;Vol. 22, No. 2, 2008, p. 124).

Such glycoengineered humanized B-Ly1 antibodies have an altered pattern of glycosylation in the Fc region, preferably having a reduced level of fucose residues. In one embodiment, the amount of fucose is 60% or less of the total amount of oligosaccharides at Asn297 (in one embodiment the amount of fucose is between 40% and 60%, in another embodiment the amount of fucose is 50% or less, and in still another embodiment the amount of fucose is 30% or less). In another embodiment, the oligosaccharides of the Fc region are preferably bisected. These glycoengineered humanized B-Ly1 antibodies have an increased ADCC.

The term "CD79b", as used herein, refers to any native CD79b from any vertebrate source, including mammals such as primates (e.g. humans, cynomolgus monkey (cyno)) and rodents (.e.g., mice and rats), unless otherwise indicated. Human CD79b is also referred herein to as "PRO36249" (SEQ ID NO: 22) and encoded by the nucleotide sequence (SEQ ID NO: 21) also referred herein to as "DNA225786". Cynomologus CD79b is also referred herein to as "cyno CD79b" or "PRO283627" (SEQ ID NO: 80) and encoded by the nucleotide sequence (SEQ ID NO: 79) also referred herein to as "DNA548455". The term "CD79b" encompasses "full-length," unprocessed CD79b as well as any form of CD79b that results from processing in the cell. The term also encompasses naturally occurring variants of CD79b, e.g., splice variants, allelic variants and isoforms. The CD79b polypeptides described herein may be isolated from a variety of sources, such as from human tissue types or from another source, or prepared by recombinant or synthetic methods. A "native sequence CD79b polypeptide" comprises a polypeptide having the same amino acid sequence as the corresponding CD79b polypeptide derived from nature. Such native sequence CD79b polypeptides can be isolated from nature or can be produced by recombinant or synthetic means. The term "native sequence CD79b polypeptide" specifically encompasses naturally-occurring truncated or secreted forms of the specific CD79b polypeptide (*e.g.*, an extracellular domain sequence), naturally-occurring variant forms (*e.g*., alternatively spliced forms) and naturally-occurring allelic variants of the polypeptide.

"MA79b" or "murine CD79b antibody" or "murine anti-CD79b antibody" is used herein to specifically refer to murine anti-CD79b monoclonal antibody wherein the murine anti-CD79b monoclonal antibody comprises the light chain variable domain of SEQ ID NO: 26 (Figure 7) and the heavy chain variable domain of SEQ ID NO: 29 (Figure 8). Murine anti-CD79b monoclonal antibody may be purchased from commercial sources such as Biomeda (anti-human CD79b antibody; Foster City, CA), BDbioscience (anti-human CD79b antibody; San Diego, CA) or Ancell (anti-human CD79b antibody; Bayport, MN) or generated from hybridoma clone 3A2-2E7 American Type Culture Collection (ATCC) deposit designation number HB11413, deposited with the ATCC on July 20, 1993.

"chMA79b" or "chimeric MA79b antibody" is used herein to specifically refer to chimeric anti-human CD79b antibody (as previously described in US2007/0207142, filed August 3, 2006) wherein the chimeric anti-CD79b antibody comprises the light chain of SEQ ID NO: 23. The light chain of SEQ ID NO: 23 further comprises the variable domain of SEQ ID NO: 26 (Figure 7) and the light chain constant domain of human IgG1. The chimeric anti-CD79b antibody further comprises the heavy chain of SEQ ID NO: 24. The heavy chain of SEQ ID NO: 24 further comprises the variable domain of SEQ ID NO: 29 (Figure 8) and the heavy chain constant domain of human IgG1.

"anti-cynoCD79b" or "anti-cyno CD79b" is used herein to refer to antibodies that binds to cyno CD79b (SEQ ID NO: 80 as previously described in US2007/0207142, filed August 3, 2006). "anti-cynoCD79b(ch10D10)" or "ch10D10" is used herein to refer to chimeric anti-cynoCD79b (as previously described in US2007/0207142, filed August 3, 2006) which binds to cynoCD79b (SEQ ID NO: 80). Anti-cynoCD79b(ch10D10) or ch10D10 is chimeric anti-cynoCD79b antibody which comprises the light chain of SEQ ID NO: 81. Anti-cynoCD79b(ch10D10) or ch10D10 further comprises the heavy chain of SEQ ID NO: 82.

"MA79b-graft" or "MA79b-grafted 'humanized' antibody" or "huMA79b graft" is used herein to specifically refer to the graft generated by grafting the hypervariable regions from murine anti-CD79b antibody (MA79b) into the acceptor human consensus VL kappa I (huKI) and human subgroup III consensus VH (huIII) with R71A, N73T and L78A (Carter et al., Proc. Natl. Acad. Sci. USA, 89:4285 (1992)) (See SEQ ID NO: 27 (Figure 7) and SEQ ID NO: 30 (Figure 8)).

The term "anti-CD79b antibody" or "an antibody that binds to CD79b" refers to an antibody that is capable of binding CD79b with sufficient affinity such that the antibody is useful as a diagnostic and/or therapeutic agent in targeting CD79b. Preferably, the extent of binding of an anti-CD79b antibody to an unrelated, non-CD79b protein is less than about 10% of the binding of the antibody to CD79b as measured, e.g., by a radioimmunoassay (RIA). In certain embodiments, an antibody that binds to CD79b has a dissociation constant (Kd) of ≤ 1 µM, ≤ 100 nM, ≤ 10 nM, ≤ 1 nM, or ≤ 0.1 nM. In certain embodiments, anti-CD79b antibody binds to an epitope of CD79b that is conserved among CD79b from different species. The term "anti-CD79b antibody" in particular refers to any anti-CD79b antibody as disclosed in WO2009/099728.

An "isolated antibody" is one which has been identified and separated and/or recovered from a component of its natural environment. Contaminant components of its natural environment are materials which would interfere with therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The basic 4-chain antibody unit is a heterotetrameric glycoprotein composed of two identical light (L) chains and two identical heavy (H) chains (an IgM antibody consists of 5 of the basic heterotetramer unit along with an additional polypeptide called J chain, and therefore contain 10 antigen binding sites, while secreted IgA antibodies can polymerize to form polyvalent assemblages comprising 2-5 of the basic 4-chain units along with J chain). In the case of IgGs, the 4-chain unit is generally about 150,000 daltons. Each L chain is linked to a H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (V_{H}) followed by three constant domains (C_{H}) for each of the α and γ chains and four C_{H} domains for µ and ε isotypes. Each L chain has at the N-terminus, a variable domain (V_{L}) followed by a constant domain (C_{L}) at its other end. The V_{L} is aligned with the V_{H} and the C_{L} is aligned with the first constant domain of the heavy chain (C_{H}1). Particular amino acid residues are believed to form an interface between the light chain and heavy chain variable domains. The pairing of a V_{H} and V_{L} together forms a single antigen-binding site. For the structure and properties of the different classes of antibodies, see, e.g., Basic and Clinical Immunology, 8th edition, Daniel P. Stites, Abba I. Terr and Tristram G. Parslow (eds.), Appleton & Lange, Norwalk, CT, 1994, page 71 and Chapter 6.

The L chain from any vertebrate species can be assigned to one of two clearly distinct types, called kappa and lambda, based on the amino acid sequences of their constant domains. Depending on the amino acid sequence of the constant domain of their heavy chains (C_{H}), immunoglobulins can be assigned to different classes or isotypes. There are five classes of immunoglobulins: IgA, IgD, IgE, IgG, and IgM, having heavy chains designated α, δ, ε, γ, and µ, respectively. The γ and α classes are further divided into subclasses on the basis of relatively minor differences in C_{H} sequence and function, e.g., humans express the following subclasses: IgG1, IgG2, IgG3, IgG4, IgA1, and IgA2.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain segments of the variable domains differ extensively in sequence among antibodies. The V domain mediates antigen binding and defines specificity of a particular antibody for its particular antigen. However, the variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

An "intact" antibody is one which comprises an antigen-binding site as well as a C_{L} and at least heavy chain constant domains, C_{H}1, C_{H}2 and C_{H}3. The constant domains may be native sequence constant domains (e.g. human native sequence constant domains) or amino acid sequence variant thereof. Preferably, the intact antibody has one or more effector functions.

A "naked antibody" for the purposes herein is an antibody that is not conjugated to a cytotoxic moiety or radiolabel.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (see U.S. Patent No. 5,641,870, Example 2; Zapata et al., Protein Eng. 8(10): 1057-1062 [1995]); single-chain antibody molecules; and multispecific antibodies formed from antibody fragments. In one embodiment, an antibody fragment comprises an antigen binding site of the intact antibody and thus retains the ability to bind antigen.

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H}1). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having divalent antigen-binding activity and is still capable of cross-linking antigen. Fab' fragments differ from Fab fragments by having additional few residues at the carboxy terminus of the C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, which region is also the part recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to antibody fragments with two antigen-binding sites, which fragments comprise a heavy-chain variable domain (VH) connected to a light-chain variable domain (VL) in the same polypeptide chain (VH-VL). The small antibody fragments are prepared by constructing sFv fragments (see preceding paragraph) with short linkers (about 5-10 residues) between the V_{H} and V_{L} domains such that inter-chain but not intra-chain pairing of the V domains is achieved, resulting in a bivalent fragment, i.e., fragment having two antigen-binding sites. Diabodies may be bivalent or bispecific. Bispecific diabodies are heterodimers of two "crossover" sFv fragments in which the V_{H} and V_{L} domains of the two antibodies are present on different polypeptide chains. Diabodies are described more fully in, for example, EP 404,097; WO 93/11161; Hudson et al., Nat. Med. 9:129-134 (2003); and Hollinger et al., Proc. Natl. Acad. Sci. USA, 90:6444-6448 (1993). Triabodies and tetrabodies are also described in Hudson et al., Nat. Med. 9:129-134 (2003).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies useful in the present invention may be prepared by the hybridoma methodology first described by Kohler et al., Nature, 256:495 (1975), or may be made using recombinant DNA methods in bacterial, eukaryotic animal or plant cells (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (*e.g.* Old World Monkey, Ape etc), and human constant region sequences.

"Humanized" forms of non-human (*e.g*., rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma and Immunol., 1:105-115 (1998); Harris, Biochem. Soc. Transactions, 23:1035-1038 (1995); Hurle and Gross, Curr. Op. Biotech., 5:428-433 (1994).

"Thio" when used herein to refer to an antibody refers to a cysteine-engineered antibody while "hu" when used herein to refer to an antibody refers to a humanized antibody.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, *e.g.,* immunized xenomice (see, *e.g.,* U.S. Pat. Nos. 6,075,181 and 6,150,584 regarding XENOMOUSE^{™} technology). See also, for example, Li et al., Proc. Natl. Acad. Sci. USA, 103:3557-3562 (2006) regarding human antibodies generated via a human B-cell hybridoma technology.

The term "hypervariable region", "HVR", or "HV", when used herein refers to the regions of an antibody variable domain which are hypervariable in sequence and/or form structurally defined loops. Generally, antibodies comprise six hypervariable regions; three in the VH (H1, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The end of the Chothia CDR-H1 loop when numbered using the Kabat numbering convention varies between H32 and H34 depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these hypervariable regions are noted below.

| Loop | Kabat | AbM | Chothia | Contact |
|---|---|---|---|---|
| L1 | L24-L34 | L24-L34 | L24-L34 | L30-L36 |
| L2 | L50-L56 | L50-L56 | L50-L56 | L46-L55 |
| L3 | L89-L97 | L89-L97 | L89-L97 | L89-L96 |
| H1 | H31-H35B | H26-H35B | H26-H32..34 | H30-H35B |

| (Kabat Numbering) | | | | |
|---|---|---|---|---|
| H1 | H31-H35 | H26-H35 | H26-H32 | H30-H35 |

| (Chothia Numbering) | | | | |
|---|---|---|---|---|
| H2 | H50-H65 | H50-H58 | H52-H56 | H47-H58 |
| H3 | H95-H102 | H95-H102 | H95-H102 | H93-H101 |

Hypervariable regions may comprise "extended hypervariable regions" as follows: 24-36 or 24-34 (L1), 46-56 or 50-56 (L2) and 89-97 (L3) in the VL and 26-35B (H1), 50-65, 47-65 or 49-65 (H2) and 93-102, 94-102 or 95-102 (H3) in the VH. The variable domain residues are numbered according to Kabat *et al., supra* for each of these definitions.

"Framework" or "FR" residues are those variable domain residues other than the hypervariable region residues herein defined.

The term "variable domain residue numbering as in Kabat" or "amino acid position numbering as in Kabat", and variations thereof, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, MD. (1991). Using this numbering system, the actual linear amino acid sequence may contain fewer or additional amino acids corresponding to a shortening of, or insertion into, a FR or CDR of the variable domain. For example, a heavy chain variable domain may include a single amino acid insert (residue 52a according to Kabat) after residue 52 of H2 and inserted residues (e.g. residues 82a, 82b, and 82c, etc according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (e.g, Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat *et al., supra*)*.* The "EU index as in Kabat" refers to the residue numbering of the human IgG1 EU antibody. Unless stated otherwise herein, references to residue numbers in the variable domain of antibodies means residue numbering by the Kabat numbering system. Unless stated otherwise herein, references to residue numbers in the constant domain of antibodies means residue numbering by the EU numbering system (e.g., see United States Provisional Application Publication No. US2010/0280227, Figures for EU numbering).

An "affinity matured" antibody is one with one or more alterations in one or more HVRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/technology 10:779-783 (1992) describes affinity maturation by VH and VL domain shuffling. Random mutagenesis of HVR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking" antibody or an "antagonist" antibody is one which inhibits or reduces biological activity of the antigen it binds. Preferred blocking antibodies or antagonist antibodies substantially or completely inhibit the biological activity of the antigen.

An "agonist antibody", as used herein, is an antibody which mimics at least one of the functional activities of a polypeptide of interest.

A "species-dependent antibody," e.g., a mammalian anti-human IgE antibody, is an antibody which has a stronger binding affinity for an antigen from a first mammalian species than it has for a homologue of that antigen from a second mammalian species. Normally, the species-dependent antibody "bind specifically" to a human antigen (i.e., has a binding affinity (Kd) value of no more than about 1 x 10⁻⁷ M, preferably no more than about 1 x 10⁻⁸ and most preferably no more than about 1 x 10⁻⁹ M) but has a binding affinity for a homologue of the antigen from a second non-human mammalian species which is at least about 50 fold, or at least about 500 fold, or at least about 1000 fold, weaker than its binding affinity for the human antigen. The species-dependent antibody can be of any of the various types of antibodies as defined above, but preferably is a humanized or human antibody.

"Binding affinity" generally refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative embodiments are described in the following.

"Or better" when used herein to refer to binding affinity refers to a stronger binding between a molecule and its binding partner. "Or better" when used herein refers to a stronger binding, represented by a smaller numerical Kd value. For example, an antibody which has an affinity for an antigen of ".6 nM or better", the antibody's affinity for the antigen is <.6 nM, i.e..59 nM, .58 nM, .57 nM etc. or any value less than .6 nM.

In one embodiment, the "Kd" or "Kd value" according to this invention is measured by a radiolabeled antigen binding assay (RIA) performed with the Fab version of an antibody of interest and its antigen as described by the following assay that measures solution binding affinity of Fabs for antigen by equilibrating Fab with a minimal concentration of (125I)-labeled antigen in the presence of a titration series of unlabeled antigen, then capturing bound antigen with an anti-Fab antibody-coated plate (Chen, et al., (1999) J. Mol Biol 293:865-881). To establish conditions for the assay, microtiter plates (Dynex) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbant plate (Nunc #269620), 100 pM or 26 pM [125I]-antigen are mixed with serial dilutions of a Fab of interest (e.g., consistent with assessment of an anti-VEGF antibody, Fab-12, in Presta et al., (1997) Cancer Res. 57:4593-4599). The Fab of interest is then incubated overnight; however, the incubation may continue for a longer period (e.g., 65 hours) to insure that equilibrium is reached. Thereafter, the mixtures are transferred to the capture plate for incubation at room temperature (e.g., for one hour). The solution is then removed and the plate washed eight times with 0.1% Tween-20 in PBS. When the plates have dried, 150 µl/well of scintillant (MicroScint-20; Packard) is added, and the plates are counted on a Topcount gamma counter (Packard) for ten minutes. Concentrations of each Fab that give less than or equal to 20% of maximal binding are chosen for use in competitive binding assays.

According to another embodiment the Kd or Kd value is measured by using surface plasmon resonance assays using a BIAcoreTM-2000 or a BIAcoreTM-3000 (BIAcore, Inc., Piscataway, NJ) at 25C with immobilized antigen CM5 chips at ∼10 response units (RU). Briefly, carboxymethylated dextran biosensor chips (CM5, BIAcore Inc.) are activated with *N*-ethyl-*N*'- (3-dimethylaminopropyl)-carbodiimide hydrochloride (EDC) and *N-*hydroxysuccinimide (NHS) according to the supplier's instructions. Antigen is diluted with 10mM sodium acetate, pH 4.8, into 5ug/ml (∼0.2uM) before injection at a flow rate of 5ul/minute to achieve approximately 10 response units (RU) of coupled protein. Following the injection of antigen, 1M ethanolamine is injected to block unreacted groups. For kinetics measurements, two-fold serial dilutions of Fab (0.78 nM to 500 nM) are injected in PBS with 0.05% Tween 20 (PBST) at 25°C at a flow rate of approximately 25ul/min. Association rates (kon) and dissociation rates (koff) are calculated using a simple one-to-one Langmuir binding model (BIAcore Evaluation Software version 3.2) by simultaneous fitting the association and dissociation sensorgram. The equilibrium dissociation constant (Kd) is calculated as the ratio koff/kon. See, e.g., Chen, Y., et al., (1999) J. Mol Biol 293:865-881. If the on-rate exceeds 106 M-1 S-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-Aminco spectrophotometer (ThermoSpectronic) with a stir red cuvette.

An "on-rate" or "rate of association" or "association rate" or "kon" according to this invention can also be determined with the same surface plasmon resonance technique described above using a BIAcoreTM-2000 or a BIAcoreTM-3000 (BIAcore, Inc., Piscataway, NJ) as described above.

The phrase "substantially similar," or "substantially the same", as used herein, denotes a sufficiently high degree of similarity between two numeric values (generally one associated with an antibody of the invention and the other associated with a reference/comparator antibody) such that one of skill in the art would consider the difference between the two values to be of little or no biological and/or statistical significance within the context of the biological characteristic measured by said values (e.g., Kd values). The difference between said two values is preferably less than about 50%, preferably less than about 40%, preferably less than about 30%, preferably less than about 20%, preferably less than about 10% as a function of the value for the reference/comparator antibody.

The phrase "substantially reduced," or "substantially different", as used herein, denotes a sufficiently high degree of difference between two numeric values (generally one associated with an antibody of the invention and the other associated with a reference/comparator antibody) such that one of skill in the art would consider the difference between the two values to be of statistical significance within the context of the biological characteristic measured by said values (e.g., Kd values, HAMA response). The difference between said two values is preferably greater than about 10%, preferably greater than about 20%, preferably greater than about 30%, preferably greater than about 40%, preferably greater than about 50% as a function of the value for the reference/comparator antibody.

An "antigen" is a predetermined antigen to which an antibody can selectively bind. The target antigen may be polypeptide, carbohydrate, nucleic acid, lipid, hapten or other naturally occurring or synthetic compound. Preferably, the target antigen is a polypeptide.

An "acceptor human framework" for the purposes herein is a framework comprising the amino acid sequence of a VL or VH framework derived from a human immunoglobulin framework, or from a human consensus framework. An acceptor human framework "derived from" a human immunoglobulin framework or human consensus framework may comprise the same amino acid sequence thereof, or may contain pre-existing amino acid sequence changes. Where pre-existing amino acid changes are present, preferably no more than 5 and preferably 4 or less, or 3 or less, pre-existing amino acid changes are present. Where pre-existing amino acid changes are present in a VH, preferably those changes are only at three, two or one of positions 71H, 73H and 78H; for instance, the amino acid residues at those positions may be 71A, 73T and/or 78A. In one embodiment, the VL acceptor human framework is identical in sequence to the VL human immunoglobulin framework sequence or human consensus framework sequence.

A "human consensus framework" is a framework which represents the most commonly occurring amino acid residue in a selection of human immunoglobulin VL or VH framework sequences. Generally, the selection of human immunoglobulin VL or VH sequences is from a subgroup of variable domain sequences. Generally, the subgroup of sequences is a subgroup as in Kabat *et al.* In one embodiment, for the VL, the subgroup is subgroup kappa I as in Kabat *et al.* In one embodiment, for the VH, the subgroup is subgroup III as in Kabat *et al.*

A "VH subgroup III consensus framework" comprises the consensus sequence obtained from the amino acid sequences in variable heavy subgroup III of Kabat *et al.* In one embodiment, the VH subgroup III consensus framework amino acid sequence comprises at least a portion or all of each of the following sequences: EVQLVESGGGLVQPGGSLRLSCAAS (SEQ ID NO: 43)-H1-WVRQAPGKGLEWV (SEQ ID NO: 44)-H2-RFTISRDNSKNTLYLQMNSLRAEDTAVYYC (SEQ ID NO: 45)-H3-WGQGTLVTVSS (SEQ ID NO: 46).

A "VL subgroup I consensus framework" comprises the consensus sequence obtained from the amino acid sequences in variable light kappa subgroup I of Kabat *et al.* In one embodiment, the VL subgroup I consensus framework amino acid sequence comprises at least a portion or all of each of the following sequences:
DIQMTQSPSSLSASVGDRVTITC (SEQ ID NO: 39)-L1-WYQQKPGKAPKLLIY (SEQ ID NO: 40)-L2-GVPSRFSGSGSGTDFTLTISSLQPEDFATYYC (SEQ ID NO: 41)-L3-FGQGTKVEIKR (SEQ ID NO: 42).

An "unmodified human framework" is a human framework which has the same amino acid sequence as the acceptor human framework, e.g. lacking human to non-human amino acid substitution(s) in the acceptor human framework.

An "altered hypervariable region" for the purposes herein is a hypervariable region comprising one or more (*e.g.* one to about 16) amino acid substitution(s) therein.

An "un-modified hypervariable region" for the purposes herein is a hypervariable region having the same amino acid sequence as a non-human antibody from which it was derived, *i.e.* one which lacks one or more amino acid substitutions therein.

An antibody "which binds" an antigen of interest, e.g. a tumor-associated polypeptide antigen target, is one that binds the antigen with sufficient affinity such that the antibody is useful as a therapeutic agent in targeting a cell or tissue expressing the antigen, and does not significantly cross-react with other proteins. In such embodiments, the extent of binding of the antibody to a "non-target" protein will be less than about 10% of the binding of the antibody to its particular target protein as determined by fluorescence activated cell sorting (FACS) analysis or radioimmunoprecipitation (RIA). With regard to the binding of an antibody to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater. In one embodiment, the term "specific binding" refers to binding where a molecule binds to a particular polypeptide or epitope on a particular polypeptide without substantially binding to any other polypeptide or polypeptide epitope.

An antibody that "inhibits the growth of tumor cells expressing a CD79b polypeptide" or a "growth inhibitory" antibody is one which results in measurable growth inhibition of cancer cells expressing or overexpressing the appropriate CD79b polypeptide. The CD79b polypeptide may be a transmembrane polypeptide expressed on the surface of a cancer cell or may be a polypeptide that is produced and secreted by a cancer cell. Preferred growth inhibitory anti-CD79b antibodies inhibit growth of CD79b-expressing tumor cells by greater than 20%, preferably from about 20% to about 50%, and even more preferably, by greater than 50% (e.g., from about 50% to about 100%) as compared to the appropriate control, the control typically being tumor cells not treated with the antibody being tested. In one embodiment, growth inhibition can be measured at an antibody concentration of about 0.1 to 30 µg/ml or about 0.5 nM to 200 nM in cell culture, where the growth inhibition is determined 1-10 days after exposure of the tumor cells to the antibody. Growth inhibition of tumor cells *in vivo* can be determined in various ways such as is described in the Experimental Examples section below. The antibody is growth inhibitory in vivo if administration of the anti-CD79b antibody at about 1 µg/kg to about 100 mg/kg body weight results in reduction in tumor size or tumor cell proliferation within about 5 days to 3 months from the first administration of the antibody, preferably within about 5 to 30 days.

An antibody which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is usually one which overexpresses a CD79b polypeptide. Preferably the cell is a tumor cell, e.g., a hematopoietic cell, such as a B cell, T cell, basophil, eosinophil, neutrophil, monocyte, platelet or erythrocyte. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody which induces apoptosis is one which results in about 2 to 50 fold, preferably about 5 to 50 fold, and most preferably about 10 to 50 fold, induction of annexin binding relative to untreated cell in an annexin binding assay.

An antibody which "induces cell death" is one which causes a viable cell to become nonviable. The cell is one which expresses a CD79b polypeptide and is of a cell type which specifically expresses or overexpresses a CD79b polypeptide. The cell may be cancerous or normal cells of the particular cell type. The CD79b polypeptide may be a transmembrane polypeptide expressed on the surface of a cancer cell or may be a polypeptide that is produced and secreted by a cancer cell. The cell may be a cancer cell, e.g., a B cell or T cell. Cell death *in vitro* may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (i.e., in the absence of complement) and in the absence of immune effector cells. To determine whether the antibody is able to induce cell death, loss of membrane integrity as evaluated by uptake of propidium iodide (PI), trypan blue (see Moore et al. Cytotechnology 17:1-11 (1995)) or 7AAD can be assessed relative to untreated cells. Preferred cell death-inducing antibodies are those which induce PI uptake in the PI uptake assay in BT474 cells.

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors (e.g., B cell receptor); and B cell activation.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

A "functional Fc region" possesses an "effector function" of a native sequence Fc region. Exemplary "effector functions" include C1q binding; CDC; Fc receptor binding; ADCC; phagocytosis; down regulation of cell surface receptors (e.g. B cell receptor; BCR), etc. Such effector functions generally require the Fc region to be combined with a binding domain (*e.g*., an antibody variable domain) and can be assessed using various assays as disclosed, for example, in definitions herein.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-92 (1991). To assess ADCC activity of a molecule of interest, an in vitro ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. (USA) 95:652-656 (1998).

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daëron, Annu. Rev. Immunol. 15:203-234 (1997)). FcRs are reviewed in Ravetch and Kinet, Annu. Rev. Immunol. 9:457-492 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

Binding to human FcRn *in vivo* and serum half life of human FcRn high affinity binding polypeptides can be assayed, *e.g*., in transgenic mice or transfected human cell lines expressing human FcRn, or in primates to which the polypeptides with a variant Fc region are administered. WO 2000/42072 (Presta) describes antibody variants with improved or diminished binding to FcRs. See also, *e.g.,* Shields et al. J. Biol. Chem. 9(2):6591-6604 (2001).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source, e.g., from blood.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, e.g., as described in Gazzano-Santoro et al., J. Immunol. Methods 202:163 (1996), may be performed. Polypeptide variants with altered Fc region amino acid sequences (polypeptides with a variant Fc region) and increased or decreased C1q binding capability are described, *e.g.,* in US Patent No. 6,194,551 B1 and WO 1999/51642. See also, *e.g.,* Idusogie et al. J. Immunol. 164: 4178-4184 (2000).

The term "Fc region-comprising antibody" refers to an antibody that comprises an Fc region. The C-terminal lysine (residue 447 according to the EU numbering system) of the Fc region may be removed, for example, during purification of the antibody or by recombinant engineering of the nucleic acid encoding the antibody. Accordingly, a composition comprising an antibody having an Fc region according to this invention can comprise an antibody with K447, with all K447 removed, or a mixture of antibodies with and without the K447 residue.

The CD79b polypeptide "extracellular domain" or "ECD" refers to a form of the CD79b polypeptide which is essentially free of the transmembrane and cytoplasmic domains. Ordinarily, a CD79b polypeptide ECD will have less than 1% of such transmembrane and/or cytoplasmic domains and preferably, will have less than 0.5% of such domains. It will be understood that any transmembrane domains identified for the CD79b polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a CD79b polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the CD79b polypeptide disclosed herein may be shown in the present specification. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

### Abbreviations

### LINKER COMPONENTS:

MC = 6-maleimidocaproyl
Val-Cit or "vc" = valine-citrulline (an exemplary dipeptide in a protease cleavable linker)
Citrulline = 2-amino-5-ureido pentanoic acid
PAB = p-aminobenzyloxycarbonyl (an example of a "self immolative" linker component)
Me-Val-Cit = N-methyl-valine-citrulline (wherein the linker peptide bond has been modified to prevent its cleavage by cathepsin B)
MC(PEG)6-OH = maleimidocaproyl- polyethylene glycol (can be attached to antibody cysteines).

### CYTOTOXIC DRUGS:

MMAE = mono-methyl auristatin E (MW 718)
MMAF = variant of auristatin E (MMAE) with a phenylalanine at the C-terminus of the drug (MW 731.5)
MMAF-DMAEA = MMAF with DMAEA (dimethylaminoethylamine) in an amide linkage to the C-terminal phenylalanine (MW 801.5)
MMAF-TEG = MMAF with tetraethylene glycol esterified to the phenylalanine
MMAF-NtBu = N-t-butyl, attached as an amide to C-terminus of MMAF
DM1 = N(2')-deacetyl-N(2')-(3-mercapto-1-oxopropyl)-maytansine
DM3 = N(2')-deacetyl-N2-(4-mercapto-1-oxopentyl)-maytansine
DM4 = N(2')-deacetyl-N2-(4-mercapto-4-methyl-1-oxopentyl)-maytansine

Further abbreviations are as follows: AE is auristatin E, Boc is *N*-(*t*-butoxycarbonyl), cit is citrulline, dap is dolaproine, DCC is 1,3-dicyclohexylcarbodiimide, DCM is dichloromethane, DEA is diethylamine, DEAD is diethylazodicarboxylate, DEPC is diethylphosphorylcyanidate, DIAD is diisopropylazodicarboxylate, DIEA is *N,N*-diisopropylethylamine, dil is dolaisoleucine, DMA is dimethylacetamide, DMAP is 4-dimethylaminopyridine, DME is ethyleneglycol dimethyl ether (or 1,2-dimethoxyethane), DMF is *N,N-*dimethylformamide, DMSO is dimethylsulfoxide, doe is dolaphenine, dov is *N,N-*dimethylvaline, DTNB is 5,5'-dithiobis(2-nitrobenzoic acid), DTPA is diethylenetriaminepentaacetic acid, DTT is dithiothreitol, EDCI is 1-(3-dimethylaminopropyl)-3-ethylcarbodiimide hydrochloride, EEDQ is 2-ethoxy-1-ethoxycarbonyl-1,2-dihydroquinoline, ES-MS is electrospray mass spectrometry, EtOAc is ethyl acetate, Fmoc is N-(9-fluorenylmethoxycarbonyl), gly is glycine, HATU is *O*-(7-azabenzotriazol-1-yl)-*N,N,N',N'*-tetramethyluronium hexafluorophosphate, HOBt is 1-hydroxybenzotriazole, HPLC is high pressure liquid chromatography, ile is isoleucine, lys is lysine, MeCN (CH₃CN) is acetonitrile, MeOH is methanol, Mtr is 4-anisyldiphenylmethyl (or 4-methoxytrityl),nor is (*1S*, *2R*)-(+)-norephedrine, PBS is phosphate-buffered saline (pH 7.4), PEG is polyethylene glycol, Ph is phenyl, Pnp is p-nitrophenyl, MC is 6-maleimidocaproyl, phe is L-phenylalanine, PyBrop is bromo *tris*-pyrrolidino phosphonium hexafluorophosphate, SEC is size-exclusion chromatography, Su is succinimide, TFA is trifluoroacetic acid, TLC is thin layer chromatography, UV is ultraviolet, and val is valine.

In one aspect, the invention includes a cysteine engineered anti-CD79b antibody in said antibody-drug conjugate according to the invention comprises one or more free cysteine amino acids wherein the cysteine engineered anti-CD79b antibody binds to a CD79b polypeptide and is prepared by a process comprising replacing one or more amino acid residues of a parent anti-CD79b antibody by cysteine wherein the parent antibody comprises at least one HVR sequence selected from:
(a) HVR-L1 comprising sequence A1-A15, wherein A1-A15 is KASQSVDYDGDSFLN (SEQ ID NO: 31) or KASQSVDYEGDSFLN (SEQ ID NO: 37);
(b) HVR-L2 comprising sequence B1-B7, wherein B1-B7 is AASNLES (SEQ ID NO: 32)
(c) HVR-L3 comprising sequence C1-C9, wherein C1-C9 is QQSNEDPLT (SEQ ID NO: 33)
(d) HVR-H1 comprising sequence D1-D10, wherein D1-D10 is GYTFSSYWIE (SEQ ID NO: 34)
(e) HVR-H2 comprising sequence E1-E18, wherein E1-E18 is GEILPGGGDTNYNEIFKG (SEQ ID NO: 35) and
(f) HVR-H3 comprising sequence F1-F10, wherein F1-F10 is TRRVPVYFDY (SEQ ID NO: 36) or TRRVPIRLDY (SEQ ID NO: 38).

In a certain aspect, the invention concerns a cysteine engineered anti-CD79b antibody in said antibody-drug conjugate according to the invention, comprising an amino acid sequence having at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, 99% or 100% amino acid sequence identity, to a cysteine engineered antibody having a full-length amino acid sequence as disclosed herein, or a cysteine engineered antibody amino acid sequence lacking the signal peptide as disclosed herein.

In a yet further aspect, the invention concerns a combination of an anti-CD20 antibody as defined herein with an isolated cysteine engineered anti-CD79b antibody in said antibody-drug conjugate according to the invention comprising an amino acid sequence that is encoded by a nucleotide sequence that hybridizes to the complement of a DNA molecule encoding (a) a cysteine engineered antibody having a full-length amino acid sequence as disclosed herein, (b) a cysteine engineered antibody amino acid sequence lacking the signal peptide as disclosed herein, (c) an extracellular domain of a transmembrane cysteine engineered antibody protein, with or without the signal peptide, as disclosed herein, (d) an amino acid sequence encoded by any of the nucleic acid sequences disclosed herein or (e) any other specifically defined fragment of a full-length cysteine engineered antibody amino acid sequence as disclosed herein.

In a specific aspect, the invention provides a combination of an anti-CD20 antibody as defined herein with an isolated cysteine engineered anti-CD79b antibody in said antibody-drug conjugate according to the invention without the N-terminal signal sequence and/or without the initiating methionine and is encoded by a nucleotide sequence that encodes such an amino acid sequence as described in. Processes for producing the same are also herein described, wherein those processes comprise culturing a host cell comprising a vector which comprises the appropriate encoding nucleic acid molecule under conditions suitable for expression of the cysteine engineered antibody and recovering the cysteine engineered antibody from the cell culture.

Another aspect of the invention provides a combination of an anti-CD20 antibody as defined herein with an isolated cysteine engineered anti-CD79b antibody in said antibody-drug conjugate according to the invention which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the cysteine engineered antibody and recovering the cysteine engineered antibody from the cell culture.

In other aspects, the invention provides a combination of an anti-CD20 antibody as defined herein with isolated anti-CD79b chimeric cysteine engineered antibodies in said antibody-drug conjugate according to the invention comprising any of the herein described cysteine engineered antibody fused to a heterologous (non-CD79b) polypeptide. Examples of such chimeric molecules comprise any of the herein described cysteine engineered antibodies fused to a heterologous polypeptide such as, for example, an epitope tag sequence or a Fc region of an immunoglobulin.

The cysteine engineered anti-CD79b antibody in said antibody-drug conjugates according to the invention may be a monoclonal antibody, antibody fragment, chimeric antibody, humanized antibody, single-chain antibody or antibody that competitively inhibits the binding of an anti-CD79b polypeptide antibody to its respective antigenic epitope. Antibodies of the present invention may optionally be conjugated to a growth inhibitory agent or cytotoxic agent such as a toxin, including, for example, an auristatin, a maytansinoid, a dolostatin derivative or a calicheamicin, an antibiotic, a radioactive isotope, a nucleolytic enzyme, or the like. The antibodies of the present invention may optionally be produced in CHO cells or bacterial cells and preferably inhibit the growth or proliferation of or induce the death of a cell to which they bind. For diagnostic purposes, the antibodies of the present invention may be detectably labeled, attached to a solid support, or the like.

In other aspects of the present invention, the invention provides vectors comprising DNA encoding any of the herein described anti-CD79b antibodies and anti-CD79b cysteine engineered antibodies in said antibody-drug conjugates according to the invention. Host cells comprising any such vector are also provided. By way of example, the host cells may be CHO cells, E. coli cells, or yeast cells. A process for producing any of the herein described polypeptides is further provided and comprises culturing host cells under conditions suitable for expression of the desired polypeptide and recovering the desired polypeptide from the cell culture.

Cysteine engineered antibodies in said antibody-drug conjugates according to the invention of the combination invention may be useful in the treatment of cancer and include antibodies specific for cell surface and transmembrane receptors, and tumor-associated antigens (TAA). Such antibodies may be used as naked antibodies (unconjugated to a drug or label moiety) or as antibody-drug conjugates (ADC). Cysteine engineered antibodies of the invention may be site-specifically and efficiently coupled with a thiol-reactive reagent. The thiol-reactive reagent may be a multifunctional linker reagent, a capture label reagent, a fluorophore reagent, or a drug-linker intermediate. The cysteine engineered antibody may be labeled with a detectable label, immobilized on a solid phase support and/or conjugated with a drug moiety. Thiol reactivity may be generalized to any antibody where substitution of amino acids with reactive cysteine amino acids may be made within the ranges in the light chain selected from amino acid ranges: L10-L20, L105-L115, L109-L119, L116-L126, L122-L132, L163-L173, L200-L210; and within the ranges in the heavy chain selected from amino acid ranges: H1-H10, H18-H28, H79-H89, H107-H117, H109-H119, H111-H121, and in the Fc region within the ranges selected from H270-H280, H366-H376, H391-401, where the numbering of amino acid positions begins at position 1 of the Kabat numbering system (Kabat et al. (1991) Sequences of Proteins of Immunological Interest, 5th Ed. Public Health Service, National Institutes of Health, Bethesda, MD) and continues sequentially thereafter as disclosed in WO2006034488; US 2007/0092940. Thiol reactivity may also be generalized to certain domains of an antibody, such as the light chain constant domain (CL) and heavy chain constant domains, CH1, CH2 and CH3. Cysteine replacements resulting in thiol reactivity values of 0.6 and higher may be made in the heavy chain constant domains α, δ, ε, γ, and µ of intact antibodies: IgA, IgD, IgE, IgG, and IgM, respectively, including the IgG subclasses: IgG1, IgG2, IgG3, IgG4, IgA, and IgA2. Such antibodies and their uses are disclosed in WO2006/034488; US 2007/0092940.

Cysteine engineered antibodies of the combination invention preferably retain the antigen binding capability of their wild type, parent antibody counterparts. Thus, cysteine engineered antibodies are capable of binding, preferably specifically, to antigens. Such antigens include, for example, tumor-associated antigens (TAA), cell surface receptor proteins and other cell surface molecules, transmembrane proteins, signalling proteins, cell survival regulatory factors, cell proliferation regulatory factors, molecules associated with (for e.g., known or suspected to contribute functionally to) tissue development or differentiation, lymphokines, cytokines, molecules involved in cell cycle regulation, molecules involved in vasculogenesis and molecules associated with (for e.g., known or suspected to contribute functionally to) angiogenesis. The tumor-associated antigen may be a cluster differentiation factor (i.e., a CD protein, including but not limited to CD79b). Cysteine engineered anti-CD79b antibodies of the invention retain the antigen binding ability of their parent anti-CD79b antibody counterparts. Thus, cysteine engineered anti-CD79b antibodies of the invention are capable of binding, preferably specifically, to CD79b antigens including human anti-CD79b isoforms beta and/or alpha, including when such antigens are expressed on the surface of cells, including, without limitation, B cells.

In one aspect, antibodies of the combination invention may be conjugated with any label moiety which can be covalently attached to the antibody through a reactive moiety, an activated moiety, or a reactive cysteine thiol group (Singh et al (2002) Anal. Biochem. 304:147-15; Harlow E. and Lane, D. (1999) Using Antibodies: A Laboratory Manual, Cold Springs Harbor Laboratory Press, Cold Spring Harbor, NY; Lundblad R.L. (1991) Chemical Reagents for Protein Modification, 2nd ed. CRC Press, Boca Raton, FL). The attached label may function to: (i) provide a detectable signal; (ii) interact with a second label to modify the detectable signal provided by the first or second label, e.g. to give FRET (fluorescence resonance energy transfer); (iii) stabilize interactions or increase affinity of binding, with antigen or ligand; (iv) affect mobility, e.g. electrophoretic mobility or cell-permeability, by charge, hydrophobicity, shape, or other physical parameters, or (v) provide a capture moiety, to modulate ligand affinity, antibody/antigen binding, or ionic complexation.

In certain embodiment s, a polynucleotide encoding any of the above antibodies is provided. In one embodiment, a vector comprising the polynucleotide is provided. In one embodiment, a host cell comprising the vector is provided. In one embodiment, the host cell is eukaryotic. In one embodiment, the host cell is a Chinese hamster ovary (CHO) cell. In one embodiment, a method of making an anti-CD79b antibody is provided, wherein the method comprises culturing the host cell under conditions suitable for expression of the polynucleotide encoding the antibody, and isolating the antibody.

### Antibody-Drug Conjugates

In another aspect, the invention provides a combination of an anti-CD20 antibody as defined herein with immunoconjugates, or antibody-drug conjugates (ADC), comprising an antibody conjugated to a cytotoxic agent such as a chemotherapeutic agent, a drug, a growth inhibitory agent, a toxin (e.g., an enzymatically active toxin of bacterial, fungal, plant, or animal origin, or fragments thereof), or a radioactive isotope (i.e., a radioconjugate). In another aspect, the invention further provides methods of using the immunoconjugates. In one aspect, an immunoconjugate comprises any of the above anti-CD79b antibodies covalently attached to a cytotoxic agent or a detectable agent.

In one aspect, a CD79b antibody of the combination invention binds to the same epitope on CD79b bound by another CD79b antibody. In another embodiment, a CD79b antibody of the invention binds to the same epitope on CD79b bound by the Fab fragment of, a monoclonal antibody generated from hybridomas deposited with the ATCC as HB11413 on July 20, 1993, a monoclonal antibody comprising the variable domains of SEQ ID NO: 26 (Figure 7) and SEQ ID NO: 29 (Figure 8) or a chimeric antibody comprising the variable domain of either antibody generated from HB11413 hybridomas deposited with the ATCC on July 20, 1993 and constant domains from IgG1, or the variable domains of monoclonal antibody comprising the sequences of SEQ ID NO: 26 (Figure 7) and SEQ ID NO: 29 (Figure 8). In another embodiment, a CD79b antibody of the invention binds to the same epitope on CD79b bound by another CD79b antibody (i.e., CB3.1 (BD Biosciences Catalog #555678; San Jose, CA), AT105-1 (AbD Serotec Catalog #MCA2208; Raleigh, NC), AT107-2 (AbD Serotec Catalog #MCA2209), anti-human CD79b antibody (BD Biosciences Catalog #557592; San Jose, CA)).

In another aspect, a CD79b antibody of the combination invention binds to an epitope on CD79b distinct from an epitope bound by another CD79b antibody. In another embodiment, a CD79b antibody of the invention binds to an epitope on CD79b distinct from an epitope bound by the Fab fragment of, monoclonal antibody generated from HB11413 hybridomas deposited with the ATCC on July 20, 1993, monoclonal antibody comprising the variable domains of SEQ ID NO: 26 (Figure 7) and SEQ ID NO: 29 (Figure 8), or chimeric antibody comprising the variable domain of either antibody generated from HB11413 hybridomas deposited with the ATCC on July 20, 1993 and constant domains from IgG1, or the variable domains of monoclonal antibody comprising the sequences of SEQ ID NO: 26 (Figure 7) and SEQ ID NO: 29 (Figure 8). In another embodiment, a CD79b antibody of the invention binds to an epitope on CD79b distinct from an epitope on CD79b bound by another CD79b antibody (i.e., CB3.1 (BD Biosciences Catalog #555678; San Jose, CA), AT105-1 (AbD Serotec Catalog #MCA2208; Raleigh, NC), AT107-2 (AbD Serotec Catalog #MCA2209), anti-human CD79b antibody (BD Biosciences Catalog #557592; San Jose, CA)).

In another aspect, a CD79b antibody of the combination invention is distinct from (i.e., it is not) a Fab fragment of, the monoclonal antibody generated from hybridomas deposited with the ATCC as HB11413 on July 20, 1993, the monoclonal antibody comprising the variable domains of SEQ ID NO: 26 (Figure 7) and SEQ ID NO: 29 (Figure 8), or chimeric antibody comprising the variable domain of antibody generated from hybridomas deposited with the ATCC as HB11413 on July 20, 1993 and constant domains from IgG1, or the variable domains of monoclonal antibody comprising the sequences of SEQ ID NO: 26 (Figure 7) and SEQ ID NO: 29 (Figure 8). In another embodiment, a CD79b antibody of the invention is distinct from (i.e., it is not) a Fab fragment of another CD79b antibody ((i.e., CB3.1 (BD Biosciences Catalog #555678; San Jose, CA), AT105-1 (AbD Serotec Catalog #MCA2208; Raleigh, NC), AT107-2 (AbD Serotec Catalog #MCA2209), anti-human CD79b antibody (BD Biosciences Catalog #557592; San Jose, CA)).

In one aspect, an antibody of the invention specifically binds to CD79b of a first animal species, and does not specifically bind to CD79b of a second animal species. In one embodiment, the first animal species is human and/or primate (e.g., cynomolgus monkey), and the second animal species is murine (e.g., mouse) and/or canine. In one embodiment, the first animal species is human. In one embodiment, the first animal species is primate, for example cynomolgus monkey. In one embodiment, the second animal species is murine, for example mouse. In one embodiment, the second animal species is canine.

In one aspect, an antibody that binds to CD79b expressed on the surface of a cell is provided. In one embodiment, the antibody binds to an epitope within a region of human or mouse CD79b comprising domain 1 or domain 2 or domains 1 and 2. In one embodiment, the cell is mammalian cell. In one embodiment, the cell is a human cell. In one embodiment, the cell is a cancer cell. In one embodiment the cell is a B cell. In one embodiment the cancer cell is a B cell.

In certain embodiment s, any of the above antibodies is a monoclonal antibody. In one embodiment, the antibody is an antibody fragment selected from a Fab, Fab'-SH, Fv, scFv, or (Fab')2 fragment. In one embodiment, the antibody is humanized. In one embodiment, the antibody is human.

A detailed description of exemplary anti-CD79b antibodies as part of the antibody-drug conjugate in the inventive combination with a anti-CD20 antibody is as follows:

### Specific embodiments of anti-CD79b antibodies

In one aspect, the invention provides an antibody which binds, preferably specifically, to any of the above or below described polypeptides. Optionally, the antibody is a monoclonal antibody, antibody fragment, including Fab, Fab', F(ab')₂, and Fv fragment, diabody, single domain antibody, chimeric antibody, humanized antibody, single-chain antibody or antibody that competitively inhibits the binding of an anti-CD79b polypeptide antibody to its respective antigenic epitope. Antibodies of the present invention may optionally be conjugated to a growth inhibitory agent or cytotoxic agent such as a toxin, including, for example, an auristatin, a maytansinoid, a dolostatin derivative or a calicheamicin, an antibiotic, a radioactive isotope, a nucleolytic enzyme, or the like. The antibodies of the present invention may optionally be produced in CHO cells or bacterial cells and preferably induce death of a cell to which they bind. For detection purposes, the antibodies of the present invention may be detectably labeled, attached to a solid support, or the like.

In one aspect, the invention provides a combination of an anti-CD20 antibody as defined herein with a humanized anti-CD79b antibody wherein the monovalent affinity (e.g affinity of the antibody as a Fab fragment to CD79b) or affinity in its bivalent form of the antibody to CD79b (e.g. affinity of the antibody as an IgG fragment to CD79b) is substantially the same as, lower than, or greater than, the monovalent affinity or affinity in its bivalent form, respectively, of a murine antibody (e.g. affinity of the murine antibody as a Fab fragment or as an IgG fragment to CD79b) or a chimeric antibody (e.g. affinity of the chimeric antibody as a Fab fragment or as an IgG fragment to CD79b), comprising, consisting or consisting essentially of a light chain and heavy chain variable domain sequence as depicted in Figure 7 (SEQ ID NO: 26) and Figure 8 (SEQ ID NO: 29).

In another aspect, the invention provides a combination of an anti-CD20 antibody as defined herein with a humanized anti-CD79b antibody wherein the monovalent affinity of the antibody to CD79b (e.g., affinity of the antibody as a Fab fragment to CD79b) is lower, for example at least 1, 2, 3, 4, 5, 6, 7, 8, 9, 10, 11, 12, 13, 14, 15, 16, 17, 18, 19, 20, 25, 30, 35, 40, 45, 50, 55 or 60-fold lower, than the monovalent affinity of a murine antibody (e.g., affinity of the murine antibody as a Fab fragment to CD79b) or a chimeric antibody (e.g. affinity of the chimeric antibody as a Fab fragment to CD79b), comprising, consisting or consisting essentially of a light chain and heavy chain variable domain sequence as depicted in Figure 7 (SEQ ID NO: 26) and Figure 8 (SEQ ID NO: 29).

In another aspect, the invention provides a combination of an anti-CD20 antibody as defined herein with a humanized anti-CD79b antibody wherein the monovalent affinity of the antibody to CD79b (e.g., affinity of the antibody as a Fab fragment to CD79b) is greater, for example at least 1, 2, 3, 4, 5, 6, 7, 8, 9 or 10-fold greater, than the monovalent affinity of a murine antibody (e.g., affinity of the murine antibody as a Fab fragment to CD79b) or a chimeric antibody (e.g. affinity of the chimeric antibody as a Fab fragment to CD79b), comprising, consisting or consisting essentially of a light chain and heavy chain variable domain sequence as depicted in Figure 7 (SEQ ID NO: 26) and Figure 8 (SEQ ID NO: 29).

In one aspect, the invention provides a combination of an anti-CD20 antibody as defined herein with a humanized anti-CD79b antibody wherein the affinity of the antibody in its bivalent form to CD79b (e.g. affinity of the antibody as an IgG to CD79b) is substantially the same as the affinity of a murine antibody (e.g. affinity of the antibody as an IgG to CD79b) or a chimeric antibody (e.g. affinity of the chimeric antibody as a Fab fragment to CD79b) in its bivalent form, comprising, consisting or consisting essentially of a light chain and heavy chain variable domain sequence as depicted in Figure 7 (SEQ ID NO: 26) and Figure 8 (SEQ ID NO: 29).

In another aspect, the invention provides a combination of an anti-CD20 antibody as defined herein with a humanized anti-CD79b antibody wherein the affinity of the antibody in its bivalent form to CD79b (e.g. affinity of the antibody as an IgG to CD79b) is lower, for example at least 1, 2, 3, 4, 5, 6, 7, 8, 9, 10, 11, 12, 13, 14, 15, 16, 17, 18, 19, 20, 25, 30, 35, 40, 45, 50, 55 or 60-fold lower, as the affinity of a murine antibody (e.g. affinity of the antibody as an IgG to CD79b) or a chimeric antibody (e.g. affinity of the chimeric antibody as an IgG fragment to CD79b) in its bivalent form, comprising, consisting or consisting essentially of a light chain and heavy chain variable domain sequence as depicted in Figure 7 (SEQ ID NO: 26) and Figure 8 (SEQ ID NO: 29).

In another aspect, the invention provides a combination of an anti-CD20 antibody as defined herein with a humanized anti-CD79b antibody wherein the affinity of the antibody in its bivalent form to CD79b (e.g. affinity of the antibody as an IgG to CD79b) is greater, for example at least 1, 2, 3, 4, 5, 6, 7, 8, 9 or 10-fold greater, than the affinity of a murine antibody (e.g. affinity of the antibody as an IgG to CD79b) or a chimeric antibody (e.g. affinity of the chimeric antibody as an IgG fragment to CD79b) in its bivalent form, comprising, consisting or consisting essentially of a light chain and heavy chain variable domain sequence as depicted in Figure 7 (SEQ ID NO: 26) and Figure 8 (SEQ ID NO: 29).

In another aspect, the invention provides a combination of an anti-CD20 antibody as defined herein with a humanized anti-CD79b antibody wherein the affinity of the antibody in its bivalent form to CD79b (e.g., affinity of the antibody as an IgG to CD79b) is 0.4 nM. In a further aspect, the invention provides a humanized anti-CD79b antibody wherein the affinity of the antibody in its bivalent form to CD79b (e.g., affinity of the antibody as an IgG to CD79b) is 0.4 nM +/- 0.04.

In another aspect, the invention provides a combination of an anti-CD20 antibody as defined herein with a humanized anti-CD79b antibody wherein the affinity of the antibody in its bivalent form to CD79b (e.g., affinity of the antibody as an IgG to CD79b) is 0.3 nM or better. In another aspect, the invention provides a humanized anti-CD79b antibody wherein the affinity of the antibody in its bivalent form to CD79b (e.g., affinity of the antibody as an IgG to CD79b) is 0.32 nM or better. In another aspect, the invention provides a humanized anti-CD79b antibody wherein the affinity of the antibody in its bivalent form to CD79b (e.g., affinity of the antibody as an IgG to CD79b) is 0.36 nM or better. In another aspect, the invention provides a humanized anti-CD79b antibody wherein the affinity of the antibody in its bivalent form to CD79b (e.g., affinity of the antibody as an IgG to CD79b) is 0.4 nM or better. In another aspect, the invention provides a humanized anti-CD79b antibody wherein the affinity of the antibody in its bivalent form to CD79b (e.g., affinity of the antibody as an IgG to CD79b) is 0.44 nM or better. In another aspect, the invention provides a humanized anti-CD79b antibody wherein the affinity of the antibody in its bivalent form to CD79b (e.g., affinity of the antibody as an IgG to CD79b) is 0.48 nM or better. In another aspect, the invention provides a humanized anti-CD79b antibody wherein the affinity of the antibody in its bivalent form to CD79b (e.g., affinity of the antibody as an IgG to CD79b) is 0.5 nM or better. In another aspect, the invention provides a humanized anti-CD79b antibody wherein the affinity of the antibody in its bivalent form to CD79b (e.g., affinity of the antibody as an IgG to CD79b) is between 0.3 nM and 0.5 nM. In another aspect, the invention provides a humanized anti-CD79b antibody wherein the affinity of the antibody in its bivalent form to CD79b (e.g., affinity of the antibody as an IgG to CD79b) is between 0.32 nM and 0.48 nM. In another aspect, the invention provides a humanized anti-CD79b antibody wherein the affinity of the antibody in its bivalent form to CD79b (e.g., affinity of the antibody as an IgG to CD79b) is between 0.36 nM and 0.44 nM.

In another aspect, the invention provides a combination of an anti-CD20 antibody as defined herein with a humanized anti-CD79b antibody wherein the affinity of the antibody in its bivalent form to CD79b (e.g., affinity of the antibody as an IgG to CD79b) is 0.2 nM. In a further aspect, the invention provides a humanized anti-CD79b antibody wherein the affinity of the antibody in its bivalent form to CD79b (e.g., affinity of the antibody as an IgG to CD79b) is 0.2 nM +/- 0.02.

In another aspect, the invention provides a combination of an anti-CD20 antibody as defined herein with a humanized anti-CD79b antibody wherein the affinity of the antibody in its bivalent form to CD79b (e.g., affinity of the antibody as an IgG to CD79b) is 0.1 nM or better. In another aspect, the invention provides a humanized anti-CD79b antibody wherein the affinity of the antibody in its bivalent form to CD79b (e.g., affinity of the antibody as an IgG to CD79b) is 0.12 nM or better. In another aspect, the invention provides a humanized anti-CD79b antibody wherein the affinity of the antibody in its bivalent form to CD79b (e.g., affinity of the antibody as an IgG to CD79b) is 0.14 nM or better. In another aspect, the invention provides a humanized anti-CD79b antibody wherein the affinity of the antibody in its bivalent form to CD79b (e.g., affinity of the antibody as an IgG to CD79b) is 0.16 nM or better. In another aspect, the invention provides a humanized anti-CD79b antibody wherein the affinity of the antibody in its bivalent form to CD79b (e.g., affinity of the antibody as an IgG to CD79b) is 0.18 nM or better. In another aspect, the invention provides a humanized anti-CD79b antibody wherein the affinity of the antibody in its bivalent form to CD79b (e.g., affinity of the antibody as an IgG to CD79b) is 0.2 nM or better. In another aspect, the invention provides a humanized anti-CD79b antibody wherein the affinity of the antibody in its bivalent form to CD79b (e.g., affinity of the antibody as an IgG to CD79b) is 0.22 nM or better. In another aspect, the invention provides a humanized anti-CD79b antibody wherein the affinity of the antibody in its bivalent form to CD79b (e.g., affinity of the antibody as an IgG to CD79b) is 0.24 nM or better. In another aspect, the invention provides a humanized anti-CD79b antibody wherein the affinity of the antibody in its bivalent form to CD79b (e.g., affinity of the antibody as an IgG to CD79b) is 0.26 nM or better. In another aspect, the invention provides a humanized anti-CD79b antibody wherein the affinity of the antibody in its bivalent form to CD79b (e.g., affinity of the antibody as an IgG to CD79b) is 0.28 nM or better. In another aspect, the invention provides a humanized anti-CD79b antibody wherein the affinity of the antibody in its bivalent form to CD79b (e.g., affinity of the antibody as an IgG to CD79b) is 0.30 nM or better. In another aspect, the invention provides a humanized anti-CD79b antibody wherein the affinity of the antibody in its bivalent form to CD79b (e.g., affinity of the antibody as an IgG to CD79b) is between 0.1 nM and 0.3 nM. In another aspect, the invention provides a humanized anti-CD79b antibody wherein the affinity of the antibody in its bivalent form to CD79b (e.g., affinity of the antibody as an IgG to CD79b) is between 0.12 nM and 0.28 nM. In another aspect, the invention provides a humanized anti-CD79b antibody wherein the affinity of the antibody in its bivalent form to CD79b (e.g., affinity of the antibody as an IgG to CD79b) is between 0.14 nM and 0.26 nM. In another aspect, the invention provides a humanized anti-CD79b antibody wherein the affinity of the antibody in its bivalent form to CD79b (e.g., affinity of the antibody as an IgG to CD79b) is between 0.16 nM and 0.24 nM. In another aspect, the invention provides a humanized anti-CD79b antibody wherein the affinity of the antibody in its bivalent form to CD79b (e.g., affinity of the antibody as an IgG to CD79b) is between 0.18 nM and 0.22 nM.

In another aspect, the invention provides a combination of an anti-CD20 antibody as defined herein with a humanized anti-CD79b antibody wherein the affinity of the antibody in its bivalent form to CD79b (e.g., affinity of the antibody as an IgG to CD79b) is 0.5 nM. In a further aspect, the invention provides a humanized anti-CD79b antibody wherein the affinity of the antibody in its bivalent form to CD79b (e.g., affinity of the antibody as an IgG to CD79b) is 0.5 nM +/- 0.1.

In another aspect, the invention provides a combination of an anti-CD20 antibody as defined herein with a humanized anti-CD79b antibody wherein the affinity of the antibody in its bivalent form to CD79b (e.g., affinity of the antibody as an IgG to CD79b) is 0.4 nM or better. In another aspect, the invention provides a humanized anti-CD79b antibody wherein the affinity of the antibody in its bivalent form to CD79b (e.g., affinity of the antibody as an IgG to CD79b) is 0.5 nM or better. In another aspect, the invention provides a humanized anti-CD79b antibody wherein the affinity of the antibody in its bivalent form to CD79b (e.g., affinity of the antibody as an IgG to CD79b) is 0.6 nM or better. In another aspect, the invention provides a humanized anti-CD79b antibody wherein the affinity of the antibody in its bivalent form to CD79b (e.g., affinity of the antibody as an IgG to CD79b) is 0.7 nM or better. In another aspect, the invention provides a humanized anti-CD79b antibody wherein the affinity of the antibody in its bivalent form to CD79b (e.g., affinity of the antibody as an IgG to CD79b) is between 0.3 nM and 0.7 nM. In another aspect, the invention provides a humanized anti-CD79b antibody wherein the affinity of the antibody in its bivalent form to CD79b (e.g., affinity of the antibody as an IgG to CD79b) is between 0.4 nM and 0.6 nM. In another aspect, the invention provides a humanized anti-CD79b antibody wherein the affinity of the antibody in its bivalent form to CD79b (e.g., affinity of the antibody as an IgG to CD79b) is between 0.5 nM and 0.55 nM.

In one aspect, the monovalent affinity of the murine antibody to CD79b is substantially the same as the binding affinity of a Fab fragment comprising variable domain sequences of SEQ ID NO: 26 (Figure 7) and SEQ ID NO: 29 (Figure 8). In another aspect, the monovalent affinity of the murine antibody to CD79b is substantially the same as the binding affinity of a Fab fragment comprising variable domain sequences of an antibody generated from hybridoma deposited with the ATCC as HB11413 on July 20, 1993 or chimeric antibody comprising the variable domains from antibody generated from hybridomas deposited with the ATCC as HB11413 on July 20, 1993.

As is well-established in the art, binding affinity of a ligand to its receptor can be determined using any of a variety of assays, and expressed in terms of a variety of quantitative values. Accordingly, in one embodiment, the binding affinity is expressed as Kd values and reflects intrinsic binding affinity (e.g., with minimized avidity effects). Generally and preferably, binding affinity is measured *in vitro,* whether in a cell-free or cell-associated setting. As described in greater detail herein, fold difference in binding affinity can be quantified in terms of the ratio of the monovalent binding affinity value of a humanized antibody (e.g., in Fab form) and the monovalent binding affinity value of a reference/comparator antibody (e.g., in Fab form) (e.g., a murine antibody having donor hypervariable region sequences), wherein the binding affinity values are determined under similar assay conditions. Thus, in one embodiment, the fold difference in binding affinity is determined as the ratio of the Kd values of the humanized antibody in Fab form and said reference/comparator Fab antibody. For example, in one embodiment, if an antibody of the invention (A) has an affinity that is "3-fold lower" than the affinity of a reference antibody (M), then if the Kd value for A is 3x, the Kd value of M would be 1x, and the ratio of Kd of A to Kd of M would be 3:1. Conversely, in one embodiment, if an antibody of the invention (C) has an affinity that is "3-fold greater" than the affinity of a reference antibody (R), then if the Kd value for C is 1x, the Kd value of R would be 3x, and the ratio of Kd of C to Kd of R would be 1:3. Any of a number of assays known in the art, including those described herein, can be used to obtain binding affinity measurements, including, for example, Biacore, radioimmunoassay (RIA) and ELISA.

In another aspect, the invention provides a humanized anti-CD79b antibody wherein the affinity of the antibody in its bivalent form to CD79b (e.g., affinity of the antibody as an IgG to CD79b) is 0.4 nM, 0.2 nM or 0.5 nM.

In one aspect, an antibody that binds to CD79b for combination of an anti-CD20 antibody as defined herein is provided, wherein the antibody comprises at least one, two, three, four, five or six HVRs selected from the group consisting of:
(i) HVR-L1 comprising sequence A1-A15, wherein A1-A15 is KASQSVDYDGDSFLN (SEQ ID NO: 31)
(ii) HVR-L2 comprising sequence B1-B7, wherein B1-B7 is AASNLES (SEQ ID NO: 32)
(iii) HVR-L3 comprising sequence C1-C9, wherein C1-C9 is QQSNEDPLT (SEQ ID NO: 33)
(iv) HVR-H1 comprising sequence D1-D10, wherein D1-D10 is GYTFSSYWIE (SEQ ID NO: 34)
(v) HVR-H2 comprising sequence E1-E18, wherein E1-E18 is GEILPGGGDTNYNEIFKG (SEQ ID NO: 35) and
(vi) HVR-H3 comprising sequence F1-F10, wherein F1-F10 IS TRRVPVYFDY (SEQ ID NO: 36).

In one aspect, a combination of an anti-CD20 antibody as defined herein with an antibody that binds to CD79b is provided, wherein the antibody comprises at least one variant HVR wherein the variant HVR sequence comprises modification of at least one residue of the sequence in SEQ ID NOs: 31, 32, 33, 34, 35 or 36.

In one aspect, the invention provides a combination of an anti-CD20 antibody as defined herein with an antibody comprising a heavy chain variable domain comprising the HVR1-HC, HVR2-HC and/or HVR3-HC sequence as depicted in Figure 3B (SEQ ID NO: 50-52).

In one aspect, the invention provides a combination of an anti-CD20 antibody as defined herein with an antibody comprising a light chain variable domain comprising HVR1-LC, HVR2-LC and/or HVR3-LC sequence as depicted in Figure 3A (SEQ ID NO: 47-49).

In one aspect, the invention provides a combination of an anti-CD20 antibody as defined herein with an antibody comprising a heavy chain variable domain comprising the HVR1-HC, HVR2-HC and/or HVR3-HC sequence as depicted in Figure 4B (SEQ ID NO: 58-60).

In one aspect, the invention provides a combination of an anti-CD20 antibody as defined herein with an antibody comprising a light chain variable domain comprising HVR1-LC, HVR2-LC and/or HVR3-LC sequence as depicted in Figure 4A (SEQ ID NO: 55-57).

In one aspect, the invention provides a combination of an anti-CD20 antibody as defined herein with an antibody comprising a heavy chain variable domain comprising the HVR1-HC, HVR2-HC and/or HVR3-HC sequence as depicted in Figure 5B (SEQ ID NO: 66-68).

In one aspect, the invention provides a combination of an anti-CD20 antibody as defined herein with an antibody comprising a light chain variable domain comprising HVR1-LC, HVR2-LC and/or HVR3-LC sequence as depicted in Figure 5A (SEQ ID NO: 63-65).

In one aspect, the invention provides a combination of an anti-CD20 antibody as defined herein with an antibody comprising a heavy chain variable domain comprising the HVR1-HC, HVR2-HC and/or HVR3-HC sequence as depicted in Figure 6B (SEQ ID NO: 74-76).

In one aspect, the invention provides a combination of an anti-CD20 antibody as defined herein with an antibody comprising a light chain variable domain comprising HVR1-LC, HVR2-LC and/or HVR3-LC sequence as depicted in Figure 6A (SEQ ID NO: 71-73).

In one aspect, the invention includes a combination of an anti-CD20 antibody as defined herein with an anti-CD79b antibody comprising a heavy chain variable domain selected from SEQ ID NOs: 54, 62, 70 or 78. In another aspect, the invention includes an anti-CD79b antibody comprising a light chain variable domain selected from SEQ ID NOs: 53, 61, 69 or 77.

In one aspect, the invention includes a combination of an anti-CD20 antibody as defined herein with a cysteine engineered anti-CD79b antibody comprising one or more free cysteine amino acids and a sequence selected from SEQ ID NOs: 83-130. The cysteine engineered anti-CD79b antibody may bind to a CD79b polypeptide. The cysteine engineered anti-CD79b antibody may be prepared by a process comprising replacing one or more amino acid residues of a parent anti-CD79b antibody by cysteine.

In one aspect, the invention includes a combination of an anti-CD20 antibody as defined herein with a cysteine engineered anti-CD79b antibody comprising one or more free cysteine amino acids wherein the cysteine engineered anti-CD79b antibody binds to a CD79b polypeptide and is prepared by a process comprising replacing one or more amino acid residues of a parent anti-CD79b antibody by cysteine wherein the parent antibody comprises at least one HVR sequence selected from:
(a) HVR-L1 comprising sequence A1-A15, wherein A1-A15 is KASQSVDYDGDSFLN (SEQ ID NO: 31) or KASQSVDYEGDSFLN (SEQ ID NO: 37);
(b) HVR-L2 comprising sequence B1-B7, wherein B1-B7 is AASNLES (SEQ ID NO: 32)
(c) HVR-L3 comprising sequence C1-C9, wherein C1-C9 is QQSNEDPLT (SEQ ID NO: 33)
(d) HVR-H1 comprising sequence D1-D10, wherein D1-D10 is GYTFSSYWIE (SEQ ID NO: 34)
(e) HVR-H2 comprising sequence E1-E18, wherein E1-E18 is GEILPGGGDTNYNEIFKG (SEQ ID NO: 35) and
(f) HVR-H3 comprising sequence F1-F10, wherein F1-F10 is TRRVPVYFDY (SEQ ID NO: 36) or TRRVPIRLDY (SEQ ID NO: 38).

The cysteine engineered anti-CD79b antibody may be a monoclonal antibody, antibody fragment, chimeric antibody, humanized antibody, single-chain antibody or antibody that competitively inhibits the binding of an anti-CD79b polypeptide antibody to its respective antigenic epitope. Antibodies of the present invention may optionally be conjugated to a growth inhibitory agent or cytotoxic agent such as a toxin, including, for example, an auristatin or maytansinoid. The antibodies of the present invention may optionally be produced in CHO cells or bacterial cells and preferably inhibit the growth or proliferation of or induce the death of a cell to which they bind. For diagnostic purposes, the antibodies of the present invention may be detectably labeled, attached to a solid support, or the like.

The term "CD79b antibody-drug conjugate ("ADC")" according to the combination invention may be of Formula I, below, wherein an antibody is conjugated (i.e., covalently attached) to one or more drug moieties (D) through an optional linker (L). ADCs may include thioMAb drug conjugates ("TDC").

Ab-(L-D)ₚ I

Accordingly, the antibody may be conjugated to the drug either directly or via a linker. In Formula I, p is the average number of drug moieties per antibody, which can range, e.g., from about 1 to about 20 drug moieties per antibody, and in certain embodiments, from 1 to about 8 drug moieties per antibody. The invention includes a composition comprising a mixture of antibody-drug compounds of Formula I where the average drug loading per antibody is about 2 to about 5, or about 3 to about 4.

In one embodiment of the combination according to the invention, the CD79b antibody-drug conjugate is anti-CD79b-MC-vc-PAB-MMAE.

### a. Exemplary Linkers

A linker may comprise one or more linker components. Exemplary linker components include 6-maleimidocaproyl ("MC"), maleimidopropanoyl ("MP"), valine-citrulline ("val-cit" or "vc"), alanine-phenylalanine ("ala-phe"), p-aminobenzyloxycarbonyl (a "PAB"), and those resulting from conjugation with linker reagents: N-Succinimidyl 4-(2-pyridylthio) pentanoate ("SPP"), N-succinimidyl 4-(N-maleimidomethyl) cyclohexane-1 carboxylate ("SMCC", also referred to herein as "MCC"), and N-Succinimidyl (4-iodo-acetyl) aminobenzoate ("SIAB"). Various linker components are known in the art, some of which are described below.

A linker may be a "cleavable linker," facilitating release of a drug in the cell. For example, an acid-labile linker (e.g., hydrazone), protease-sensitive (e.g., peptidase-sensitive) linker, photolabile linker, dimethyl linker or disulfide-containing linker (Chari et al., Cancer Research 52:127-131 (1992); U.S. Patent No. 5,208,020) may be used.

In certain embodiments, a linker is as shown in the following Formula II:

-Aₐ-W_{w}-Y_{y}- II

wherein A is a stretcher unit, and a is an integer from 0 to 1; W is an amino acid unit, and w is an integer from 0 to 12; Y is a spacer unit, and y is 0, 1, or 2; and Ab, D, and p are defined as above for Formula I. Exemplary embodiments of such linkers are described in US 2005-0238649 A1.

In some embodiments, a linker component may comprise a "stretcher unit" that links an antibody to another linker component or to a drug moiety. Exemplary stretcher units are shown below (wherein the wavy line indicates sites of covalent attachment to an antibody):

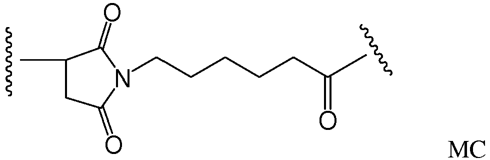

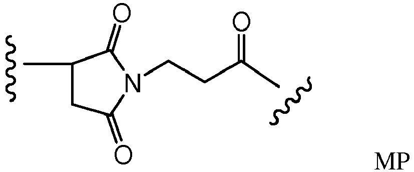

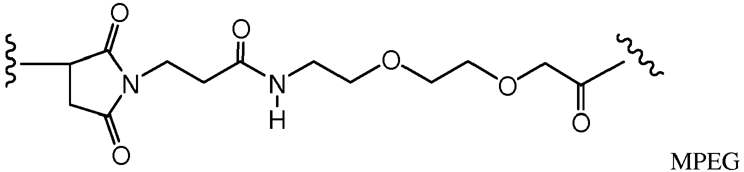

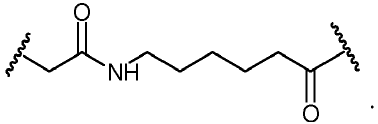

In some embodiments, a linker component may comprise an amino acid unit. In one such embodiment, the amino acid unit allows for cleavage of the linker by a protease, thereby facilitating release of the drug from the immunoconjugate upon exposure to intracellular proteases, such as lysosomal enzymes. *See, e.g.,* Doronina et al. (2003) Nat. Biotechnol. 21:778-784. Exemplary amino acid units include, but are not limited to, a dipeptide, a tripeptide, a tetrapeptide, and a pentapeptide. Exemplary dipeptides include: valine-citrulline (vc or val-cit), alanine-phenylalanine (af or ala-phe); phenylalanine-lysine (fk or phe-lys); or N-methyl-valine-citrulline (Me-val-cit). Exemplary tripeptides include: glycine-valine-citrulline (gly-val-cit) and glycine-glycine-glycine (gly-gly-gly). An amino acid unit may comprise amino acid residues that occur naturally, as well as minor amino acids and non-naturally occurring amino acid analogs, such as citrulline. Amino acid units can be designed and optimized in their selectivity for enzymatic cleavage by a particular enzyme, for example, a tumor-associated protease, cathepsin B, C and D, or a plasmin protease.

In some embodiments, a linker component may comprise a "spacer" unit that links the antibody to a drug moiety, either directly or by way of a stretcher unit and/or an amino acid unit. A spacer unit may be "self-immolative" or a "non-self-immolative." A "non-self-immolative" spacer unit is one in which part or all of the spacer unit remains bound to the drug moiety upon enzymatic (e.g., proteolytic) cleavage of the ADC. Examples of non-self-immolative spacer units include, but are not limited to, a glycine spacer unit and a glycine-glycine spacer unit. Other combinations of peptidic spacers susceptible to sequence-specific enzymatic cleavage are also contemplated. For example, enzymatic cleavage of an ADC containing a glycine-glycine spacer unit by a tumor-cell associated protease would result in release of a glycine-glycine-drug moiety from the remainder of the ADC. In one such embodiment, the glycine-glycine-drug moiety is then subjected to a separate hydrolysis step in the tumor cell, thus cleaving the glycine-glycine spacer unit from the drug moiety.

A "self-immolative" spacer unit allows for release of the drug moiety without a separate hydrolysis step. In certain embodiments, a spacer unit of a linker comprises a p-aminobenzyl unit. In one such embodiment, a p-aminobenzyl alcohol is attached to an amino acid unit via an amide bond, and a carbamate, methylcarbamate, or carbonate is made between the benzyl alcohol and a cytotoxic agent. *See, e.g.,* Hamann et al. (2005) Expert Opin. Ther. Patents (2005) 15:1087-1103. In one embodiment, the spacer unit is p-aminobenzyloxycarbonyl (PAB). In certain embodiments, the phenylene portion of a p-amino benzyl unit is substituted with Qm, wherein Q is -C₁-C₈ alkyl, -O-(C₁-C₈ alkyl), -halogen,- nitro or -cyano; and m is an integer ranging from 0-4. Examples of self-immolative spacer units further include, but are not limited to, aromatic compounds that are electronically similar to p-aminobenzyl alcohol (*see, e.g.,* US 2005/0256030 A1), such as 2-aminoimidazol-5-methanol derivatives (Hay et al. (1999) Bioorg. Med. Chem. Lett. 9:2237) and ortho- or para-aminobenzylacetals. Spacers can be used that undergo cyclization upon amide bond hydrolysis, such as substituted and unsubstituted 4-aminobutyric acid amides (Rodrigues et al., Chemistry Biology, 1995, 2, 223); appropriately substituted bicyclo[2.2.1] and bicyclo[2.2.2] ring systems (Storm, et al., J. Amer. Chem. Soc., 1972, 94, 5815); and 2-aminophenylpropionic acid amides (Amsberry, et al., J. Org. Chem., 1990, 55, 5867). Elimination of amine-containing drugs that are substituted at the α-position of glycine (Kingsbury, et al., J. Med. Chem., 1984, 27, 1447) are also examples of self-immolative spacers useful in ADCs.

In one embodiment, a spacer unit is a branched bis(hydroxymethyl)styrene (BHMS) unit as depicted below, which can be used to incorporate and release multiple drugs. 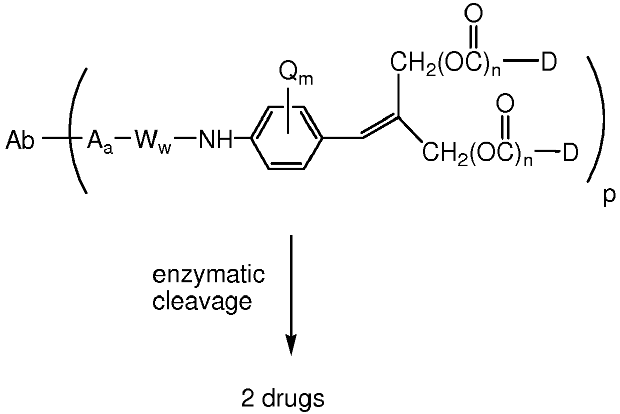 wherein Q is -C₁-C₈ alkyl, -O-(C₁-C₈ alkyl), -halogen, -nitro or -cyano; m is an integer ranging from 0-4; n is 0 or 1; and p ranges raging from 1 to about 20.

In another embodiment, linker L may be a dendritic type linker for covalent attachment of more than one drug moiety through a branching, multifunctional linker moiety to an antibody (Sun et al (2002) Bioorganic & Medicinal Chemistry Letters 12:2213-2215; Sun et al (2003) Bioorganic & Medicinal Chemistry 11:1761-1768). Dendritic linkers can increase the molar ratio of drug to antibody, i.e. loading, which is related to the potency of the ADC. Thus, where a cysteine engineered antibody bears only one reactive cysteine thiol group, a multitude of drug moieties may be attached through a dendritic linker.

Exemplary linker components and combinations thereof are shown below in the context of ADCs of Formula II:

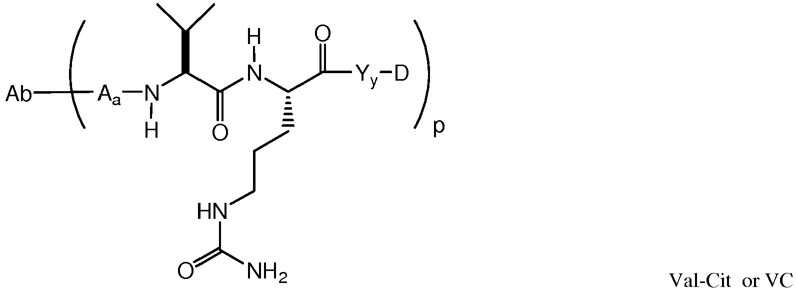

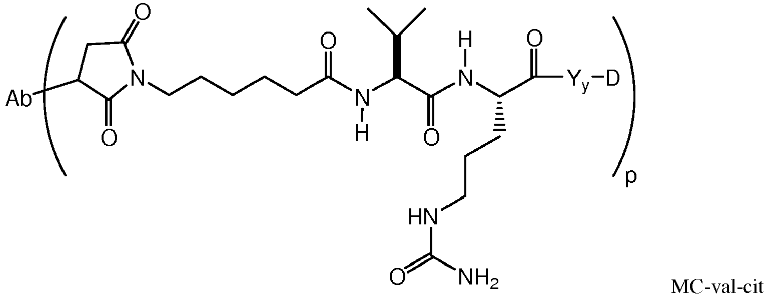

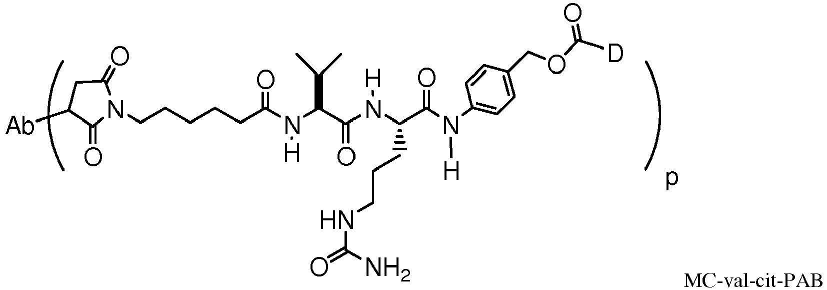

Linkers components, including stretcher, spacer, and amino acid units, may be synthesized by methods known in the art, such as those described in US 2005-0238649 A1.

### b. Exemplary Drug Moieties

### (1) Maytansine and maytansinoids

In some embodiments, a combination of an anti-CD20 antibody as defined herein with an immunoconjugate comprises an antibody conjugated to one or more maytansinoid molecules. Maytansinoids are mitototic inhibitors which act by inhibiting tubulin polymerization. Maytansine was first isolated from the east African shrub Maytenus serrata (U.S. Patent No. 3896111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Maytansinoid drug moieties are attractive drug moieties in antibody-drug conjugates because they are: (i) relatively accessible to prepare by fermentation or chemical modification or derivatization of fermentation products, (ii) amenable to derivatization with functional groups suitable for conjugation through disulfide and non-disulfide linkers to antibodies, (iii) stable in plasma, and (iv) effective against a variety of tumor cell lines.

Maytansine compounds suitable for use as maytansinoid drug moieties are well known in the art and can be isolated from natural sources according to known methods or produced using genetic engineering and fermentation techniques (US 6790952; US 2005/0170475; Yu et al (2002) PNAS 99:7968-7973). Maytansinol and maytansinol analogues may also be prepared synthetically according to known methods.

Exemplary maytansinoid drug moieties include those having a modified aromatic ring, such as: C-19-dechloro (US Pat. No. 4256746) (prepared by lithium aluminum hydride reduction of ansamytocin P2); C-20-hydroxy (or C-20-demethyl) +/-C-19-dechloro (US Pat. Nos. 4361650 and 4307016) (prepared by demethylation using *Streptomyces* or *Actinomyces* or dechlorination using LAH); and C-20-demethoxy, C-20-acyloxy (-OCOR), +/-dechloro (U.S. Pat. No. 4,294,757) (prepared by acylation using acyl chlorides). and those having modifications at other positions.

Exemplary maytansinoid drug moieties also include those having modifications such as: C-9-SH (US Pat. No. 4424219) (prepared by the reaction of maytansinol with H₂S or P₂S₅); C-14-alkoxymethyl(demethoxy/CH₂ OR)(US 4331598); C-14-hydroxymethyl or acyloxymethyl (CH₂OH or CH₂OAc) (US Pat. No. 4450254) (prepared from Nocardia); C-15-hydroxy/acyloxy (US 4364866) (prepared by the conversion of maytansinol by Streptomyces); C-15-methoxy (US Pat. Nos. 4313946 and 4315929) (isolated from Trewia nudlflora); C-18-N-demethyl (US Pat. Nos. 4362663 and 4322348) (prepared by the demethylation of maytansinol by Streptomyces); and 4,5-deoxy (US 4371533) (prepared by the titanium trichloride/LAH reduction of maytansinol).

Many positions on maytansine compounds are known to be useful as the linkage position, depending upon the type of link. For example, for forming an ester linkage, the C-3 position having a hydroxyl group, the C-14 position modified with hydroxymethyl, the C-15 position modified with a hydroxyl group and the C-20 position having a hydroxyl group are all suitable (US 5208020; US RE39151; US 6913748; US 7368565; US 2006/0167245; US 2007/0037972).

Maytansinoid drug moieties include those having the structure: 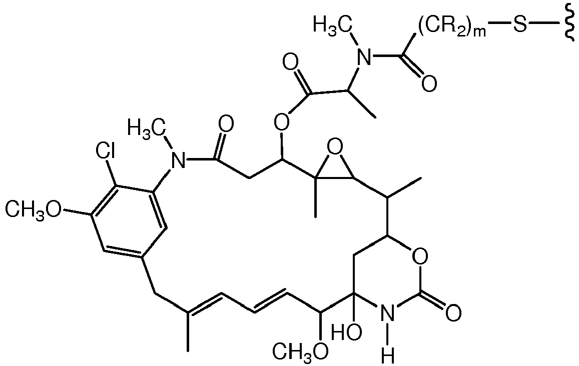 where the wavy line indicates the covalent attachment of the sulfur atom of the maytansinoid drug moiety to a linker of an ADC. R may independently be H or a C₁-C₆ alkyl. The alkylene chain attaching the amide group to the sulfur atom may be methanyl, ethanyl, or propyl, i.e., m is 1, 2, or 3 (US 633410 ; US 5208020; US 7276497; Chari et al (1992) Cancer Res. 52:127-131; Liu et al (1996) Proc. Natl. Acad. Sci USA 93:8618-8623).

All stereoisomers of the maytansinoid drug moiety are contemplated for the compounds of the invention, i.e. any combination of R and S configurations at the chiral carbons of D. In one embodiment, the maytansinoid drug moiety will have the following stereochemistry:

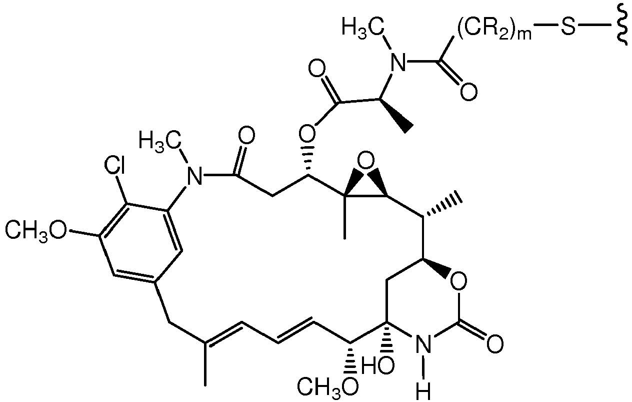

Exemplary embodiments of maytansinoid drug moieities include: DM1; DM3; and DM4, having the structures: 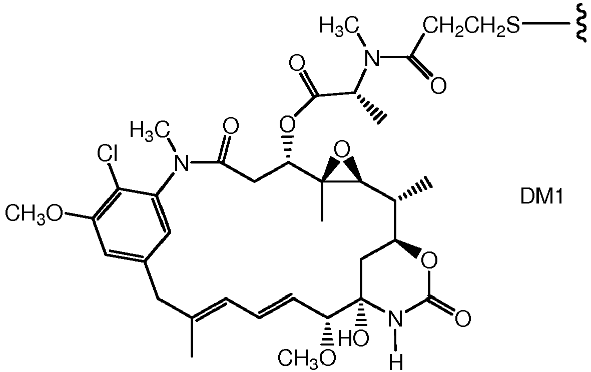 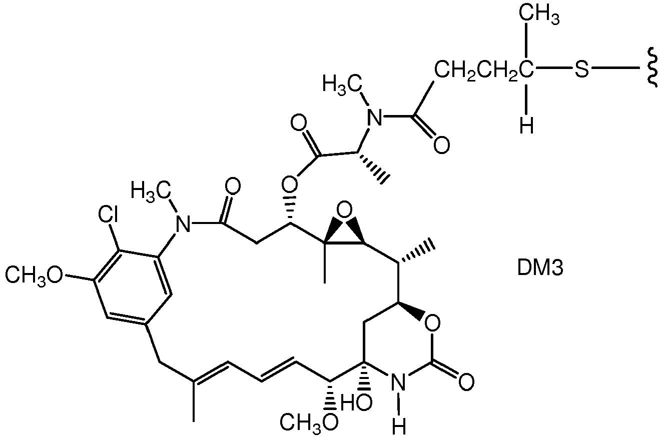 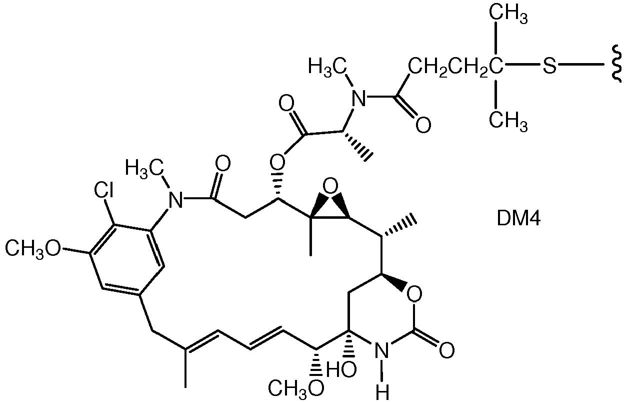 wherein the wavy line indicates the covalent attachment of the sulfur atom of the drug to a linker (L) of an antibody-drug conjugate. (WO 2005/037992; US 2005/0276812 A1).

Other exemplary maytansinoid antibody-drug conjugates have the following structures and abbreviations, (wherein Ab is antibody and p is 1 to about 8):

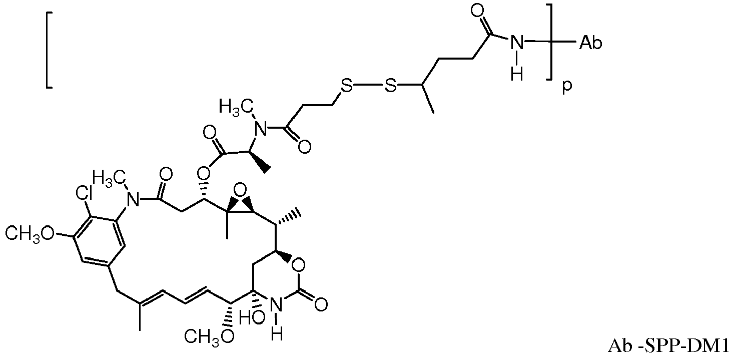

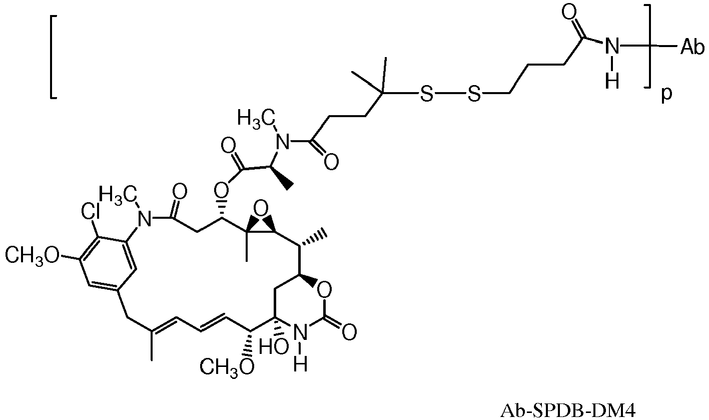

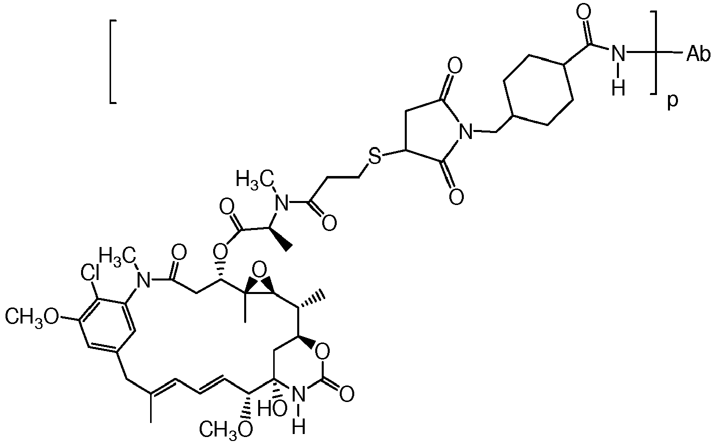

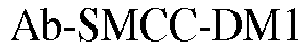

Exemplary antibody-drug conjugates where DM1 is linked through a BMPEO linker to a thiol group of the antibody have the structure and abbreviation: 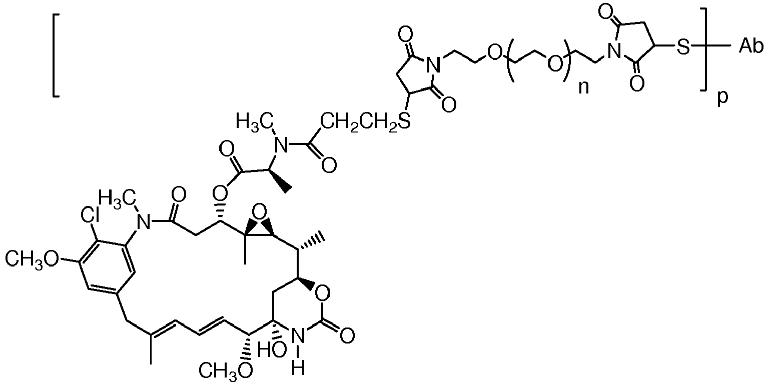 where Ab is antibody; n is 0, 1, or 2; and p is 1, 2, 3, or 4.

Immunoconjugates containing maytansinoids, methods of making the same, and their therapeutic use are disclosed, for example, in Erickson, et al (2006) Cancer Res. 66(8):4426-4433; U.S. Patent Nos. 5,208,020, 5,416,064, US 2005/0276812 A1, and European Patent EP 0 425 235 B1.

Antibody-maytansinoid conjugates according to the present combination invention are prepared by chemically linking an antibody to a maytansinoid molecule without significantly diminishing the biological activity of either the antibody or the maytansinoid molecule. See, e.g., U.S. Patent No. 5,208,020. Maytansinoids can be synthesized by known techniques or isolated from natural sources. Suitable maytansinoids are disclosed, for example, in U.S. Patent No. 5,208,020 and in the other patents and nonpatent publications referred to hereinabove, such as maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5208020 or EP Patent 0 425 235 B1; Chari et al. Cancer Research 52:127-131 (1992); and US 2005/016993 A1. Antibody-maytansinoid conjugates comprising the linker component SMCC may be prepared as disclosed in US 2005/0276812 A1, "Antibody-drug conjugates and Methods." The linkers comprise disulfide groups, thioether groups, acid labile groups, photolabile groups, peptidase labile groups, or esterase labile groups, as disclosed in the above-identified patents. Additional linkers are described and exemplified herein.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). In certain embodiments, the coupling agent is N-succinimidyl-3-(2-pyridyldithio) propionate (SPDP) (Carlsson et al., Biochem. J. 173:723-737 (1978)) or N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hydroxymethyl, the C-15 position modified with a hydroxyl group, and the C-20 position having a hydroxyl group. In one embodiment, the linkage is formed at the C-3 position of maytansinol or a maytansinol analogue.

### (2) Auristatins and dolastatins

In some embodiments, a combination of an anti-CD20 antibody as defined herein with an immunoconjugate comprises an antibody conjugated to dolastatin or a dolastatin peptidic analog or derivative, e.g., an auristatin (US Pat. Nos. 5635483; 5780588). Dolastatins and auristatins have been shown to interfere with microtubule dynamics, GTP hydrolysis, and nuclear and cellular division (Woyke et al (2001) Antimicrob. Agents and Chemother. 45(12):3580-3584) and have anticancer (US Pat. No.5663149) and antifungal activity (Pettit et al (1998) Antimicrob. Agents Chemother. 42:2961-2965). The dolastatin or auristatin drug moiety may be attached to the antibody through the N (amino) terminus or the C (carboxyl) terminus of the peptidic drug moiety (WO 02/088172).

Exemplary auristatin embodiments include the N-terminus linked monomethylauristatin drug moieties DE and DF (US 2005/0238649, disclosed in Senter et al, Proceedings of the American Association for Cancer Research, Volume 45, Abstract Number 623, presented March 28, 2004).

A peptidic drug moiety may be selected from Formulas D_{E} and D_{F} below: 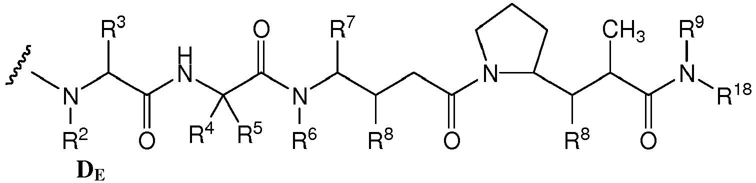 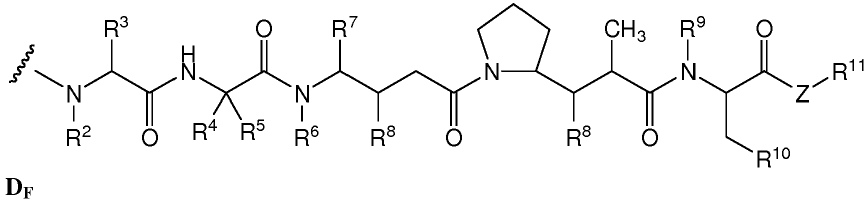 wherein the wavy line of D_{E} and D_{F} indicates the covalent attachment site to an antibody or antibody-linker component, and independently at each location:
R² is selected from H and C₁-C₈ alkyl;
R³ is selected from H, C₁-C₈ alkyl, C₃-C₈ carbocycle, aryl, C₁-C₈ alkyl-aryl, C₁-C₈ alkyl-(C₃-C₈ carbocycle), C₃-C₈ heterocycle and C₁-C₈ alkyl-(C₃-C₈ heterocycle);
R⁴ is selected from H, C₁-C₈ alkyl, C₃-C₈ carbocycle, aryl, C₁-C₈ alkyl-aryl, C₁-C₈ alkyl-(C₃-C₈ carbocycle), C₃-C₈ heterocycle and C₁-C₈ alkyl-(C₃-C₈ heterocycle);
R⁵ is selected from H and methyl;
or R⁴ and R⁵ jointly form a carbocyclic ring and have the formula -(CR^{a}R^{b})ₙ- wherein R^{a} and R^{b} are independently selected from H, C₁-C₈ alkyl and C₃-C₈ carbocycle and n is selected from 2, 3, 4, 5 and 6;
R⁶ is selected from H and C₁-C₈ alkyl;
R⁷ is selected from H, C₁-C₈ alkyl, C₃-C₈ carbocycle, aryl, C₁-C₈ alkyl-aryl, C₁-C₈ alkyl-(C₃-C₈ carbocycle), C₃-C₈ heterocycle and C₁-C₈ alkyl-(C₃-C₈ heterocycle);
each R⁸ is independently selected from H, OH, C₁-C₈ alkyl, C₃-C₈ carbocycle and O-(C₁-C₈ alkyl);
R⁹ is selected from H and C₁-C₈ alkyl;
R¹⁰ is selected from aryl or C₃-C₈ heterocycle;
Z is O, S, NH, or NR¹², wherein R¹² is C₁-C₈ alkyl;
R¹¹ is selected from H, C₁-C₂₀ alkyl, aryl, C₃-C₈ heterocycle, -(R¹³O)ₘ-R¹⁴, or -(R¹³O)ₘ-CH(R¹⁵)₂;
m is an integer ranging from 1-1000;
R¹³ is C₂-C₈ alkyl;
R¹⁴ is H or C₁-C₈ alkyl;
each occurrence of R¹⁵ is independently H, COOH, -(CH₂)ₙ-N(R¹⁶)₂, -(CH₂)ₙ-SO₃H, or -(CH₂)ₙ-SO₃-C₁-C₈ alkyl;
each occurrence of R¹⁶ is independently H, C₁-C₈ alkyl, or -(CH₂)ₙ-COOH;
R¹⁸ is selected from -C(R⁸)₂-C(R⁸)₂-aryl, -C(R⁸)₂-C(R⁸)₂-(C₃-C₈ heterocycle), and -C(R⁸)₂-C(R⁸)₂-(C₃-C₈ carbocycle); and
n is an integer ranging from 0 to 6.

In one embodiment, R³, R⁴ and R⁷ are independently isopropyl or sec-butyl and R⁵ is -H or methyl. In an exemplary embodiment, R³ and R⁴ are each isopropyl, R⁵ is -H, and R⁷ is sec-butyl.

In yet another embodiment, R² and R⁶ are each methyl, and R⁹ is -H.

In still another embodiment, each occurrence of R⁸ is -OCH_{3.}

In an exemplary embodiment, R³ and R⁴ are each isopropyl, R² and R⁶ are each methyl, R⁵ is -H, R⁷ is sec-butyl, each occurrence of R⁸ is -OCH₃, and R⁹ is -H.

In one embodiment, Z is -O- or -NH-.

In one embodiment, R¹⁰ is aryl.

In an exemplary embodiment, R¹⁰ is -phenyl.

In an exemplary embodiment, when Z is -O-, R¹¹ is -H, methyl or t-butyl.

In one embodiment, when Z is -NH, R¹¹ is -CH(R¹⁵)₂, wherein R¹⁵ is -(CH₂)ₙ-N(R¹⁶)₂, and R¹⁶ is -C₁-C₈ alkyl or -(CH₂)ₙ-COOH.

In another embodiment, when Z is -NH, R¹¹ is -CH(R¹⁵)₂, wherein R¹⁵ is -(CH₂)ₙ-SO₃H.

An exemplary auristatin embodiment of formula D_{E} is MMAE, wherein the wavy line indicates the covalent attachment to a linker (L) of an antibody-drug conjugate:

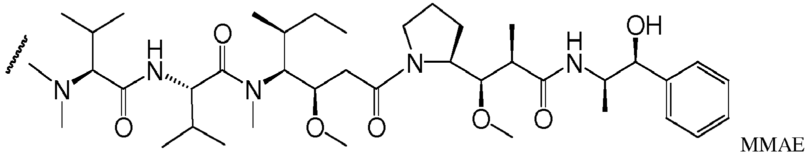

An exemplary auristatin embodiment of formula D_{F} is MMAF, wherein the wavy line indicates the covalent attachment to a linker (L) of an antibody-drug conjugate (*see* US 2005/0238649 and Doronina et al. (2006) Bioconjugate Chem. 17:114-124):

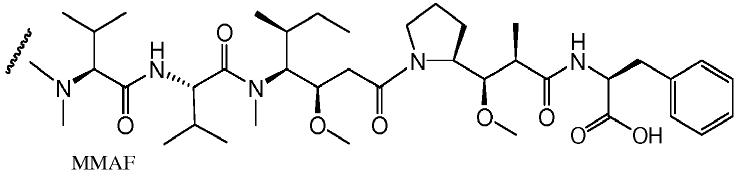

Other exemplary embodiments include monomethylvaline compounds having phenylalanine carboxy modifications at the C-terminus of the pentapeptide auristatin drug moiety (WO 2007/008848) and monomethylvaline compounds having phenylalanine sidechain modifications at the C-terminus of the pentapeptide auristatin drug moiety (WO 2007/008603).

Other drug moieties include the following MMAF derivatives, wherein the wavy line indicates the covalent attachment to a linker (L) of an antibody-drug conjugate: 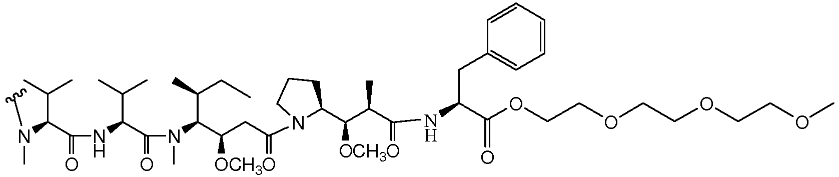 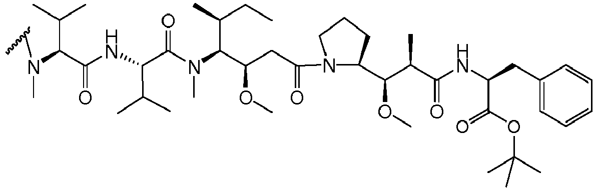 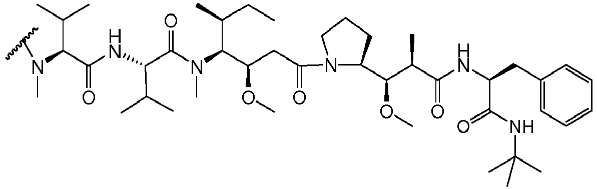 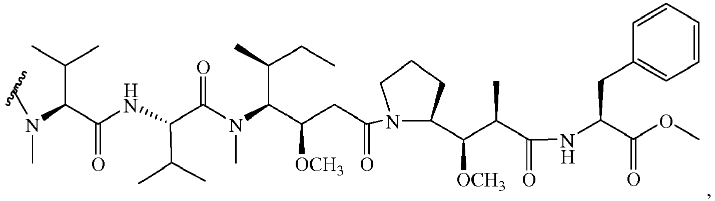 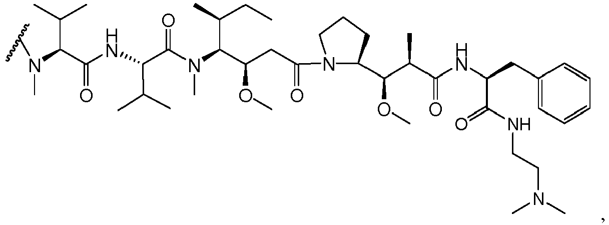 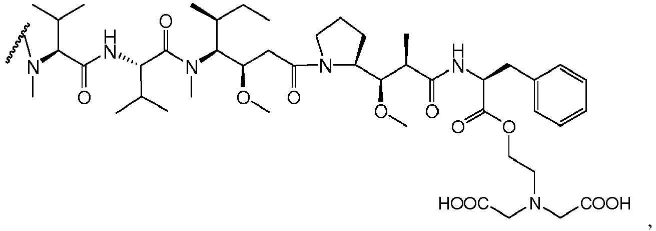 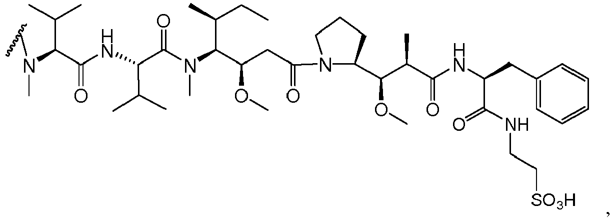 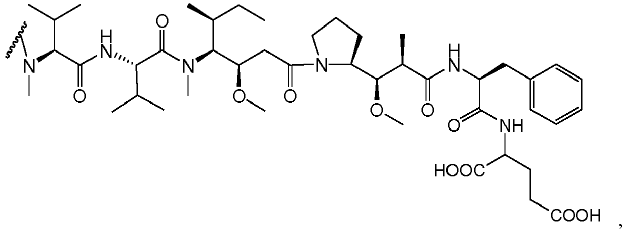 and

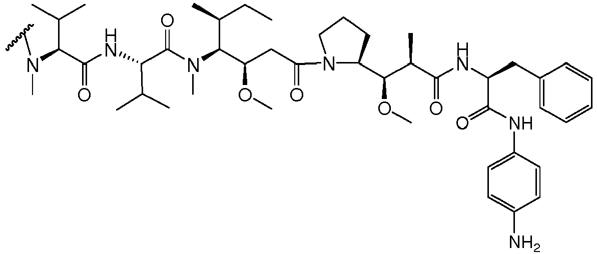

In one aspect, hydrophilic groups including but not limited to, triethylene glycol esters (TEG), as shown above, can be attached to the drug moiety at R¹¹. Without being bound by any particular theory, the hydrophilic groups assist in the internalization and non-agglomeration of the drug moiety.

Exemplary embodiments of a combination of an anti-CD20 antibody as defined herein with ADCs of Formula I comprising an auristatin/dolastatin or derivative thereof are described in US 2005-0238649 and Doronina et al. (2006) Bioconjugate Chem. 17:114-124. Exemplary embodiments of ADCs of Formula I comprising MMAE or MMAF and various linker components have the following structures and abbreviations (wherein "Ab" is an antibody; p is 1 to about 8, "Val-Cit" or "vc" is a valine-citrulline dipeptide; and "S" is a sulfur atom. It will be noted that in certain of the structural descriptions of sulfur linked ADC herein the antibody is represented as "Ab-S" merely to indicate the sulfur link feature and not to indicate that a particular sulfur atom bears multiple linker-drug moieties. The left parentheses of the following structures may also be placed to the left of the sulfur atom, between Ab and S, which would be an equivalent description of the ADC of the invention described throughout herein.

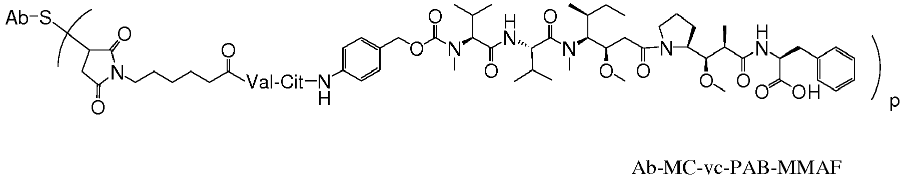

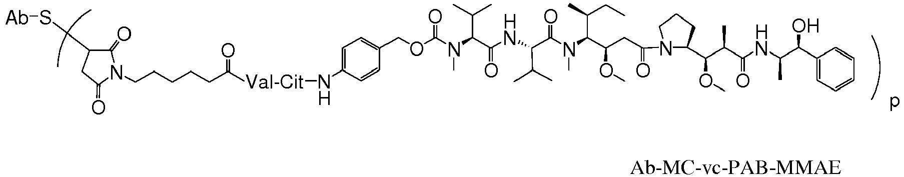

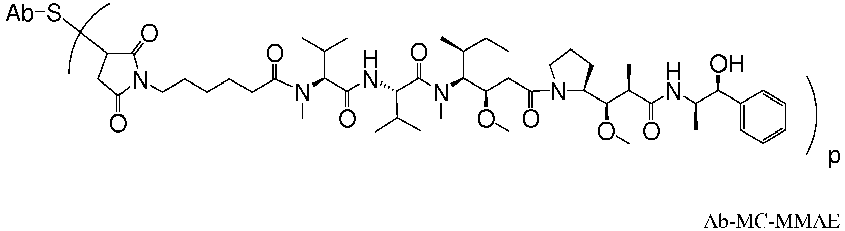

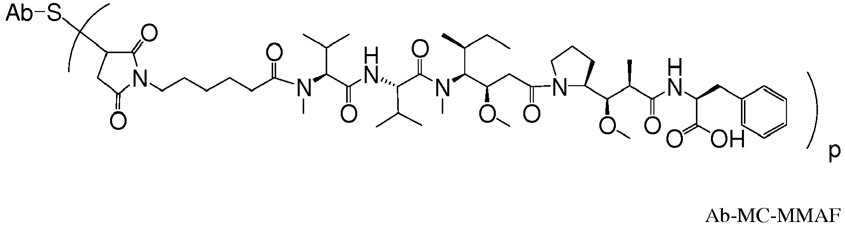

Exemplary embodiments of a combination of an anti-CD20 antibody as defined herein with ADCs of Formula I comprising MMAF and various linker components further include Ab-MC-PAB-MMAF and Ab-PAB-MMAF. Interestingly, immunoconjugates comprising MMAF attached to an antibody by a linker that is not proteolytically cleavable have been shown to possess activity comparable to immunoconjugates comprising MMAF attached to an antibody by a proteolytically cleavable linker. *See,* Doronina et al. (2006) Bioconjugate Chem. 17:114-124. In such instances, drug release is believed to be effected by antibody degradation in the cell. *Id.*

Typically, peptide-based drug moieties can be prepared by forming a peptide bond between two or more amino acids and/or peptide fragments. Such peptide bonds can be prepared, for example, according to the liquid phase synthesis method (see E. Schröder and K. Lübke, "The Peptides", volume 1, pp 76-136, 1965, Academic Press) that is well known in the field of peptide chemistry. Auristatin/dolastatin drug moieties may be prepared according to the methods of: US 2005-0238649 A1; US Pat. No.5635483; US Pat. No.5780588; Pettit et al (1989) J. Am. Chem. Soc. 111:5463-5465; Pettit et al (1998) Anti-Cancer Drug Design 13:243-277; Pettit, G.R., et al. Synthesis, 1996, 719-725; Pettit et al (1996) J. Chem. Soc. Perkin Trans. 1 5:859-863; and Doronina (2003) Nat. Biotechnol. 21(7):778-784.

In particular, auristatin/dolastatin drug moieties of formula D_{F}, such as MMAF and derivatives thereof, may be prepared using methods described in US 2005-0238649 A1 and Doronina et al. (2006) Bioconjugate Chem. 17:114-124. Auristatin/dolastatin drug moieties of formula D_{E}, such as MMAE and derivatives thereof, may be prepared using methods described in Doronina et al. (2003) Nat. Biotech. 21:778-784. Drug-linker moieties MC-MMAF, MC-MMAE, MC-vc-PAB-MMAF, and MC-vc-PAB-MMAE may be conveniently synthesized by routine methods, e.g., as described in Doronina et al. (2003) Nat. Biotech. 21:778-784, and Patent Application Publication No. US 2005/0238649 A1, and then conjugated to an antibody of interest.

### (3) Calicheamicin

In other embodiments, a combination of an anti-CD20 antibody as defined herein with the immunoconjugate comprises an antibody conjugated to one or more calicheamicin molecules. The calicheamicin family of antibiotics are capable of producing double-stranded DNA breaks at sub-picomolar concentrations. For the preparation of conjugates of the calicheamicin family, see U.S. Pat. Nos. 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I}, PSAG and θ^{I}₁ (Hinman et al., Cancer Research 53:3336-3342 (1993), Lode et al., Cancer Research 58:2925-2928 (1998), and the aforementioned U.S. patents to American Cyanamid). Another anti-tumor drug to which the antibody can be conjugated is QFA, which is an antifolate. Both calicheamicin and QFA have intracellular sites of action and do not readily cross the plasma membrane. Therefore, cellular uptake of these agents through antibody-mediated internalization greatly enhances their cytotoxic effects.

### c. Other cytotoxic agents

Other antitumor agents that can be conjugated to an antibody include BCNU, streptozocin, vincristine and 5-fluorouracil, the family of agents known collectively as the LL-E33288 complex, described in US Pat. Nos. 5,053,394, 5,770,710, as well as esperamicins (US Pat. No. 5,877,296).

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates a combination of an anti-CD20 antibody as defined herein with an immunoconjugate formed between an antibody and a compound with nucleolytic activity (e.g., a ribonuclease or a DNA endonuclease such as a deoxyribonuclease; DNase).

In certain embodiments, a combination of an anti-CD20 antibody as defined herein with an immunoconjugate may comprise a highly radioactive atom. A variety of radioactive isotopes are available for the production of radioconjugated antibodies. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the immunoconjugate is used for detection, it may comprise a radioactive atom for scintigraphic studies, for example tc^{99m} or I¹²³, or a spin label for nuclear magnetic resonance (NMR) imaging (also known as magnetic resonance imaging, mri), such as iodine-123, iodine-131, indium-111, fluorine-19, carbon-13, nitrogen-15, oxygen-17, gadolinium, manganese or iron.

The radio- or other labels may be incorporated in the immunoconjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal, CRC Press 1989) describes other methods in detail.

In certain embodiments, a combination of an anti-CD20 antibody as defined herein with an immunoconjugate may comprise an antibody conjugated to a prodrug-activating enzyme that converts a prodrug (e.g., a peptidyl chemotherapeutic agent, see WO 81/01145) to an active drug, such as an anti-cancer drug. Such immunoconjugates are useful in antibody-dependent enzyme-mediated prodrug therapy ("ADEPT"). Enzymes that may be conjugated to an antibody include, but are not limited to, alkaline phosphatases, which are useful for converting phosphate-containing prodrugs into free drugs; arylsulfatases, which are useful for converting sulfate-containing prodrugs into free drugs; cytosine deaminase, which is useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), which are useful for converting peptide-containing prodrugs into free drugs; D-alanylcarboxypeptidases, which are useful for converting prodrugs that contain D-amino acid substituents; carbohydrate-cleaving enzymes such as β-galactosidase and neuraminidase, which are useful for converting glycosylated prodrugs into free drugs; β-lactamase, which is useful for converting drugs derivatized with β-lactams into free drugs; and penicillin amidases, such as penicillin V amidase and penicillin G amidase, which are useful for converting drugs derivatized at their amine nitrogens with phenoxyacetyl or phenylacetyl groups, respectively, into free drugs. Enzymes may be covalently bound to antibodies by recombinant DNA techniques well known in the art. See, e.g., Neuberger et al., Nature 312:604-608 (1984).

### d. Drug Loading

Drug loading is represented by p, the average number of drug moieties per antibody in a molecule of Formula I. Drug loading may range from 1 to 20 drug moieties (D) per antibody. ADCs of Formula I include collections of antibodies conjugated with a range of drug moieties, from 1 to 20. The average number of drug moieties per antibody in preparations of ADC from conjugation reactions may be characterized by conventional means such as mass spectroscopy, ELISA assay, and HPLC. The quantitative distribution of ADC in terms of p may also be determined. In some instances, separation, purification, and characterization of homogeneous ADC where p is a certain value from ADC with other drug loadings may be achieved by means such as reverse phase HPLC or electrophoresis. Pharmaceutical formulations of Formula I antibody-drug conjugates may thus be a heterogeneous mixture of such conjugates with antibodies linked to 1, 2, 3, 4, or more drug moieties.

For some antibody-drug conjugates, p may be limited by the number of attachment sites on the antibody. For example, where the attachment is a cysteine thiol, as in the exemplary embodiments above, an antibody may have only one or several cysteine thiol groups, or may have only one or several sufficiently reactive thiol groups through which a linker may be attached. In certain embodiments, higher drug loading, e.g. p >5, may cause aggregation, insolubility, toxicity, or loss of cellular permeability of certain antibody-drug conjugates. In certain embodiments, the drug loading for an ADC of the invention ranges from 1 to about 8; from about 2 to about 6; or from about 3 to about 5. Indeed, it has been shown that for certain ADCs, the optimal ratio of drug moieties per antibody may be less than 8, and may be about 2 to about 5. *See* US 2005-0238649 A1.

In certain embodiments, fewer than the theoretical maximum of drug moieties are conjugated to an antibody during a conjugation reaction. An antibody may contain, for example, lysine residues that do not react with the drug-linker intermediate or linker reagent, as discussed below. Generally, antibodies do not contain many free and reactive cysteine thiol groups which may be linked to a drug moiety; indeed most cysteine thiol residues in antibodies exist as disulfide bridges. In certain embodiments, an antibody may be reduced with a reducing agent such as dithiothreitol (DTT) or tricarbonylethylphosphine (TCEP), under partial or total reducing conditions, to generate reactive cysteine thiol groups. In certain embodiments, an antibody is subjected to denaturing conditions to reveal reactive nucleophilic groups such as lysine or cysteine.

The loading (drug/antibody ratio) of an ADC may be controlled in different ways, e.g., by: (i) limiting the molar excess of drug-linker intermediate or linker reagent relative to antibody, (ii) limiting the conjugation reaction time or temperature, and (iii) partial or limiting reductive conditions for cysteine thiol modification.

It is to be understood that where more than one nucleophilic group reacts with a drug-linker intermediate or linker reagent followed by drug moiety reagent, then the resulting product is a mixture of ADC compounds with a distribution of one or more drug moieties attached to an antibody. The average number of drugs per antibody may be calculated from the mixture by a dual ELISA antibody assay, which is specific for antibody and specific for the drug. Individual ADC molecules may be identified in the mixture by mass spectroscopy and separated by HPLC, e.g. hydrophobic interaction chromatography (*see, e.g.,* McDonagh et al (2006) Prot. Engr. Design & Selection 19(7):299-307; Hamblett et al (2004) Clin. Cancer Res. 10:7063-7070; Hamblett, K.J., et al. "Effect of drug loading on the pharmacology, pharmacokinetics, and toxicity of an anti-CD30 antibody-drug conjugate," Abstract No. 624, American Association for Cancer Research, 2004 Annual Meeting, March 27-31, 2004, Proceedings of the AACR, Volume 45, March 2004; Alley, S.C., et al. "Controlling the location of drug attachment in antibody-drug conjugates," Abstract No. 627, American Association for Cancer Research, 2004 Annual Meeting, March 27-31, 2004, Proceedings of the AACR, Volume 45, March 2004). In certain embodiments, a homogeneous ADC with a single loading value may be isolated from the conjugation mixture by electrophoresis or chromatography.

### Preparation of antibody drug conjugates:

The antibody drug conjugates (ADC) of the combination invention as described herein may be prepared by processes as known to the person skilled in the art. Exemplary processes are e.g. disclosed in WO2009/099728. Said processes are e.g. described in WO2009/099728, paragraphs 538-545 (preparation of immunoconjugates), paragraphs 546-585 (preparation of exemplary immunoconjugates - Thio-Antibody Drug Conjugates), paragraphs 586-591 (Linkers), paragraphs 592-605 (Stretcher units), paragraphs 606-610 (Amino acid units), paragraphs 611-617 (Spacer units), paragraphs 618-624 (Dendritic linkers), paragraphs 625-632 (Linker reagents); paragraphs 633-636 (Preparation of cysteine engineered anti-CD79b antibody-drug conjugates).

### In Vitro Activity Assay for IC50 determination of a CD79b antibody-drug conjugate according to the combination invention

"IC50" refers to the concentration of a particular compound required to inhibit 50% of a specific measured activity. IC50 of the agents that inhibit the CD79b interaction can be measured, inter alia, as is described subsequently.

The term "cytotoxic activity" refers to a cell-killing, cytostatic or growth inhibitory effect of an antibody-drug conjugate or an intracellular metabolite of an antibody-drug conjugate. Cytotoxic activity may be expressed as the IC₅₀ value, which is the concentration (molar or mass) per unit volume at which half the cells survive.

### Surface expression of human CD79b on multiple lymphoma cell lines.

Nineteen lymphoma cell lines expressing varying amounts of CD79b on their surface were cultured and harvested in log phase growth. Cells were resuspended in FACS wash buffer (PBS; 0.5% bovine serum albumin; 0.1% sodium azide) containing 100 µg/ml each normal mouse IgG and normal human IgG and maintained on ice. Approximately 1 x 10⁶ cells/100 µl were stained with anti-huCD79b APC (mIgG1, clone RFB4, Southern Biotech #9361-11) or murine IgG1 APC isotype (BD Pharmingen #555751) for 30 minutes on ice. Dead cells were stained with 7-AAD (BD Pharmingen #559925). Data were acquired on a BD FacsCalibur™ flow cytometer and analyzed with FlowJo™ software. The IC50 determination for huMA79b.v28-MCvcPAB-DM1 or huMA79b.v28-MCvcPAB-MMAF or huMA79b.v28-MCvcPAB-MMAE or each free drug (DM1, MMAF, or MMAE) were determined by culturing lymphoma cells as above, harvesting the cultured cells in log phase and seeding 5,000 cells in 90 µl culture medium per well in 96 well plate. ADC and free drug were diluted serially within the detection range (starting at 300 µg/ml for ADC, or 90 nM for free drug and diluting to essentially zero assay target). Aliquots of 10 µl diluted ADC or free drug were added to replicate wells containing cells and incubated for 3 days at 37 °C. To each well, 100 µl CellTiter G1o™ was added and incubated for 30 min. Chemiluminescence was detected and data were analyzed using Prism™ software.

The oligosaccharide component can significantly affect properties relevant to the efficacy of a therapeutic glycoprotein, including physical stability, resistance to protease attack, interactions with the immune system, pharmacokinetics, and specific biological activity. Such properties may depend not only on the presence or absence, but also on the specific structures, of oligosaccharides. Some generalizations between oligosaccharide structure and glycoprotein function can be made. For example, certain oligosaccharide structures mediate rapid clearance of the glycoprotein from the bloodstream through interactions with specific carbohydrate binding proteins, while others can be bound by antibodies and trigger undesired immune reactions (Jenkins, N., et al., Nature Biotechnol. 14 (1996) 975-981).

Mammalian cells are the excellent hosts for production of therapeutic glycoproteins, due to their capability to glycosylate proteins in the most compatible form for human application (Cumming, D.A., et al., Glycobiology 1 (1991) 115-130; Jenkins, N., et al., Nature Biotechnol. 14 (1996) 975-981). Bacteria very rarely glycosylate proteins, and like other types of common hosts, such as yeasts, filamentous fungi, insect and plant cells, yield glycosylation patterns associated with rapid clearance from the blood stream, undesirable immune interactions, and in some specific cases, reduced biological activity. Among mammalian cells, Chinese hamster ovary (CHO) cells have been most commonly used during the last two decades. In addition to giving suitable glycosylation patterns, these cells allow consistent generation of genetically stable, highly productive clonal cell lines. They can be cultured to high densities in simple bioreactors using serum free media, and permit the development of safe and reproducible bioprocesses. Other commonly used animal cells include baby hamster kidney (BHK) cells, NSO- and SP2/0-mouse myeloma cells. More recently, production from transgenic animals has also been tested (Jenkins, N., et al., Nature Biotechnol. 14 (1996) 975-981).

All antibodies contain carbohydrate structures at conserved positions in the heavy chain constant regions, with each isotype possessing a distinct array of N-linked carbohydrate structures, which variably affect protein assembly, secretion or functional activity (Wright, A., and Morrison, S.L., Trends Biotech. 15 (1997) 26-32). The structure of the attached N-linked carbohydrate varies considerably, depending on the degree of processing, and can include high-mannose, multiply-branched as well as biantennary complex oligosaccharides (Wright, A., and Morrison, S.L., Trends Biotech. 15 (1997) 26-32). Typically, there is heterogeneous processing of the core oligosaccharide structures attached at a particular glycosylation site such that even monoclonal antibodies exist as multiple glycoforms. Likewise, it has been shown that major differences in antibody glycosylation occur between cell lines, and even minor differences are seen for a given cell line grown under different culture conditions (Lifely, M.R., et al., Glycobiology 5 (1995) 813-822).

One way to obtain large increases in potency, while maintaining a simple production process and potentially avoiding significant, undesirable side effects, is to enhance the natural, cell-mediated effector functions of monoclonal antibodies by engineering their oligosaccharide component as described in Umana, P. et al., Nature Biotechnol. 17 (1999) 176-180 and US 6,602,684. IgG1 type antibodies, the most commonly used antibodies in cancer immunotherapy, are glycoproteins that have a conserved N-linked glycosylation site at Asn297 in each CH2 domain. The two complex biantennary oligosaccharides attached to Asn297 are buried between the CH2 domains, forming extensive contacts with the polypeptide backbone, and their presence is essential for the antibody to mediate effector functions such as antibody dependent cellular cytotoxicity (ADCC) (Lifely, M.R., et al., Glycobiology 5 (1995) 813-822; Jefferis, R., et al., Immunol. Rev. 163 (1998) 59-76; Wright, A. and Morrison, S.L., Trends Biotechnol. 15 (1997) 26-32).

It was previously shown that overexpression in Chinese hamster ovary (CHO) cells of ß(1,4)-N-acetylglucosaminyltransferase I11 ("GnTII17y), a glycosyltransferase catalyzing the formation of bisected oligosaccharides, significantly increases the in vitro ADCC activity of an antineuroblastoma chimeric monoclonal antibody (chCE7) produced by the engineered CHO cells (see Umana, P. et al., Nature Biotechnol. 17 (1999) 176-180; and WO 99/154342). The antibody chCE7 belongs to a large class of unconjugated monoclonal antibodies which have high tumor affinity and specificity, but have too little potency to be clinically useful when produced in standard industrial cell lines lacking the GnTIII enzyme (Umana, P., et al., Nature Biotechnol. 17 (1999) 176-180). That study was the first to show that large increases of ADCC activity could be obtained by engineering the antibody producing cells to express GnTIII, which also led to an increase in the proportion of constant region (Fc)-associated, bisected oligosaccharides, including bisected, non-fucosylated oligosaccharides, above the levels found in naturally-occurring antibodies.

The term "cancer" as used herein includes lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, including refractory versions of any of the above cancers, or a combination of one or more of the above cancers. In one embodiment, the term cancer refers to a CD20 expressing cancer.

The term "expression of the CD20" antigen is intended to indicate an significant level of expression of the CD20 antigen in a cell, preferably on the cell surface of a T- or B- cell, more preferably a B-cell, from a tumor or cancer, respectively, preferably a non-solid tumor. Patients having a "CD20 expressing cancer" can be determined by standard assays known in the art. For example CD20 antigen expression can be measured using immunohistochemical (IHC) detection, FACS or via PCR-based detection of the corresponding mRNA.

The term "CD20 expressing cancer" as used herein refers to all cancers in which the cancer cells show an expression of the CD20 antigen. Preferably CD20 expressing cancer as used herein refers to lymphomas (preferably B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma) c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma) f) hairy cell leukemia, g ) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma j) Hodgkin's disease.

In one embodiment, the CD20 expressing cancer is a B-Cell Non-Hodgkin's lymphomas (NHL). In another embodiment, the CD20 expressing cancer is a Mantle cell lymphoma (MCL), acute lymphocytic leukemia (ALL), chronic lymphocytic leukemia (CLL), B-cell diffuse large cell lymphoma (DLCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia, or primary CNS lymphoma.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "co-administration" or "co-administering " refer to the administration of said afucosylated anti-CD20, and said CD79b antibody-drug conjugate as two separate formulations (or as one single formulation). The co-administration can be simultaneous or sequential in either order, wherein preferably there is a time period while both (or all) active agents simultaneously exert their biological activities. Said anti-CD20 afucosylated antibody and said CD79b antibody-drug conjugate are co-administered either simultaneously or sequentially (e.g. intravenous (i.v.) through a continuous infusion (one for the anti-CD20 antibody and eventually one for said CD79b antibody-drug conjugate; or e.g. the anti-CD20 antibody is administered intravenous (i.v.) through a continuous infusion and said CD79b antibody-drug conjugate is administered orally). When both therapeutic agents are co-administered sequentially the dose is administered either on the same day in two separate administrations, or one of the agents is administered on day 1 and the second is co-administered on day 2 to day 7, preferably on day 2 to 4. Thus in one embodiment the term "sequentially" means within 7 days after the dose of the first component (anti-CD20 antibody or CD79b antibody-drug conjugate), preferably within 4 days after the dose of the first component; and the term "simultaneously" means at the same time. The terms "co-administration" with respect to the maintenance doses of said afucosylated anti-CD20 antibody and said CD79b antibody-drug conjugate mean that the maintenance doses can be either co-administered simultaneously, if the treatment cycle is appropriate for both drugs, e.g. every week. Or CD79b antibody-drug conjugate is e.g. administered e.g. every first to third day and said afucosylated antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration of said anti-CD20 afucosylated antibody and said CD79b antibody-drug conjugate and the timing of co-administration will depend on the type (species, gender, age, weight, etc.) and condition of the patient being treated and the severity of the disease or condition being treated. Said afucosylated anti-CD20 antibody and said CD79b antibody-drug conjugate are suitably co-administered to the patient at one time or over a series of treatments e.g. on the same day or on the day after.

If the administration is intravenous the initial infusion time for said afucosylated anti-CD20 antibody or said CD79b antibody-drug conjugate may be longer than subsequent infusion times, for instance approximately 90 minutes for the initial infusion, and approximately 30 minutes for subsequent infusions (if the initial infusion is well tolerated).

Depending on the type and severity of the disease, about 0.1 mg /kg to 50 mg/kg (e.g. 0.1-20 mg/kg) of said afucosylated anti-CD20 antibody; and 1 µg /kg to 50 mg/kg (e.g. 0.1-20 mg/kg) of said CD79b antibody-drug conjugate is an initial candidate dosage for co-administration of both drugs to the patient. In one embodiment the preferred dosage of said afucosylated anti-CD20 antibody (preferably the afocusylated humanized B-Ly1 antibody) will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient. In one embodiment the preferred dosage of said CD79b antibody-drug conjugate will be in the range from about 0.05mg/kg to about 30mg/kg. Thus, one or more doses of about 0.5mg/kg, 2.0mg/kg, 4.0mg/kg, 10mg/kg or 30mg/kg (or any combination thereof) may be co-administered to the patient.

For treating these cancers, in one embodiment, said CD79b antibody-drug conjugate are administered via intravenous infusion, as mentioned above. The dosage administered via infusion is in the range of about 1 µg/m² to about 10,000 µg/m² per dose, generally one dose per week for a total of one, two, three or four doses. Alternatively, the dosage range is of about 1 µg/m² to about 1000 µg/m², about µg/m² to about 800 µg/m², about 1 µg/m² to about 600 µg/m² , about 1 µg/m² to about 400 µg/m², about 10 µg/m² to about 500 µg/m² , about 10 µg/m² to about 300 µg/m², about 10 µg/m² to about 200 µg/m² , and about 1 µg/m² to about 200 µg/m². The dose may be administered once per day, once per week, multiple times per week, but less than once per day, multiple times per month but less than once per day, multiple times per month but less than once per week, once per month or intermittently to relieve or alleviate symptoms of the disease. Administration may continue at any of the disclosed intervals until remission of the tumor or symptoms of the lymphoma, leukemia being treated. Administration may continue after remission or relief of symptoms is achieved where such remission or relief is prolonged by such continued administration.

Depending on the on the type (species, gender, age, weight, etc.) and condition of the patient and on the type of afucosylated anti-CD20 antibody , the dosage and the administration schedule of said afucosylated anti-CD20 antibody can differ from said CD79b antibody-drug conjugate. E.g. the said afucosylated anti-CD20 antibody may be administered e.g. every one to three weeks and said CD79b antibody-drug conjugate may be administered daily or every 2 to 10 days. An initial higher loading dose, followed by one or more lower doses may also be administered.

In one embodiment, the preferred dosage of said afucosylated anti-CD20 antibody (preferably the afocusylated humanized B-Ly1 antibody) in the combination with the CD79b antibody-drug conjugate according to the invention will be 800 to 1600 mg (in on embodiment 800 to 1200 mg) on day 1, 8, 15 of a 3-to 6-weeks-dosage-cycle and then in a dosage of 400 to 1200 (in one embodiment 800 to 1200 mg on day 1 of up to nine 3- to 4-weeks-dosage-cycles. Most preferably, the dose is a flat dose 1000 mg in a three-weeks-dosage schedule, with the possibility of an adddtional cycle of a flat dose of 1000 mg in the second week.

In yet another embodiment, the dose of the CD79b antibody-drug conjugate in the combination with the afucosylated anti-CD20 antibody according to the invention is about 1.5mg/kg to about 3 mg/kg in a three-weeks-dosage schedule, preferably about 1.7 mg/kg to about 2.5 mg/kg, most preferably about 1.8 mg/kg or about 2.4 mg/kg. Said most preferred dosages are currently tested in phase 2 trials for CD79b antibody-drug conjugate monotherapy.

In yet another embodiment, the dose of the afucosylated anti-CD20 antibody in the combination with the CD79b antibody-drug conjugate according to the invention is a flat dose of about 1000 mg on day 1 (cycle 1 day 1 (C1D1)), another flat dose of about 1000 mg day 8 (C1D8) and another flat dose of about 1000 mg day 15 (C1D15) followed by six more a flat doses of about 1000 mg of said afucosylated anti-CD20 antibody (Cycle 2) every three weeks: day 22 (C2D1), day 43 (C2D2), day 64 (C2D3), day 85 (C2D4), day 106 (C2D5), and day 127 (C2D6). In said embodiment, the dose of the CD79b antibody-drug conjugate in the combination with the afucosylated anti-CD20 antibody according to the invention is about 2.4 mg/kg every three weeks or alternatively 1.8 mg/kg every three weeks. In said embodiment, the dosing of the CD79b antibody-drug conjugate in the combination with the afucosylated anti-CD20 antibody according to the invention is day 1 (C1D1), day 22 (C2D1), day 43 (C2D2), day 64 (C2D3), day 85 (C2D4), day 106 (C2D5) and day 127 (C2D6).

Preferably, in said dosage regimens as described above, the afucosylated anti-CD20 antibody is obinutuzumab or GA101. Also preferably, in said dosage regimens as described above, said CD79b antibody-drug conjugate is anti-CD79b-MC-vc-PAB-MMAE.

The invention also provides a method of alleviating an autoimmune disease, comprising administering to a patient suffering from the autoimmune disease, a therapeutically effective amount of said afucosylated anti-CD20 antibody as disclosed herein and a humanized huMA79b.v28 antibody-drug conjugate of any one of the preceding embodiments. In preferreds the antibody is administered intravenously or subcutaneously. The antibody-drug conjugate is administered intravenously at a dosage in the range of about 1 µg/m² to about 100 mg/ m² per dose and in a specific embodiment, the dosage is 1 µg/m² to about 500 µg/m². The dose may be administered once per day, once per week, multiple times per week, but less than once per day, multiple times per month but less than once per day, multiple times per month but less than once per week, once per month or intermittently to relieve or alleviate symptoms of the disease. Administration may continue at any of the disclosed intervals until relief from or alleviation of symptoms of the autoimmune disease being treated. Administration may continue after relief from or alleviation of symptoms is achieved where such alleviation or relief is prolong by such continued administration.

The invention also provides a method of treating a B cell disorder comprising administering to a patient suffering from a B cell disorder, such as a B cell proliferative disorder (including without limitation lymphoma and leukemia) or an autoimmune disease, a therapeutically effective amount of said afucosylated anti-CD20 antibody as disclosed herein and a humanized huMA79b.v28 antibody of any one of the preceding embodiments, which antibody is not conjugated to a cytotoxic molecule or a detectable molecule. The antibody will typically be administered in a dosage range of about 1 µg/m² to about 1000 mg/m².

The recommended dose may vary whether there is a further co-administration of chemotherapeutic agent and based on the type of chemotherapeutic agent.

In an embodiment, the medicament is useful for preventing or reducing metastasis or further dissemination in such a patient suffering from cancer, preferably CD20 expressing cancer. The medicament is useful for increasing the duration of survival of such a patient, increasing the progression free survival of such a patient, increasing the duration of response, resulting in a statistically significant and clinically meaningful improvement of the treated patient as measured by the duration of survival, progression free survival, response rate or duration of response. In a preferred embodiment, the medicament is useful for increasing the response rate in a group of patients.

In the context of this invention, additional other cytotoxic, chemotherapeutic or anti-cancer agents, or compounds that enhance the effects of such agents (e.g. cytokines) may be used in the afucosylated anti-CD20 antibody and said CD79b antibody-drug conjugate combination treatment of cancer. Such molecules are suitably present in combination in amounts that are effective for the purpose intended. In one embodiment, the said afucosylated anti-CD20 antibody and said CD79b antibody-drug conjugate combination treatment is used without such additional cytotoxic, chemotherapeutic or anti-cancer agents, or compounds that enhance the effects of such agents.

Such agents include, for example: alkylating agents or agents with an alkylating action, such as cyclophosphamide (CTX; e.g. cytoxan®), chlorambucil (CHL; e.g. leukeran®), cisplatin (CisP; e.g. platinol®) busulfan (e.g. myleran®), melphalan, carmustine (BCNU), streptozotocin, triethylenemelamine (TEM), mitomycin C, and the like; anti-metabolites, such as methotrexate (MTX), etoposide (VP16; e.g. vepesid®), 6-mercaptopurine (6MP), 6-thiocguanine (6TG), cytarabine (Ara-C), 5-fluorouracil (5-FU), capecitabine (e.g. Xeloda®), dacarbazine (DTIC), and the like; antibiotics, such as actinomycin D, doxorubicin (DXR; e.g. adriamycin®), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol®) and paclitaxel derivatives, the cytostatic agents, glucocorticoids such as dexamethasone (DEX; e.g. decadron®) and corticosteroids such as prednisone, nucleoside enzyme inhibitors such as hydroxyurea, amino acid depleting enzymes such as asparaginase, leucovorin and other folic acid derivatives, and similar, diverse antitumor agents. The following agents may also be used as additional agents: arnifostine (e.g. ethyol®), dactinomycin, mechlorethamine (nitrogen mustard), streptozocin, cyclophosphamide, lomustine (CCNU), doxorubicin lipo (e.g. doxil®), gemcitabine (e.g. gemzar®), daunorubicin lipo (e.g. daunoxome®), procarbazine, mitomycin, docetaxel (e.g. taxotere®), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon beta, interferon alpha, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, chlorambucil. In one embodiment, the afucosylated anti-CD20 antibody and said CD79b antibody-drug conjugate combination treatment is used without such additional agents.

The use of the cytotoxic and anticancer agents described above as well as antiproliferative target-specific anticancer drugs like protein kinase inhibitors in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using histoculture methods. Generally, the dosage will be reduced compared to the amount used in the absence of additional other agents.

Typical dosages of an effective cytotoxic agent can be in the ranges recommended by the manufacturer, and where indicated by in vitro responses or responses in animal models, can be reduced by up to about one order of magnitude concentration or amount. Thus, the actual dosage will depend upon the judgment of the physician, the condition of the patient, and the effectiveness of the therapeutic method based on the in vitro responsiveness of the primary cultured malignant cells or histocultured tissue sample, or the responses observed in the appropriate animal models.

In the context of this invention, an effective amount of ionizing radiation may be carried out and/or a radiopharmaceutical may be used in addition to the afucosylated anti-CD20 antibody and said CD79b antibody-drug conjugate combination treatment of CD20 expressing cancer. The source of radiation can be either external or internal to the patient being treated. When the source is external to the patient, the therapy is known as external beam radiation therapy (EBRT). When the source of radiation is internal to the patient, the treatment is called brachytherapy (BT). Radioactive atoms for use in the context of this invention can be selected from the group including, but not limited to, radium, cesium-137, iridium-192, americium-241, gold-198, cobalt-57, copper-67, technetium-99, iodine-123, iodine-131, and indium-111. Is also possible to label the antibody with such radioactive isotopes. In one embodiment, the afucosylated anti-CD20 antibody and said CD79b antibody-drug conjugate combination treatment is used without such ionizing radiation.

Radiation therapy is a standard treatment for controlling unresectable or inoperable tumors and/or tumor metastases. Improved results have been seen when radiation therapy has been combined with chemotherapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the combination treatment of the invention and radiation. In other words, the inhibition of tumor growth by means of the agents comprising the combination of the invention is enhanced when combined with radiation, optionally with additional chemotherapeutic or anticancer agents. Parameters of adjuvant radiation therapies are, for example, contained in WO 99/60023.

The afucosylated anti-CD20 antibodies and/or the CD79b antibody-drug conjugate according to the invention are administered to a patient according to known methods, by intravenous administration as a bolus or by continuous infusion over a period of time, by intramuscular, intraperitoneal, intracerobrospinal, subcutaneous, intra-articular, intrasynovial, or intrathecal routes. In one embodiment, the administration of the antibody is intravenous or subcutaneous.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

### Pharmaceutical Compositions:

Pharmaceutical compositions can be obtained by processing the anti-CD20 antibody and/or the CD79b antibody-drug conjugate according to this invention with pharmaceutically acceptable, inorganic or organic carriers. Lactose, corn starch or derivatives thereof, talc, stearic acids or it's salts and the like can be used, for example, as such carriers for tablets, coated tablets, dragees and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

In one embodiment of the invention the composition comprises both said afucosylated anti-CD20 antibody with an amount of fucose is 60% or less (preferably said afucosylated humanized B-Ly1 antibody) and said CD79b antibody-drug conjugate for use in the treatment of cancer, in particular of CD20 expressing cancer (preferably a lymphoma or lymphocytic leukemiae.g., a B-Cell Non-Hodgkin's lymphoma (NHL).

Said pharmaceutical composition may further comprise one or more pharmaceutically acceptable carriers.

The present invention further provides a pharmaceutical composition, e.g. for use in cancer, comprising (i) an effective first amount of an afucosylated anti-CD20 antibody with an amount of fucose is 60% or less (preferably an afucosylated humanized B-Ly1 antibody), and (ii) an effective second amount of a CD79b antibody-drug conjugate. Such composition optionally comprises pharmaceutically acceptable carriers and / or excipients.

Pharmaceutical compositions of the afucosylated anti-CD20 antibody alone used in accordance with the present invention are prepared for storage by mixing an antibody having the desired degree of purity with optional pharmaceutically acceptable carriers, excipients or stabilizers (Remington's Pharmaceutical Sciences 16th edition, Osol, A. (ed.) (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

Pharmaceutical compositions of antibody CD79b antibody-drug conjugates can be similar to those describe above for the afucosylated anti-CD20 antibody.

Pharmaceutical compositions of small molecule CD79b antibody-drug conjugate include those suitable for oral, nasal, topical (including buccal and sublingual), rectal, vaginal and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of a formula I compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent. Methods of preparing these compositions include the step of bringing into association a CD79b antibody-drug conjugate with the carrier and, optionally, one or more accessory ingredients. In general, the pharmaceutical compositions of the CD79b antibody-drug conjugate are prepared by uniformly and intimately bringing into association a CD79b antibody-drug conjugate with liquid carriers, or finely divided solid carriers, or both, and then, if necessary, shaping the product. compositions suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In one further embodiment of the invention, the afucosylated anti-CD20 antibody and the CD79b antibody-drug conjugate are formulated into two separate pharmaceutical compositions.

The active ingredients may also be entrapped in microcapsules prepared, for example, by coacervation techniques or by interracial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano- particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (US 3,773,919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

One embodiment is a composition comprising a humanized B-Ly1 antibody which is afucosylated with an amount of fucose of 60% or less of the total amount of oligosaccharides (sugars) at Asn297, and CD79b antibody-drug conjugate as disclosed herein, for the treatment of cancer.

The present invention further provides a method for the treatment of cancer, comprising administering to a patient in need of such treatment (i) an effective first amount of an afucosylated anti-CD20 antibody with an amount of fucose is 60% or less, (preferably an afucosylated humanized B-Ly1 antibody); and (ii) an effective second amount of a CD79b antibody-drug conjugate.

In one embodiment, the amount of fucose of is between 40% and 60%.

Preferably said cancer is a CD20 expressing cancer.

Preferably said CD20 expressing cancer is a lymphoma or lymphocytic leukemia.

Preferably said afucosylated anti-CD20 antibody is a type II anti-CD20 antibody.

Preferably said antibody is a humanized B-Ly1 antibody. Preferably, said humanized B-Ly1 antibody is obinutuzumab.

Preferably said CD79b antibody-drug conjugate is anti-CD79b-MC-vc-PAB-MMAE. Preferably, the anti-CD79b antibody in this CD79b antibody-drug conjugate is huMA79b.v28.

Most preferably said anti-CD20 antibody is obinutuzumab in combination with said CD79b antibody-drug conjugate which is anti-CD79b-MC-vc-PAB-MMAE.

Preferably said afucosylated anti-CD20 antibody is a humanized B-Ly1 antibody and said CD79b antibody-drug conjugate is anti-CD79b-MC-vc-PAB-MMAE and said cancer is a CD20 expressing cancer, preferably a lymphoma or lymphocytic leukemia.

As used herein, the term "patient" preferably refers to a human in need of treatment with an afucosylated anti-CD20 antibody (e.g. a patient suffering from CD20 expressing cancer) for any purpose, and more preferably a human in need of such a treatment to treat cancer, or a precancerous condition or lesion. However, the term "patient" can also refer to non-human animals, preferably mammals such as dogs, cats, horses, cows, pigs, sheep and non-human primates, among others.

The invention further comprises an afucosylated anti-CD20 antibody with an amount of fucose is 60% or less, and a CD79b antibody-drug conjugate for use in the treatment of cancer.

Preferably said afucosylated anti-CD20 antibody is a humanized B-Ly1 antibody.

Preferably said CD79b antibody-drug conjugate is in one aspect of the method according to the invention, the CD79b antibody-drug conjugate is anti-CD79b-MC-vc-PAB-MMAE. Preferably, the anti-CD79b antibody in this CD79b antibody-drug conjugate is huMA79b.v28.

Preferably said afucosylated anti-CD20 antibody is a humanized B-Ly1 antibody and said CD79b antibody-drug conjugate is anti-CD79b-MC-vc-PAB-MMAE. Most preferable, the anti-CD79b antibody in this CD79b antibody-drug conjugate is huMA79b.v28.

Preferably, said cancer is a CD20 expressing cancer, preferably a lymphoma or lymphocytic leukemia.

The following examples and figures are provided to aid the understanding of the present invention, the true scope of which is set forth in the appended claims.

### Experimental Procedures

### Example 1 - CD79b antibody drug conjugate

### BJAB-Luciferase (Burkitt's Lymphoma) Xenografts

### In Vivo Tumor Cell Killing Assay

### A. Xenografts- DM1 conjugates

To test the efficacy of IgG variants of MA79b-grafted "humanized" antibody variants having changes in HVR-L2 and HVR-H3 (huMA79b L2/H3), the huMA79b L2/H3 variant was conjugated to DM1 and the effect of the conjugated variant on tumors in mice were analyzed.

Specifically, the ability of the antibodies to regress tumors in multiple xenograft models, including RAMOS cells, BJAB cells (Burkitt's lymphoma cell line that contain the t(2;8)(p112;q24) (IGK-MYC) translocation, a mutated p53 gene and are Epstein-Barr virus (EBV) negative) (Drexler, H.G., The Leukemia-Lymphoma Cell Line Facts Book, San Diego: Academic Press, 2001)), Granta 519 cells (mantle cell lymphoma cell line that contains the t(11;14)(q13;q32) (BCL1-IGH) translocation that results in the over-expression of cyclin D1 (BCL1), contains P16INK4B and P16INK4A deletions and are EBV positive) (Drexler, H.G., The Leukemia-Lymphoma Cell Line Facts Book, San Diego: Academic Press, 2001)), U698M cells (lymphoblastic lymphosarcoma B cell line; (Drexler, H.G., The Leukemia-Lymphoma Cell Line Facts Book, San Diego: Academic Press, 2001) and DoHH2 cells (follicular lymphoma cell line that contains the translocation characteristic of follicular lymphoma t(14;18)(q32;q21) that results in the over-expression of Bcl-2 driven by the Ig heavy chain, contains the P16INK4A deletion, contains the t(8;14)(q24;q32) (IGH-MYC) translocation and are EBV negative) (Drexler, H.G., The Leukemia-Lymphoma Cell Line Facts Book, San Diego: Academic Press, 2001)), may be examined.

For analysis of efficacy of MA79b-grafted "humanized" antibody variants, female CB17 ICR SCID mice (6-8 weeks of age from Charles Rivers Laboratories; Hollister, CA) were inoculated subcutaneously with 2 X 10⁷ BJAB-luciferase cells or Granta-519 cells via injection into the flanks of CB17 ICR SCID mice and the xenograft tumors were allowed to grow to an average of 200 mm². Day 0 refers to the day the tumors were an average of 200 mm² and when the first/or only dose of treatment was administered, unless indicated specifically below. Tumor volume was calculated based on two dimensions, measured using calipers, and was expressed in mm³ according to the formula: V= 0.5a X b², where a and b are the long and the short diameters of the tumor, respectively. Data collected from each experimental group were expressed as mean ± SE. Groups of 10 mice were treated with a single intravenous (i.v.) dose of between 50 µg and 210 µg of antibody-linked drug/m² mouse (corresponding to ∼1-4 mg/kg of mouse) with MA79b-grafted "humanized" antibody variants or control antibody-drug conjugates. Tumors were measured either once or twice a week throughout the experiment. Body weights of mice were measured either once or twice a week throughout the experiment. Mice were euthanized before tumor volumes reached 3000 mm³ or when tumors showed signs of impending ulceration. All animal protocols were approved by an Institutional Animal Care and Use Committee (IACUC).

Linkers between the antibody and the toxin that were used were thioether crosslinker SMCC for DM1. Additional linkers may include disulfide linker SPP or thioether crosslinker SMCC for DM1 or MC or MC-valine-citrulline(vc)-PAB or (a valine-citrulline (vc)) dipeptide linker reagent) having a maleimide component and a para-aminobenzylcarbamoyl (PAB) self-immolative component for monomethylauristatin E (MMAE) or monomethylauristan F (MMAF). Toxins used were DM1. Additional toxins may include MMAE or MMAF.

CD79b antibodies for this experiment included chimeric MA79b (chMA79b) antibodies as described in US2007/0207142, filed August 3, 2006 as well as MA79b-grafted "humanized" antibody variants described herein. Additional antibodies may include commercially available antibodies, including anti-CD79b antibody, and MA79b monoclonal antibodies generated from hybridomas deposited with the ATCC as HB11413 on July 20, 1993.

### B. Xenografts- further conjugates

Negative controls included anti-HER2 (HERCEPTIN® (trastuzumab)) based conjugates (SMCC-DM1).In a similar study, using the same xenograft study protocol as disclosed in Example A.) (above), varying the drug conjugates and doses administered, efficacy of additional drug conjugates were tested in BJAB-luciferase xenografts (Burkitt's Lymphoma) in CB17 SCID mice. The drug conjugates and doses (administered at day 0 for all ADCs and controls) are shown in Table 3, below.

The control antibody was huMA79b.v28 (conjugated to SMCC-DM1). The control HC (A118C) thioMAb was thio hu-anti-HER2-HC(A118C) antibody thioMAb (conjugated to BMPEO-DM1, MC-MMAF or MCvcPAB-MMAE), thio huMA79b.v28-HC(A118C) thioMAb or thio hu-anti-CD22(10F4v3)-HC(A118C) thioMAb (conjugated to MC-MMAF). The results are shown in Table 3, below.

Administration of the thio huMA79b.v28-HC(A118C)-BMPEO-DM1, thio-huMA79b.v28-HC(A118C)-MC-MMAF and thio huMA79b.v28-HC(A118C)-MCvcPAB-MMAE thioMAb drug conjugate showed an inhibition in tumor growth when compared to the negative control antibody drug conjugates (thio-hu-anti-HER2-HC(A118C)-BMPEO-DM1, thio-hu-anti-HER2-HC(A118C)-MC-MMAF and thio-hu-anti-HER2-HC(A118C)-MCvcPAB-MMAE). Other controls were thio-huMA79b.v28-HC(A118C), huMA79b.v28-SMCC-DM1 and thio-hu-anti-CD22(10F4v3)-HC(A118C)-MC-MMAF.

Further, in the same study, the percent body weight change in the first 7 days was determined in each dosage group. The results indicated administration of these thioMAb drug conjugates did not cause a significant decrease in percent body weight or weight loss during this time.

Even further, in Table 3, the number of mice out of the total number tested showing PR = Partial Regression (where the tumor volume at any time after administration dropped below 50% of the tumor volume measured at day 0) or CR = Complete Remission (where the tumor volume at any time after administration dropped to 0 mm³) are indicated and NA = not applicable. (DAR = Drug to Antibody Ratio)

**Table 3**

| In Vivo Tumor Volume Reduction, Thio HuMA79b.v28-HC(A118C) MMAE, MMAF, and DM1 Conjugate Administration | | | | | |
|---|---|---|---|---|---|
| In BJAB-Luciferase Xenografts in CB17 SCID Mice | | | | | |
| **Antibody administered** | **PR** | **CR** | **Dose MMAF, MMAE or DM1 (µg/m²)** | **Dose Ab (mg/kg)** | **DAR (Drug /Ab)** |
| Thio Control hu-anti-HER2-HC(A118C)-BMPEO-DM1 | 0/10 | 0/10 | 57 | 2 | 1.86 |
| Thio Control hu-anti-HER2-HC(A118C)-MC-MMAF | 1/10 | 0/10 | 58 | 2 | 1.9 |
| Thio Control hu-anti-HER2-HC(A118C)-MCvcPAB-MMAE | 0/10 | 0/10 | 46 | 2 | 1.55 |
| Control huMA79b.v28-SMCC-DM1 | 2/10 | 3/10 | 101 | 2 | 3.4 |
| Thio huMA79b.v28-HC(A118C)-BMPEO-DM1 | 3/10 | 2/10 | 55 | 2 | 1.85 |
| Thio huMA79b.v28-HC(A118C)-MC-MMAF | 0/10 | 10/10 | 57 | 2 | 1.95 |
| Thio huMA79b.v28-HC(A118C)-MCvcPAB-MMAE | 0/10 | 10/10 | 54 | 2 | 1.87 |
| Thio Control huMA79b.v28-HC(A118C) | 0/10 | 0/10 | NA | 2 | NA |
| Thio Control hu-anti-CD22(10F4v3)-HC(A118C)-MC-MMAF | 1/10 | 4/10 | 59 | 2 | 1.96 |

### Example 2 - Combination of GA101 with a CD79b antibody drug conjugate

The experimental part relates to GA101 (obinutuzumab as defined herein) in combination with the CD79b antibody drug conjugate anti-CD79b-MC-vc-PAB-MMAE, wherein the anti-CD79b antibody in this CD79b antibody-drug conjugate is huMA79b.v28. This CD79b antibody drug conjugate is termed herein "CD79b-ADC". Primary aim of the study was to investigate the effect of GA101 in combination with CD79b-ADC in the disseminated Z138 mantle cell lymphoma (MCL) xenograft model in SCID beige mice as compared to single agent therapy with GA101, single agent therapy with rituximab and the combination of rituximab with CD79b-ADC. The study design is depicted in Table 4.

**Table 4: Study design**

| **Group** | **Number of animals** | **Compound** | **Dose (µg)** | **Route of administration** | **No of treatments** |
|---|---|---|---|---|---|
| 1 | 10 | vehicle | - | i.v., once/week | 3 |
| 2 | 10 | GA101 in 20 mM Histidine, 140 mM NaCl, pH 6.0, 65% afucosylation | 600 µg (30 mg/kg) | i.v., once/week | 3 |
| 3 | 10 | Rituximab in 25 mM NaCitrate, 154 mM NaCl, 0.07 w/v % Tween80, pH 6.5 ± 0.3, 8% afucosylation | 600 µg (30 mg/kg) | i.v., once/week | 3 |
| 4 | 10 | CD79b-ADC in 20mM Histidine Acetate, 240mM Sucrose, 0.02% PS 20, pH 5.5 | 80 µg (4 mg/kg) | i.v., once | 1 |
| 5 | 10 | GA101 | 600 µg | i.v., once/week i.v., once | 3 |
| | | CD79b-ADC | 80 ug | | 1 |
| 6 | 10 | Rituximab | 600 µg | i.v., once/week i.v., once | 3 |
| | | CD79b-ADC | 80 ug | | 1 |

### Cell culture and cell application

Z138 human mantle cell lymphoma (MCL) cells were originally obtained from Martin Dyer and after expansion deposited in the Glycart internal cell bank. Tumor cell line was routinely cultured in DMEM containing 10 % FCS (Gibco) at 37 °C in a water-saturated atmosphere at 5 % CO2. Passage 26 was used for transplantation, at a viability of 96.4%. 10x106 cells were injected i.v. per animal into the tail vein in 200µl of Aim V cell culture medium (GIBCO) Expression of CD20 and CD79b was confirmed on Z138 MCL cells by FACS. For this purpose 0.2 Mio cells were stained in triplicates with anti-human CD20 PE (BD Bioscience #555623), anti-human CD79b-PE (BD Bioscience #555679) or the isotype controls mouse IgG1 (BD Bioscience #555749) or mouse IgG2b (BD Bioscience #555743). Mean fluorescence was measured using the plate protocol in the FACS CantoII (Software FACS Diva).

### Animals

62 SCID beige female mice; age 7-8 weeks at start of experiment (purchased from Taconic, Denmark) were maintained under specific-pathogen-free condition with daily cycles of 12 h light /12 h darkness according to committed guidelines (GV-Solas; Felasa; TierschG). Experimental study protocol was reviewed and approved by local veterinary office, license no. P2008016. After arrival animals were maintained for one week to get accustomed to new environment and for observation. Continuous health monitoring was carried out on regular basis.

### Treatment

Treatment started at day 21 after cell transplantation. Therapeutic antibodies and the corresponding vehicle were given i.v. on study day 21, 28 and 35 at the dose of 30 mg/kg as single agent. The CD79bADC was given once on study day 21 at the dose of 4mg/kg. The antibody dilutions were prepared freshly from stock before use. The study was terminated on day 309.

### Monitoring, termination criteria and autopsy

Animals were controlled daily for clinical symptoms and detection of adverse effects. Termination criteria for animals were visible sickness: scruffy fur, arched back, heavy breathing, impaired locomotion, HLP (hind leg paralysis). Mice were sacrificed according to the termination criteria. One scout was taken at the beginning of the experiment to check for tumor burden. Mice were sacrificed according to the termination criteria.

### Statistics

Survival data were statistically analyzed by pairwise Wilcoxon and pairwise log-rank test.

### Results

The human mantle cell lymphoma cell line Z138 was intravenously inoculated (10x106 cells) into the tail vein of the mice. Mice were randomized before 1st therapy and treatment started at day 21 after cell transplantation. The antibody drug conjugate was given once at day 21 at a dose of 4mg/kg whereas GA101, rituximab and the corresponding vehicle were given i.v. on study day 21, 28 and 35 at the dose of 30 mg/kg. Animals in the control group received PBS. All animals were controlled daily for clinical symptoms and detection of adverse effects and sacrificed according to the set termination criteria. Study termination was on study day 309. Survival data were represented with the survival curve (Figure 1) and were statistically analysed by Pairwise Wilcoxon and Pairwise Log-Rank test (Figure 2). Values marked with * in Figure 2 indicate a significant difference. Median and overall survival values for the different treatment groups are given in table 4 and table 5. All animals surviving until day 309 when the experiment was finished appeared tumor free during biopsy.

**Table 5: Median survival**

| Group | PBS | CD79b-ADC | GA101 + CD79b-ADC | Rituximab + CD79b-ADC | GA101 | Rituximab |
|---|---|---|---|---|---|---|
| Median Survival [days] | 30 | 56.5 | 81 | 65.5 | 38 | 34 |

**Table 6: Overall Survival at end of experiment (day 273):**

| Group | PBS | CD79b-ADC | GA101 + CD79b-ADC | Rituximab + CD79b-ADC | GA101 | Rituximab |
|---|---|---|---|---|---|---|
| Overall Survival | 0/10 | 0/10 | 2/10 | 3/10 | 0/10 | 0/10 |

All groups are significantly different from the vehicle group except rituximab in the Pairwise log rank test. Both combinations, GA101 plus anti-CD79b-ADC as well as rituximab plus anti-CD79b-ADC, show significantly increased survival compared to the respective monotherapies. The combination of GA101 + CD79b-ADC showed the best median survival followed by the combination of rituximab + CD79b-ADC and resulted in 2/10 or 3/10 tumor free animals respectively. Furthermore, the combinations of rituximab and GA101 with the CD79b-ADC show strong anti-tumoral activity compared to the respective monotherapies with RTX, GA101 or the anti-CD79b-ADC in terms of overall survival.

## Claims

1. Ibinutuzumab, for use in the treatment of cancer in combination with the CD79b antibody-drug conjugate anti-CD79b-MC-vc-PAB-MMAE, wherein the anti-CD79b antibody in said CD79b antibody-drug conjugate is huMA79b.v28 comprising the light chain variable domain SEQ ID NO: 69 and the heavy chain variable domain SEQ ID NO: 70.

2. Obinutuzumab, for use according to claim 1 in the treatment of cancer in combination with the CD79b antibody-drug conjugate anti-CD79b-MC-vc-PAB-MMAE, **characterized in that** one or more additional other cytotoxic, chemotherapeutic or anti-cancer agents, or compounds or ionizing radiation that enhance the effects of such agents are administered.

3. Obinutuzumab for use according to claim 1 or 2 in the treatment of cancer in combination with the CD79b antibody-drug conjugate anti-CD79b-MC-vc-PAB-MMAE, **characterized in that** said cancer is a CD20 expressing cancer.

4. Obinutuzumab for use according to claim 3 in the treatment of cancer in combination with the CD79b antibody-drug conjugate anti-CD79b-MC-vc-PAB-MMAE, **characterized in that** said CD20 expressing cancer is a lymphoma or lymphocytic leukemia.

5. A composition for use in the treatment of cancer comprising obinutuzumab and the CD79b antibody-drug conjugate anti-CD79b-MC-vc-PAB-MMAE, wherein the anti-CD79b antibody in said CD79b antibody-drug conjugate is huMA79b.v28 comprising the light chain variable domain SEQ ID NO: 69 and the heavy chain variable domain SEQ ID NO: 70.

6. The composition for use in the treatment of cancer according to claim 5, **characterized in that** one or more additional other cytotoxic, chemotherapeutic or anti-cancer agents, or compounds or ionizing radiation that enhance the effects of such agents are administered.

## Patentansprüche

1. Obinutuzumab zur Verwendung bei der Behandlung von Krebs in Kombination mit dem CD79b-Antikörper-Wirkstoff-Konjugat Anti-CD79b-MC-vc-PAB-MMAE, wobei der Anti-CD79b-Antikörper in dem CD79b-Antikörper-Wirkstoff-Konjugat huMA79b.v28 ist, umfassend die variable Leichtkettendomäne SEQ ID NO: 69 und die variable Schwerkettendomäne SEQ ID NO: 70.

2. Obinutuzumab zur Verwendung nach Anspruch 1 bei der Behandlung von Krebs in Kombination mit dem CD79b-Antikörper-Wirkstoff-Konjugat Anti-CD79b-MC-vc-PAB-MMAE, **dadurch gekennzeichnet, dass** ein oder mehrere zusätzliche andere zytotoxische, chemotherapeutische oder Antikrebsmittel oder Verbindungen oder ionisierende Strahlung, welche die Wirkungen solcher Mittel verstärken, verabreicht werden.

3. Obinutuzumab zur Verwendung nach Anspruch 1 oder 2 bei der Behandlung von Krebs in Kombination mit dem CD79b-Antikörper-Wirkstoff-Konjugat Anti-CD79b-MC-vc-PAB-MMAE, **dadurch gekennzeichnet, dass** der Krebs ein CD20-exprimierender Krebs ist.

4. Obinutuzumab zur Verwendung nach Anspruch 3 bei der Behandlung von Krebs in Kombination mit dem CD79b-Antikörper-Wirkstoff-Konjugat Anti-CD79b-MC-vc-PAB-MMAE, **dadurch gekennzeichnet, dass** der CD20-exprimierende Krebs ein Lymphom oder lymphatische Leukämie ist.

5. Zusammensetzung zur Verwendung bei der Behandlung von Krebs, umfassend Obinutuzumab und das CD79b-Antikörper-Wirkstoff-Konjugat Anti-CD79b-MC-vc-PAB-MMAE, wobei der Anti-CD79b-Antikörper in dem CD79b-Antikörper-Wirkstoff-Konjugat huMA79b.v28 ist, umfassend die variable Leichtkettendomäne SEQ NO: 69 und die variable Schwerkettendomäne SEQ ID NO: 70.

6. Zusammensetzung zur Verwendung bei der Behandlung von Krebs nach Anspruch 5, **dadurch gekennzeichnet, dass** ein oder mehrere zusätzliche andere zytotoxische, chemotherapeutische oder Antikrebsmittel oder Verbindungen oder ionisierende Strahlung, welche die Wirkungen solcher Mittel verstärken, verabreicht werden.

## Revendications

1. Obinutuzumab destiné à être utilisé dans le traitement d'un cancer en association avec le conjugué anticorps CD79b-médicament anti-CD79b-MC-vc-PAB-MMAE, l'anticorps anti-CD79b dans ledit conjugué anticorps CD79b-médicament étant huMA79b.v28 qui comprend le domaine variable de chaîne légère SEQ ID NO : 69 et le domaine variable de chaîne lourde SEQ ID NO : 70.

2. Obinutuzumab destiné à être utilisé selon la revendication 1 dans le traitement d'un cancer en association avec le conjugué anticorps CD79b-médicament anti-CD79b-MC-vc-PAB-MMAE, **caractérisé en ce que** sont administrés un ou plusieurs autres agents cytotoxiques, chimiothérapeutiques ou anticancéreux supplémentaires, ou des composés ou des rayonnements ionisants qui amplifient les effets de ces agents.

3. Obinutuzumab destiné à être utilisé selon la revendication 1 ou 2 dans le traitement d'un cancer en association avec le conjugué anticorps CD79b-médicament anti-CD79b-MC-vc-PAB-MMAE, **caractérisé en ce que** ledit cancer est un cancer exprimant CD20.

4. Obinutuzumab destiné à être utilisé selon la revendication 3 dans le traitement d'un cancer en association avec le conjugué anticorps CD79b-médicament anti-CD79b-MC-vc-PAB-MMAE, **caractérisé en ce que** ledit cancer exprimant CD20 est un lymphome ou la leucémie lymphoïde.

5. Composition destinée à être utilisée dans le traitement d'un cancer comprenant de l'obinutuzumab et le conjugué anticorps CD79b-médicament anti-CD79b-MC-vc-PAB-MMAE, l'anticorps anti-CD79b dans ledit conjugué anticorps CD79b-médicament étant huMA79b.v28 qui comprend le domaine variable de chaîne légère SEQ ID NO : 69 et le domaine variable de chaîne lourde SEQ ID NO : 70.

6. Composition destinée à être utilisée dans le traitement d'un cancer selon la revendication 5, **caractérisée en ce que** sont administrés un ou plusieurs autres agents cytotoxiques, chimiothérapeutiques ou anticancéreux supplémentaires, ou des composés ou des rayonnements ionisants qui amplifient les effets de ces agents.


### SEQUENCE LISTING

<110> F. Hoffmann-La Roche AG
<120> Combination therapy of an afucosylated CD20 antibody with a CD79b antibody-drug conjugate
<130> 31511 and PR5608
<140>
   <141>
<150> US 61/818821
   <151> 2013-05-02
<160> 182
<170> PatentIn version 3.2
<210> 1
   <211> 112
   <212> PRT
   <213> Mus sp.
<220>
   <221> MISC_FEATURE
   <223> amino acid sequence of variable region of the heavy chain (VH) of murine monoclonal anti-CD20 antibody B-Ly1
<400> 1
   Gly Pro Glu Leu Val Lys Pro Gly Ala Ser Val Lys Ile Ser Cys Lys
   1               5                   10                  15
   Ala Ser Gly Tyr Ala Phe Ser Tyr Ser Trp Met Asn Trp Val Lys Leu
               20                  25                  30
   Arg Pro Gly Gln Gly Leu Glu Trp Ile Gly Arg Ile Phe Pro Gly Asp
           35                  40                  45
   Gly Asp Thr Asp Tyr Asn Gly Lys Phe Lys Gly Lys Ala Thr Leu Thr
       50                  55                  60
   Ala Asp Lys Ser Ser Asn Thr Ala Tyr Met Gln Leu Thr Ser Leu Thr
   65                  70                  75                  80
   Ser Val Asp Ser Ala Val Tyr Leu Cys Ala Arg Asn Val Phe Asp Gly
                   85                  90                  95
   Tyr Trp Leu Val Tyr Trp Gly Gln Gly Thr Leu Val Thr Val Ser Ala
               100                 105                 110
<210> 2
   <211> 103
   <212> PRT
   <213> Mus sp.
<220>
   <221> MISC_FEATURE
   <223> amino acid sequence of variable region of the light chain (VL) of murine monoclonal anti-CD20 antibody B-Ly1
<400> 2
   Asn Pro Val Thr Leu Gly Thr Ser Ala Ser Ile Ser Cys Arg Ser Ser
   1               5                   10                  15
   Lys Ser Leu Leu His Ser Asn Gly Ile Thr Tyr Leu Tyr Trp Tyr Leu
               20                  25                  30
   Gln Lys Pro Gly Gln Ser Pro Gln Leu Leu Ile Tyr Gln Met Ser Asn
           35                  40                  45
   Leu Val Ser Gly Val Pro Asp Arg Phe Ser Ser Ser Gly Ser Gly Thr
       50                  55                  60
   Asp Phe Thr Leu Arg Ile Ser Arg Val Glu Ala Glu Asp Val Gly Val
   65                  70                  75                  80
   Tyr Tyr Cys Ala Gln Asn Leu Glu Leu Pro Tyr Thr Phe Gly Gly Gly
                   85                  90                  95
   Thr Lys Leu Glu Ile Lys Arg
               100
<210> 3
   <211> 119
   <212> PRT
   <213> Artificial
<220>
   <223> amino acid sequences of variable region of the heavy chain (VH) of humanized B-Ly1 antibody (B-HH2)
<400> 3
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 4
   <211> 119
   <212> PRT
   <213> Artificial
<220>
   <223> amino acid sequences of variable region of the heavy chain (VH) of humanized B-Ly1 antibody (B-HH3)
<400> 4
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Leu Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 5
   <211> 119
   <212> PRT
   <213> Artificial
<220>
   <223> amino acid sequences of variable region of the heavy chain (VH) of humanized B-Ly1 antibody (B-HH4)
<400> 5
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 6
   <211> 119
   <212> PRT
   <213> Artificial
<220>
   <223> amino acid sequences of variable region of the heavy chain (VH) of humanized B-Ly1 antibody (B-HH5)
<400> 6
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 7
   <211> 119
   <212> PRT
   <213> Artificial
<220>
   <223> amino acid sequences of variable region of the heavy chain (VH) of humanized B-Ly1 antibody (B-HH6)
<400> 7
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Ala Phe Ser Tyr Ser
   20 25 30
   Trp Ile Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 8
   <211> 119
   <212> PRT
   <213> Artificial
<220>
   <223> amino acid sequences of variable region of the heavy chain (VH) of humanized B-Ly1 antibody (B-HH7)
<400> 8
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Ile Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 9
   <211> 119
   <212> PRT
   <213> Artificial
<220>
   <223> amino acid sequences of variable region of the heavy chain (VH) of humanized B-Ly1 antibody (B-HH8)
<400> 9
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Tyr Ser
               20                  25                  30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 10
   <211> 119
   <212> PRT
   <213> Artificial
<220>
   <223> amino acid sequences of variable region of the heavy chain (VH) of humanized B-Ly1 antibody (B-HH9)
<400> 10
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Ser Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 11
   <211> 119
   <212> PRT
   <213> Artificial
<220>
   <223> amino acid sequences of variable region of the heavy chain (VH) of humanized B-Ly1 antibody (B-HL8)
<400> 11
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 12
   <211> 119
   <212> PRT
   <213> Artificial
<220>
   <223> amino acid sequences of variable region of the heavy chain (VH) of humanized B-Ly1 antibody (B-HL10)
<400> 12
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Ala Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 13
   <211> 119
   <212> PRT
   <213> Artificial
<220>
   <223> amino acid sequences of variable region of the heavy chain (VH) of humanized B-Ly1 antibody (B-HL11)
<400> 13
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 14
   <211> 119
   <212> PRT
   <213> Artificial
<220>
   <223> amino acid sequences of variable region of the heavy chain (VH) of humanized B-Ly1 antibody (B-HL12)
<400> 14
   Glu Val Gln Leu Val Glu Ser Gly Ala Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 15
   <211> 119
   <212> PRT
   <213> Artificial
<220>
   <223> amino acid sequences of variable region of the heavy chain (VH) of humanized B-Ly1 antibody (B-HL13)
<400> 15
   Glu Val Gln Leu Val Glu Ser Gly Gly Gly Val Val Lys Pro Gly Gly
   1 5 10 15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 16
   <211> 119
   <212> PRT
   <213> Artificial
<220>
   <223> amino acid sequences of variable region of the heavy chain (VH) of humanized B-Ly1 antibody (B-HL14)
<400> 16
   Glu Val Gln Leu Val Glu Ser Gly Gly Gly Leu Lys Lys Pro Gly Gly
   1 5 10 15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 17
   <211> 119
   <212> PRT
   <213> Artificial
<220>
   <223> amino acid sequences of variable region of the heavy chain (VH) of humanized B-Ly1 antibody (B-HL15)
<400> 17
   Glu Val Gln Leu Val Glu Ser Gly Gly Gly Leu Val Lys Pro Gly Ser
   1 5 10 15
   Ser Leu Arg Leu Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 18
   <211> 119
   <212> PRT
   <213> Artificial
<220>
   <223> amino acid sequences of variable region of the heavy chain (VH) of humanized B-Ly1 antibody (B-HL16)
<400> 18
   Glu Val Gln Leu Val Glu Ser Gly Gly Gly Leu Val Lys Pro Gly Gly
   1               5                   10                  15
   Ser Leu Arg Val Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 19
   <211> 119
   <212> PRT
   <213> Artificial
<220>
   <223> amino acid sequences of variable region of the heavy chain (VH) of humanized B-Ly1 antibody (B-HL17)
<400> 19
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 20
   <211> 115
   <212> PRT
   <213> Artificial
<220>
   <223> amino acid sequences of variable region of the light chain (VL) of humanized B-Ly1 antibody B-KV1
<400> 20
   Asp Ile Val Met Thr Gln Thr Pro Leu Ser Leu Pro Val Thr Pro Gly
   1               5                   10                  15
   Glu Pro Ala Ser Ile Ser Cys Arg Ser Ser Lys Ser Leu Leu His Ser
               20                  25                  30
   Asn Gly Ile Thr Tyr Leu Tyr Trp Tyr Leu Gln Lys Pro Gly Gln Ser
           35                  40                  45
   Pro Gln Leu Leu Ile Tyr Gln Met Ser Asn Leu Val Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Asn
                   85                  90                  95
   Leu Glu Leu Pro Tyr Thr Phe Gly Gly Gly Thr Lys Val Glu Ile Lys
               100                 105                 110
   Arg Thr Val
           115
<210> 21
   <211> 1270
   <212> DNA
   <213> Homo sapiens
<400> 21
   caggggacag gctgcagccg gtgcagttac acgttttcct ccaaggagcc 50
   tcggacgttg tcacgggttt ggggtcgggg acagagcagt gaccatggcc 100
   aggctggcgt tgtctcctgt gcccagccac tggatggtgg cgttgctgct 150
   gctgctctca gctgagccag taccagcagc cagatcggag gaccggtacc 200
   ggaatcccaa aggtagtgct tgttcgcgga tctggcagag cccacgtttc 250
   atagccagga aacggggctt cacggtgaaa atgcactgct acatgaacag 300
   cgcctccggc aatgtgagct ggctctggaa gcaggagatg gacgagaatc 350
   cccagcagct gaagctggaa aagggccgca tggaagagtc ccagaacgaa 400
   tctctcgcca ccctcaccat ccaaggcatc cggtttgagg acaatggcat 450
   ctacttctgt cagcagaagt gcaacaacac ctcggaggtc taccagggct 500
   gcggcacaga gctgcgagtc atgggattca gcaccttggc acagctgaag 550
   cagaggaaca cgctgaagga tggtatcatc atgatccaga cgctgctgat 600
   catcctcttc atcatcgtgc ctatcttcct gctgctggac aaggatgaca 650
   gcaaggctgg catggaggaa gatcacacct acgagggcct ggacattgac 700
   cagacagcca cctatgagga catagtgacg ctgcggacag gggaagtgaa 750
   gtggtctgta ggtgagcacc caggccagga gtgagagcca ggtcgcccca 800
   tgacctgggt gcaggctccc tggcctcagt gactgcttcg gagctgcctg 850
   gctcatggcc caaccccttt cctggacccc ccagctggcc tctgaagctg 900
   gcccaccaga gctgccattt gtctccagcc cctggtcccc agctcttgcc 950
   aaagggcctg gagtagaagg acaacagggc agcaacttgg agggagttct 1000
   ctggggatgg acgggaccca gccttctggg ggtgctatga ggtgatccgt 1050
   ccccacacat gggatggggg aggcagagac tggtccagag cccgcaaatg 1100
   gactcggagc cgagggcctc ccagcagagc ttgggaaggg ccatggaccc 1150
   aactgggccc cagaagagcc acaggaacat cattcctctc ccgcaaccac 1200
   tcccacccca gggaggccct ggcctccagt gccttccccc gtggaataaa 1250
   cggtgtgtcc tgagaaacca 1270
<210> 22
   <211> 229
   <212> PRT
   <213> Homo sapiens
<400> 22
   Met Ala Arg Leu Ala Leu Ser Pro Val Pro Ser His Trp Met Val
    1              5                   10                  15
   Ala Leu Leu Leu Leu Leu Ser Ala Glu Pro Val Pro Ala Ala Arg
                   20                  25                  30
   Ser Glu Asp Arg Tyr Arg Asn Pro Lys Gly Ser Ala Cys Ser Arg
                   35                  40                  45
   Ile Trp Gln Ser Pro Arg Phe Ile Ala Arg Lys Arg Gly Phe Thr
                   50                  55                  60
   Val Lys Met His Cys Tyr Met Asn Ser Ala Ser Gly Asn Val Ser
                   65                  70                  75
   Trp Leu Trp Lys Gln Glu Met Asp Glu Asn Pro Gln Gln Leu Lys
                   80                  85                  90
   Leu Glu Lys Gly Arg Met Glu Glu Ser Gln Asn Glu Ser Leu Ala
                   95                  100                 105
   Thr Leu Thr Ile Gln Gly Ile Arg Phe Glu Asp Asn Gly Ile Tyr
                   110                 115                 120
   Phe Cys Gln Gln Lys Cys Asn Asn Thr Ser Glu Val Tyr Gln Gly
                   125                 130                 135
   Cys Gly Thr Glu Leu Arg Val Met Gly Phe Ser Thr Leu Ala Gln
                   140                 145                 150
   Leu Lys Gln Arg Asn Thr Leu Lys Asp Gly Ile Ile Met Ile Gln
                   155                 160                 165
   Thr Leu Leu Ile Ile Leu Phe Ile Ile Val Pro Ile Phe Leu Leu
                   170                 175                 180
   Leu Asp Lys Asp Asp Ser Lys Ala Gly Met Glu Glu Asp His Thr
                   185                 190                 195
   Tyr Glu Gly Leu Asp Ile Asp Gln Thr Ala Thr Tyr Glu Asp Ile
                   200                 205                 210
   Val Thr Leu Arg Thr Gly Glu Val Lys Trp Ser Val Gly Glu His
                   215                 220                 225
   Pro Gly Gln Glu
<210> 23
   <211> 218
   <212> PRT
   <213> Artificial sequence
<220>
   <223> Light Chain of Chimeric Antibody (chMA79b)
<400> 23
   Asp Ile Val Leu Thr Gln Ser Pro Ala Ser Leu Ala Val Ser Leu
   1               5                   10                  15
   Gly Gln Arg Ala Thr Ile Ser Cys Lys Ala Ser Gln Ser Val Asp
                   20                  25                  30
   Tyr Asp Gly Asp Ser Phe Leu Asn Trp Tyr Gln Gln Lys Pro Gly
                   35                  40                  45
   Gln Pro Pro Lys Leu Phe Ile Tyr Ala Ala Ser Asn Leu Glu Ser
                   50                  55                  60
   Gly Ile Pro Ala Arg Phe Ser Gly Ser Gly Ser Gly Thr Asp Phe
                   65                  70                  75
   Thr Leu Asn Ile His Pro Val Glu Glu Glu Asp Ala Ala Thr Tyr
                   80                  85                  90
   Tyr Cys Gln Gln Ser Asn Glu Asp Pro Leu Thr Phe Gly Ala Gly
                   95                  100                 105
   Thr Glu Leu Glu Leu Lys Arg Thr Val Ala Ala Pro Ser Val Phe
                   110                 115                 120
   Ile Phe Pro Pro Ser Asp Glu Gln Leu Lys Ser Gly Thr Ala Ser
                   125                 130                 135
   Val Val Cys Leu Leu Asn Asn Phe Tyr Pro Arg Glu Ala Lys Val
                   140                 145                 150
   Gln Trp Lys Val Asp Asn Ala Leu Gln Ser Gly Asn Ser Gln Glu
                   155                 160                 165
   Ser Val Thr Glu Gln Asp Ser Lys Asp Ser Thr Tyr Ser Leu Ser
                   170                 175                 180
   Ser Thr Leu Thr Leu Ser Lys Ala Asp Tyr Glu Lys His Lys Val
                   185                 190                 195
   Tyr Ala Cys Glu Val Thr His Gln Gly Leu Ser Ser Pro Val Thr
                   200                 205                 210
   Lys Ser Phe Asn Arg Gly Glu Cys
                   215
<210> 24
   <211> 446
   <212> PRT
   <213> Artificial sequence
<220>
   <223> Heavy Chain of Chimeric Antibody (chMA79b)
<400> 24
   Glu Val Gln Leu Gln Gln Ser Gly Ala Glu Leu Met Lys Pro Gly
   1               5                   10                  15
   Ala Ser Val Lys Ile Ser Cys Lys Ala Thr Gly Tyr Thr Phe Ser
                   20                  25                  30
   Ser Tyr Trp Ile Glu Trp Val Lys Gln Arg Pro Gly His Gly Leu
                   35                  40                  45
   Glu Trp Ile Gly Glu Ile Leu Pro Gly Gly Gly Asp Thr Asn Tyr
                   50                  55                  60
   Asn Glu Ile Phe Lys Gly Lys Ala Thr Phe Thr Ala Asp Thr Ser
                   65                  70                  75
   Ser Asn Thr Ala Tyr Met Gln Leu Ser Ser Leu Thr Ser Glu Asp
                   80                  85                  90
   Ser Ala Val Tyr Tyr Cys Thr Arg Arg Val Pro Val Tyr Phe Asp
                   95                  100                 105
   Tyr Trp Gly Gln Gly Thr Ser Val Thr Val Ser Ser Ala Ser Thr
                   110                 115                 120
   Lys Gly Pro Ser Val Phe Pro Leu Ala Pro Ser Ser Lys Ser Thr
                   125                 130                 135
   Ser Gly Gly Thr Ala Ala Leu Gly Cys Leu Val Lys Asp Tyr Phe
                   140                 145                 150
   Pro Glu Pro Val Thr Val Ser Trp Asn Ser Gly Ala Leu Thr Ser
                   155                 160                 165
   Gly Val His Thr Phe Pro Ala Val Leu Gln Ser Ser Gly Leu Tyr
                   170                 175                 180
   Ser Leu Ser Ser Val Val Thr Val Pro Ser Ser Ser Leu Gly Thr
                   185                 190                 195
   Gln Thr Tyr Ile Cys Asn Val Asn His Lys Pro Ser Asn Thr Lys
                   200                 205                 210
   Val Asp Lys Lys Val Glu Pro Lys Ser Cys Asp Lys Thr His Thr
                   215                 220                 225
   Cys Pro Pro Cys Pro Ala Pro Glu Leu Leu Gly Gly Pro Ser Val
                   230                 235                 240
   Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp
                   260                 265                 270
   Pro Glu Val Lys Phe Asn Trp Tyr Val Asp Gly Val Glu Val His
                   275                 280                 285
   Asn Ala Lys Thr Lys Pro Arg Glu Glu Gln Tyr Asn Ser Thr Tyr
                   290                 295                 300
   Arg Val Val Ser Val Leu Thr Val Leu His Gln Asp Trp Leu Asn
                   305                 310                 315
   Gly Lys Glu Tyr Lys Cys Lys Val Ser Asn Lys Ala Leu Pro Ala
                   320                 325                 330
   Pro Ile Glu Lys Thr Ile Ser Lys Ala Lys Gly Gln Pro Arg Glu
                   335                 340                 345
   Pro Gln Val Tyr Thr Leu Pro Pro Ser Arg Glu Glu Met Thr Lys
                   350                 355                 360
   Asn Gln Val Ser Leu Thr Cys Leu Val Lys Gly Phe Tyr Pro Ser
                   365                 370                 375
   Asp Ile Ala Val Glu Trp Glu Ser Asn Gly Gln Pro Glu Asn Asn
                   380                 385                 390
   Tyr Lys Thr Thr Pro Pro Val Leu Asp Ser Asp Gly Ser Phe Phe
                   395                 400                 405
   Leu Tyr Ser Lys Leu Thr Val Asp Lys Ser Arg Trp Gln Gln Gly
                   410                 415                 420
   Asn Val Phe Ser Cys Ser Val Met His Glu Ala Leu His Asn His
                   425                 430                 435
   Tyr Thr Gln Lys Ser Leu Ser Leu Ser Pro Gly
                   440                 445
<210> 25
   <211> 108
   <212> PRT
   <213> Homo sapiens
<400> 25
   Asp Ile Gln Met Thr Gln Ser Pro Ser Ser Leu Ser Ala Ser Val
    1               5                  10                  15
   Gly Asp Arg Val Thr Ile Thr Cys Arg Ala Ser Gln Ser Ile Ser
                   20                  25                  30
   Asn Tyr Leu Ala Trp Tyr Gln Gln Lys Pro Gly Lys Ala Pro Lys
                   35                  40                  45
   Leu Leu Ile Tyr Ala Ala Ser Ser Leu Glu Ser Gly Val Pro Ser
                   50                  55                  60
   Arg Phe Ser Gly Ser Gly Ser Gly Thr Asp Phe Thr Leu Thr Ile
                   65                  70                  75
   Ser Ser Leu Gln Pro Glu Asp Phe Ala Thr Tyr Tyr Cys Gln Gln
                   80                  85                  90
   Tyr Asn Ser Leu Pro Trp Thr Phe Gly Gln Gly Thr Lys Val Glu
                   95                  100                 105
   Ile Lys Arg
<210> 26
   <211> 111
   <212> PRT
   <213> Artificial sequence
<220>
   <223> Variable domain of Light Chain of Chimeric Antibody (chMA79b)
<400> 26
   Asp Ile Val Leu Thr Gln Ser Pro Ala Ser Leu Ala Val Ser Leu
   1               5                   10                  15
   Gly Gln Arg Ala Thr Ile Ser Cys Lys Ala Ser Gln Ser Val Asp
               20                  25                  30
   Tyr Asp Gly Asp Ser Phe Leu Asn Trp Tyr Gln Lys Pro Gly Gln
           35                  40                  45
   Pro Pro Lys Leu Phe Ile Tyr Ala Ala Ser Asn Leu Glu Ser Gly
       50                  55                  60
   Ile Pro Ala Arg Phe Ser Gly Ser Gly Ser Gly Thr Asp Phe Thr
   65                  70                  75
   Leu Asn Ile His Pro Val Glu Glu Glu Asp Ala Ala Thr Tyr Tyr
                   80                  85                  90
   Cys Gln Gln Ser Asn Glu Asp Pro Leu Thr Phe Gly Ala Gly Thr
               95                  100                 105
   Glu Leu Glu Leu Lys Arg
           110
<210> 27
   <211> 112
   <212> PRT
   <213> Artificial sequence
<220>
   <223> Variable Domain of Light Chain of Humanized Antibody Graft (huMA79b
   graft)
<400> 27
   Asp Ile Gln Met Thr Gln Ser Pro Ser Ser Leu Ser Ala Ser Val
   1               5                   10                  15
   Gly Asp Arg Val Thr Ile Thr Cys Lys Ala Ser Gln Ser Val Asp
               20                  25                  30
   Tyr Asp Gly Asp Ser Phe Leu Asn Trp Tyr Gln Gln Lys Pro Gly
           35                  40                  45
   Lys Ala Pro Lys Leu Leu Ile Tyr Ala Ala Ser Asn Leu Glu Ser
       50                  55                  60
   Gly Val Pro Ser Arg Phe Ser Gly Ser Gly Ser Gly Thr Asp Phe
   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
               95                  100                 105
   Thr Lys Val Glu Ile Lys Arg
           110
<210> 28
   <211> 113
   <212> PRT
   <213> Homo sapiens
<400> 28
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Phe Thr Phe Ser
                   20                  25                  30
   Ser Tyr Ala Met Ser Trp Val Arg Gln Ala Pro Gly Lys Gly Leu
                   35                  40                  45
   Glu Trp Val Ser Val Ile Ser Gly Asp Gly Gly Ser Thr Tyr Tyr
                   50                  55                  60
   Ala Asp Ser Val Lys Gly Arg Phe Thr Ile Ser Arg Asp Asn Ser
                   65                  70                  75
   Lys Asn Thr Leu Tyr Leu Gln Met Asn Ser Leu Arg Ala Glu Asp
                   80                  85                  90
   Thr Ala Val Tyr Tyr Cys Ala Arg Gly Phe Asp Tyr Trp Gly Gln
                   95                  100                 105
   Gly Thr Leu Val Thr Val Ser Ser
                   110
<210> 29
   <211> 117
   <212> PRT
   <213> Artificial Sequence
<220>
   <223> Variable domain of Heavy Chain of Chimeric Antibody (chMA79b)
<400> 29
   Glu Val Gln Leu Gln Gln Ser Gly Ala Glu Leu Met Lys Pro Gly
   1 5 10 15
   Ala Ser Val Lys Ile Ser Cys Lys Ala Thr Gly Tyr Thr Phe Ser
   20 25 30
   Ser Tyr Trp Ile Glu Trp Val Lys Gln Arg Pro Gly His Gly Leu
   35 40 45
   Glu Trp Ile Gly Glu Ile Leu Pro Gly Gly Gly Asp Thr Asn Tyr
   50 55 60
   Asn Glu Ile Phe Lys Gly Lys Ala Thr Phe Thr Ala Asp Thr Ser
   65 70 75
   Ser Asn Thr Ala Tyr Met Gln Leu Ser Ser Leu Thr Ser Glu Asp
   80 85 90
   Ser Ala Val Tyr Tyr Cys Thr Arg Arg Val Pro Val Tyr Phe Asp
   95 100 105
   Tyr Trp Gly Gln Gly Thr Ser Val Thr Val Ser Ser
   110 115
<210> 30
   <211> 117
   <212> PRT
   <213> Artificial sequence
<220>
   <223> Variable Domain of Heavy Chain of Humanized Antibody Graft (huMA79b
   graft)
<400> 30
   Glu Val Gln Leu Val Glu Ser Gly Gly Gly Leu Val Gln Pro Gly
   1 5 10 15
   Gly Ser Leu Arg Leu Ser Cys Ala Ala Ser Gly Tyr Thr Phe Ser
   20 25 30
   Ser Tyr Trp Ile Glu Trp Val Arg Gln Ala Pro Gly Lys Gly Leu
   35 40 45
   Glu Trp Val Gly Glu Ile Leu Pro Gly Gly Gly Asp Thr Asn Tyr
   50 55 60
   Asn Glu Ile Phe Lys Gly Arg Phe Thr Ile Ser Ala Asp Thr Ser
   65 70 75
   Lys Asn Thr Ala Tyr Leu Gln Met Asn Ser Leu Arg Ala Glu Asp
   80 85 90
   Thr Ala Val Tyr Tyr Cys Thr Arg Arg Val Pro Val Tyr Phe Asp
   95 100 105
   Tyr Trp Gly Gln Gly Thr Leu Val Thr Val Ser Ser
   110 115
<210> 31
   <211> 15
   <212> PRT
   <213> Artificial Sequence
<220>
   <223> HVR1-LC of huMA79b graft
<400> 31
   Lys Ala Ser Gln Ser Val Asp Tyr Asp Gly Asp Ser Phe Leu Asn
   1 5 10 15
<210> 32
   <211> 7
   <212> PRT
   <213> Artificial Sequence
<220>
   <223> HVR2-LC of huMA79b graft
<400> 32
   Ala Ala Ser Asn Leu Glu Ser
   5
<210> 33
   <211> 9
   <212> PRT
   <213> Artificial Sequence
<220>
   <223> HVR3-LC of huMA79b graft
<400> 33
   Gln Gln Ser Asn Glu Asp Pro Leu Thr
   5
<210> 34
   <211> 10
   <212> PRT
   <213> Artificial Sequence
<220>
   <223> HVR1-HC of huMA79b graft
<400> 34
   Gly Tyr Thr Phe Ser Ser Tyr Trp Ile Glu
   5 10
<210> 35
   <211> 18
   <212> PRT
   <213> Artificial Sequence
<220>
   <223> HVR2-HC of huMA79b graft
<400> 35
   Gly Glu Ile Leu Pro Gly Gly Gly Asp Thr Asn Tyr Asn Glu Ile
   1               5                   10                  15
   Phe Lys Gly
<210> 36
   <211> 10
   <212> PRT
   <213> Artificial Sequence
<220>
   <223> HVR3-HC of huMA79b graft
<400> 36
   Thr Arg Arg Val Pro Val Tyr Phe Asp Tyr
                   5                   10
<210> 37
   <211> 15
   <212> PRT
   <213> Artificial sequence
<220>
   <223> HVR1-LC of Humanized Antibody Variant (huMA79b. V28)
<400> 37
   Lys Ala Ser Gln Ser Val Asp Tyr Glu Gly Asp Ser Phe Leu Asn
   1               5                   10                  15
<210> 38
   <211> 10
   <212> PRT
   <213> Artificial sequence
<220>
   <223> HVR3-HC of Humanized Antibody Variant (huMA79b. V28)
<400> 38
   Thr Arg Arg Val Pro Ile Arg Leu Asp Tyr
                   5                   10
<210> 39
   <211> 23
   <212> PRT
   <213> Homo sapiens
<400> 39
   Asp Ile Gln Met Thr Gln Ser Pro Ser Ser Leu Ser Ala Ser Val
   1               5                   10                  15
   Gly Asp Arg Val Thr Ile Thr Cys
                   20
<210> 40
   <211> 15
   <212> PRT
   <213> Homo sapiens
<400> 40
   Trp Tyr Gln Gln Lys Pro Gly Lys Ala Pro Lys Leu Leu Ile Tyr
   1               5                   10                  15
<210> 41
   <211> 32
   <212> PRT
   <213> Homo sapiens
<400> 41
   Gly Val Pro Ser Arg Phe Ser Gly Ser Gly Ser Gly Thr Asp Phe
   1               5                   10                  15
   Thr Leu Thr Ile Ser Ser Leu Gln Pro Glu Asp Phe Ala Thr Tyr
                   20                  25                  30
   Tyr Cys
<210> 42
   <211> 11
   <212> PRT
   <213> Homo sapiens
<400> 42
   Phe Gly Gln Gly Thr Lys Val Glu Ile Lys Arg
                       5                   10
<210> 43
   <211> 25
   <212> PRT
   <213> Homo sapiens
<400> 43
<210> 44
   <211> 13
   <212> PRT
   <213> Homo sapiens
<400> 44
   Trp Val Arg Gln Ala Pro Gly Lys Gly Leu Glu Trp Val
   5 10
<210> 45
   <211> 30
   <212> PRT
   <213> Homo sapiens
<400> 45
   Arg Phe Thr Ile Ser Arg Asp Asn Ser Lys Asn Thr Leu Tyr Leu
   1 5 10 15
   Gln Met Asn Ser Leu Arg Ala Glu Asp Thr Ala Val Tyr Tyr Cys
   20 25 30
<210> 46
   <211> 11
   <212> PRT
   <213> Homo sapiens
<400> 46
   Trp Gly Gln Gly Thr Leu Val Thr Val Ser Ser
   5 10
<210> 47
   <211> 15
   <212> PRT
   <213> Artificial sequence
<220>
   <223> HVR1-LC of Humanized Antibody Variant (huMA79b.v17)
<400> 47
   Lys Ala Ser Gln Ser Val Asp Tyr Asp Gly Asp Ser Phe Leu Asn
   1 5 10 15
<210> 48
   <211> 7
   <212> PRT
   <213> Artificial sequence
<220>
   <223> HVR2-LC of Humanized Antibody Variant (huMA79b.v17)
<400> 48
   Ala Ala Ser Asn Leu Glu Ser
   5
<210> 49
   <211> 9
   <212> PRT
   <213> Artificial sequence
<220>
   <223> HVR3-LC of Humanized Antibody Variant (huMA79b.v17)
<400> 49
   Gln Gln Ser Asn Glu Asp Pro Leu Thr
                   1               5
<210> 50
   <211> 10
   <212> PRT
   <213> Artificial sequence
<220>
   <223> HVR1-HC of Humanized Antibody Variant (huMA79b.v17)
<400> 50
   Gly Tyr Thr Phe Ser Ser Tyr Trp Ile Glu
                   5                   10
<210> 51
   <211> 18
   <212> PRT
   <213> Artificial sequence
<220>
   <223> HVR2-HC of Humanized Antibody Variant (huMA79b.v17)
<400> 51
   Gly Glu Ile Leu Pro Gly Gly Gly Asp Thr Asn Tyr Asn Glu Ile
     1               5                  10                  15
   Phe Lys Gly
<210> 52
   <211> 10
   <212> PRT
   <213> Artificial sequence
<220>
   <223> HVR-H3 of Humanized Antibody Variant (huMA79b.v17)
<400> 52
   Thr Arg Arg Val Pro Val Tyr Phe Asp Tyr
                   5                   10
<210> 53
   <211> 112
   <212> PRT
   <213> Artificial sequence
<220>
   <223> LC-HC Variety Domain of Humanized Antibody Varient(huMA79b.v17)
<400> 53
   Asp Ile Gln Leu Thr Gln Ser Pro Ser Ser Leu Ser Ala Ser Val
    1               5                  10                  15
   Gly Asp Arg Val Thr Ile Thr Cys Lys Ala Ser Gln Ser Val Asp
                   20                  25                  30
   Tyr Asp Gly Asp Ser Phe Leu Asn Trp Tyr Gln Gln Lys Pro Gly
                   35                  40                  45
   Lys Ala Pro Lys Leu Leu Ile Tyr Ala Ala Ser Asn Leu Glu Ser
                   50                  55                  60
   Gly Val Pro Ser Arg Phe Ser Gly Ser Gly Ser Gly Thr Asp Phe
                   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
                   95                  100                 105
   Thr Lys Val Glu Ile Lys Arg
                  110
<210> 54
   <211> 117
   <212> PRT
   <213> Artificial sequence
<220>
   <223> HC-Variable Domain of Humanized Antibody Variant (huMA79b.v17)
<400> 54
   Glu Val Gln Leu Val Glu Ser Gly Gly Gly Leu Val Gln Pro Gly
   1 5 10 15
   Gly Ser Leu Arg Leu Ser Cys Ala Ala Ser Gly Tyr Thr Phe Ser
   20 25 30
   Ser Tyr Trp Ile Glu Trp Val Arg Gln Ala Pro Gly Lys Gly Leu
   35 40 45
   Glu Trp Ile Gly Glu Ile Leu Pro Gly Gly Gly Asp Thr Asn Tyr
   50 55 60
   Asn Glu Ile Phe Lys Gly Arg Ala Thr Phe Ser Ala Asp Thr Ser
   65 70 75
   Lys Asn Thr Ala Tyr Leu Gln Met Asn Ser Leu Arg Ala Glu Asp
   80 85 90
   Thr Ala Val Tyr Tyr Cys Thr Arg Arg Val Pro Val Tyr Phe Asp
   95 100 105
   Tyr Trp Gly Gln Gly Thr Leu Val Thr Val Ser Ser
   110 115
<210> 55
   <211> 15
   <212> PRT
   <213> Artificial sequence
<220>
   <223> HVR1-LC of Humanized Antibody Variant (huMA79b.v18)
<400> 55
   Lys Ala Ser Gln Ser Val Asp Tyr Asp Gly Asp Ser Phe Leu Asn
   1 5 10 15
<210> 56
   <211> 7
   <212> PRT
   <213> Artificial sequence
<220>
   <223> HVR2-LC of Humanized Antibody Variant (huMA79b.v18)
<400> 56
   Ala Ala Ser Asn Leu Glu Ser
   5
<210> 57
   <211> 9
   <212> PRT
   <213> Artificial sequence
<220>
   <223> HVR3-LC of Humanized Antibody Variant (huMA79b.v18)
<400> 57
   Gln Gln Ser Asn Glu Asp Pro Leu Thr
   5
<210> 58
   <211> 10
   <212> PRT
   <213> Artificial sequence
<220>
   <223> HVR1-HC of Humanized Antibody Variant (huMA79b.v18)
<400> 58
   Gly Tyr Thr Phe Ser Ser Tyr Trp Ile Glu
   5 10
<210> 59
   <211> 18
   <212> PRT
   <213> Artificial sequence
<220>
   <223> HVR2-HC of Humanized Antibody Variant (huMA79b.v18)
<400> 59
   Gly Glu Ile Leu Pro Gly Gly Gly Asp Thr Asn Tyr Asn Glu Ile
   1 5 10 15
   Phe Lys Gly
<210> 60
   <211> 10
   <212> PRT
   <213> Artificial sequence
<220>
   <223> HVR3-HC of Humanized Antibody Variant (huMA79b.v18)
<400> 60
   Thr Arg Arg Val Pro Ile Arg Leu Asp Tyr
   5 10
<210> 61
   <211> 112
   <212> PRT
   <213> Artificial sequence
<220>
   <223> LC-Variable Domain of Humanized Antibody Variant (huMA79b.v18)
<400> 61
   Asp Ile Gln Leu Thr Gln Ser Pro Ser Ser Leu Ser Ala Ser Val
   1               5                   10                  15
   Gly Asp Arg Val Thr Ile Thr Cys Lys Ala Ser Gln Ser Val Asp
                   20                  25                  30
   Tyr Asp Gly Asp Ser Phe Leu Asn Trp Tyr Gln Gln Lys Pro Gly
                   35                  40                  45
   Lys Ala Pro Lys Leu Leu Ile Tyr Ala Ala Ser Asn Leu Glu Ser
                   50                  55                  60
   Gly Val Pro Ser Arg Phe Ser Gly Ser Gly Ser Gly Thr Asp Phe
                   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
                   95                  100                 105
   Thr Lys Val Glu Ile Lys Arg
                   110
<210> 62
   <211> 117
   <212> PRT
   <213> Artificial sequence
<220>
   <223> HC-Variable Domain of Humanized Antibody Variant (huMA79b.v18)
<400> 62
<210> 63
   <211> 15
   <212> PRT
   <213> Artificial sequence
<220>
   <223> HVR1-LC of Humanized Antibody Variant (huMA79b.v28)
<400> 63
   Lys Ala Ser Gln Ser Val Asp Tyr Glu Gly Asp Ser Phe Leu Asn
   1               5                   10                  15
<210> 64
   <211> 7
   <212> PRT
   <213> Artificial sequence
<220>
   <223> HVR2-LC of Humanized Antibody Variant (huMA79b.v28)
<400> 64
   Ala Ala Ser Asn Leu Glu Ser
   1               5
<210> 65
   <211> 9
   <212> PRT
   <213> Artificial sequence
<220>
   <223> HVR3-LC of Humanized Antibody Variant (huMA79b.v28)
<400> 65
   Gln Gln Ser Asn Glu Asp Pro Leu Thr
                   5
<210> 66
   <211> 10
   <212> PRT
   <213> Artificial sequence
<220>
   <223> HVR1-HC of Humanized Antibody Variant (huMA79b.v28)
<400> 66
   Gly Tyr Thr Phe Ser Ser Tyr Trp Ile Glu
                   5                   10
<210> 67
   <211> 18
   <212> PRT
   <213> Artificial sequence
<220>
   <223> HVR2-HLC of Humanized Antibody Variant (huMA79b.v28)
<400> 67
   Gly Glu Ile Leu Pro Gly Gly Gly Asp Thr Asn Tyr Asn Glu Ile
    1               5                  10                  15
   Phe Lys Gly
<210> 68
   <211> 10
   <212> PRT
   <213> Artificial sequence
<220>
   <223> HVR3-HC of Humanized Antibody Variant (huMA79b.v28)
<400> 68
   Thr Arg Arg Val Pro Ile Arg Leu Asp Tyr
                    5                  10
<210> 69
   <211> 112
   <212> PRT
   <213> Artificial sequence
<220>
   <223> LC-variable domain of (huMA79b.v28)
<400> 69
   Asp Ile Gln Leu Thr Gln Ser Pro Ser Ser Leu Ser Ala Ser Val
    1               5                  10                  15
   Gly Asp Arg Val Thr Ile Thr Cys Lys Ala Ser Gln Ser Val Asp
                   20                  25                  30
   Tyr Glu Gly Asp Ser Phe Leu Asn Trp Tyr Gln Gln Lys Pro Gly
                   35                  40                  45
   Lys Ala Pro Lys Leu Leu Ile Tyr Ala Ala Ser Asn Leu Glu Ser
                   50                  55                  60
   Gly Val Pro Ser Arg Phe Ser Gly Ser Gly Ser Gly Thr Asp Phe
                   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
                   95                  100                 105
   Thr Lys Val Glu Ile Lys Arg
                   110
<210> 70
   <211> 117
   <212> PRT
   <213> Artificial sequence
<220>
   <223> HC-Variable Domain of Humanized Antibody (huMA79b.v28)
<400> 70
<210> 71
   <211> 15
   <212> PRT
   <213> Artificial sequence
<220>
   <223> HVR1-LC of Humanized Antibody Variant (huMA79b.v32)
<400> 71
   Lys Ala Ser Gln Ser Val Asp Tyr Ser Gly Asp Ser Phe Leu Asn
   1               5                   10                  15
<210> 72
   <211> 7
   <212> PRT
   <213> Artificial sequence
<220>
   <223> HVR2-LC of Humanized Antibody Variant (huMA79b.v32)
<400> 72
   Ala Ala Ser Asn Leu Glu Ser
                   5
<210> 73
   <211> 9
   <212> PRT
   <213> Artificial sequence
<220>
   <223> HVR3-LC of Humanized Antibody Variant (huMA79b.v32)
<400> 73
   Gln Gln Ser Asn Glu Asp Pro Leu Thr
   5
<210> 74
   <211> 10
   <212> PRT
   <213> Artificial sequence
<220>
   <223> HVR1-HC of Humanized Antibody Variant (huMA79b.v32)
<400> 74
   Gly Tyr Thr Phe Ser Ser Tyr Trp Ile Glu
   5 10
<210> 75
   <211> 18
   <212> PRT
   <213> Artificial sequence
<220>
   <223> HVR2-HC of Humanized Antibody Variant (huMA79b.v32)
<400> 75
   Gly Glu Ile Leu Pro Gly Gly Gly Asp Thr Asn Tyr Asn Glu Ile
   1 5 10 15
   Phe Lys Gly
<210> 76
   <211> 10
   <212> PRT
   <213> Artificial sequence
<220>
   <223> HVR3-HC of Humanized Antibody Variant (huMA79b.v32)
<400> 76
   Thr Arg Arg Val Pro Ile Arg Leu Asp Tyr
   5 10
<210> 77
   <211> 112
   <212> PRT
   <213> Artificial sequence
<220>
   <223> LC -Variable Domain of Humanized Antibody Variant (huMA79b.v32)
<400> 77
   Asp Ile Gln Leu Thr Gln Ser Pro Ser Ser Leu Ser Ala Ser Val
    1               5                  10                  15
   Gly Asp Arg Val Thr Ile Thr Cys Lys Ala Ser Gln Ser Val Asp
                   20                  25                  30
   Tyr Ser Gly Asp Ser Phe Leu Asn Trp Tyr Gln Gln Lys Pro Gly
                   35                  40                  45
   Lys Ala Pro Lys Leu Phe Ile Tyr Ala Ala Ser Asn Leu Glu Ser
                   50                  55                  60
   Gly Val Pro Ser Arg Phe Ser Gly Ser Gly Ser Gly Thr Asp Phe
                   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
                   95                  100                 105
   Thr Lys Val Glu Ile Lys Arg
                   110
<210> 78
   <211> 117
   <212> PRT
   <213> Artificial sequence
<220>
   <223> HC-Variable Domain of Humanized Antibody Variant (huMA79b.v32)
<400> 78
<210> 79
   <211> 893
   <212> DNA
   <213> Macaca fascicularis
<400> 79
   tcatggtgat ggtgatgatg accggtacgc gtagaatcga gaccgaggag 50
   agggttaggg ataggcttac cttcgaaccg cgggccctct agactcgagc 100
   ggccgccact gtgctggata tctgcagaat tgcccttggg gacagagcag 150
   tgaccatggc caggctggcg ttgtctcctg tgtccagcca ctggctggtg 200
   gcgttgctgc tgctgctctc agcagctgag ccagtgccag cagccaaatc 250
   agaggacctg tacccgaatc ccaaaggtag tgcttgttct cggatctggc 300
   agagcccacg tttcatagcc aggaaacggg gcttcacggt gaaaatgcac 350
   tgctacgtga ccaacagcac cttcagcatc gtgagctggc tccggaagcg 400
   ggagacggac aaggagcccc aacaggtgaa cctggagcag ggccacatgc 450
   atcagaccca aaacagctct gtcaccaccc tcatcatcca agacatccgg 500
   tttgaggaca acggcatcta cttctgtcag caggagtgca gcaagacctc 550
   ggaggtctac cggggctgcg gcacggagct gcgagtcatg ggcttcagca 600
   ccttggcaca gctgaagcag aggaacacgc tgaaggatgg catcatcatg 650
   atccagacgc tgctgatcat cctcttcatc atcgtgccca tcttcctgct 700
   gctggacaag gatgacagca aggccggcat ggaggaagat cacacctacg 750
   agggcctgga cattgaccag acggccacct acgaggacat agtgacgctg 800
   cggacagggg aagtgaagtg gtctgtgggt gagcacccag gtcaggagtg 850
   agagccagga cctccccacg gcctgggtgc aggctcccca  gcc 893
<210> 80
   <211> 231
   <212> PRT
   <213> Macaca fascicularis
<400> 80
   Met Ala Arg Leu Ala Leu Ser Pro Val Ser Ser His Trp Leu Val
   1 5 10 15
   Ala Leu Leu Leu Leu Leu Ser Ala Ala Glu Pro Val Pro Ala Ala
   20 25 30
   Lys Ser Glu Asp Leu Tyr Pro Asn Pro Lys Gly Ser Ala Cys Ser
   35 40 45
   Arg Ile Trp Gln Ser Pro Arg Phe Ile Ala Arg Lys Arg Gly Phe
   50 55 60
   Thr Val Lys Met His Cys Tyr Val Thr Asn Ser Thr Phe Ser Ile
   65 70 75
   Val Ser Trp Leu Arg Lys Arg Glu Thr Asp Lys Glu Pro Gln Gln
   80 85 90
   Val Asn Leu Glu Gln Gly His Met His Gln Thr Gln Asn Ser Ser
   95 100 105
   Val Thr Thr Leu Ile Ile Gln Asp Ile Arg Phe Glu Asp Asn Gly
   110 115 120
   Ile Tyr Phe Cys Gln Gln Glu Cys Ser Lys Thr Ser Glu Val Tyr
   125 130 135
   Arg Gly Cys Gly Thr Glu Leu Arg Val Met Gly Phe Ser Thr Leu
   140 145 150
   Ala Gln Leu Lys Gln Arg Asn Thr Leu Lys Asp Gly Ile Ile Met
   155 160 165
   Ile Gln Thr Leu Leu Ile Ile Leu Phe Ile Ile Val Pro Ile Phe
   170 175 180
   Leu Leu Leu Asp Lys Asp Asp Ser Lys Ala Gly Met Glu Glu Asp
   185 190 195
   His Thr Tyr Glu Gly Leu Asp Ile Asp Gln Thr Ala Thr Tyr Glu
   200 205 210
   Asp Ile Val Thr Leu Arg Thr Gly Glu Val Lys Trp Ser Val Gly
   215 220 225
   Glu His Pro Gly Gln Glu
   230
<210> 81
   <211> 218
   <212> PRT
   <213> Artificial sequence
<220>
   <223> Chimeric anti-cyno CD79b (ch10D10)- LC
<400> 81
   Asp Ile Val Leu Thr Gln Ser Pro Pro Ser Leu Ala Val Ser Leu
   1               5                   10                  15
   Gly Gln Arg Ala Thr Ile Ser Cys Arg Ala Ser Glu Ser Val Asp
                   20                  25                  30
   Ser Tyr Gly Lys Thr Phe Met His Trp His Gln Gln Lys Pro Gly
                   35                  40                  45
   Gln Pro Pro Lys Leu Leu Ile Tyr Arg Val Ser Asn Leu Glu Ser
                   50                  55                  60
   Gly Ile Pro Ala Arg Phe Ser Gly Ser Gly Ser Arg Thr Asp Phe
                   65                  70                  75
   Thr Leu Thr Ile Asn Pro Val Glu Ala Asp Asp Val Ala Thr Tyr
                   80                  85                  90
   Tyr Cys Gln Gln Ser Asn Glu Asp Pro Phe Thr Phe Gly Gly Gly
                   95                  100                 105
   Thr Lys Leu Glu Ile Lys Arg Thr Val Ala Ala Pro Ser Val Phe
                   110                 115                 120
   Ile Phe Pro Pro Ser Asp Glu Gln Leu Lys Ser Gly Thr Ala Ser
                   125                 130                 135
   Val Val Cys Leu Leu Asn Asn Phe Tyr Pro Arg Glu Ala Lys Val
                   140                 145                 150
   Gln Trp Lys Val Asp Asn Ala Leu Gln Ser Gly Asn Ser Gln Glu
                   155                 160                 165
   Ser Val Thr Glu Gln Asp Ser Lys Asp Ser Thr Tyr Ser Leu Ser
                   170                 175                 180
   Ser Thr Leu Thr Leu Ser Lys Ala Asp Tyr Glu Lys His Lys Val
                   185                 190                 195
   Tyr Ala Cys Glu Val Thr His Gln Gly Leu Ser Ser Pro Val Thr
                   200                 205                 210
   Lys Ser Phe Asn Arg Gly Glu Cys
                   215
<210> 82
   <211> 441
   <212> PRT
   <213> Artificial sequence
<220>
   <223> Chimeric anti-cyno CD79b (ch10D10)- LC
<400> 82
   Glu Val Gln Leu Gln Glu Ser Gly Pro Gly Leu Val Lys Pro Ser
   1               5                   10                  15
   Gln Ser Leu Ser Leu Thr Cys Thr Val Thr Gly Tyr Ser Ile Thr
                   20                  25                  30
   Ser Asp Tyr Ala Trp Asn Trp Ile Arg Gln Phe Pro Gly Asn Lys
                   35                  40                  45
   Leu Glu Trp Met Gly Asn Ile Trp Tyr Ser Gly Ser Thr Thr Tyr
                   50                  55                  60
   Asn Pro Ser Leu Lys Ser Arg Ile Ser Ile Thr Arg Asp Thr Ser
                   65                  70                  75
   Lys Asn Gln Phe Phe Leu Gln Leu Asn Ser Val Thr Ser Glu Asp
                   80                  85                  90
   Thr Ala Thr Tyr Tyr Cys Ser Arg Met Asp Phe Trp Gly Gln Gly
                   95                  100                 105
   Thr Thr Leu Thr Val Ser Ser Ala Ser Thr Lys Gly Pro Ser Val
                   110                 115                 120
   Phe Pro Leu Ala Pro Ser Ser Lys Ser Thr Ser Gly Gly Thr Ala
                   125                 130                 135
   Ala Leu Gly Cys Leu Val Lys Asp Tyr Phe Pro Glu Pro Val Thr
                   140                 145                 150
   Val Ser Trp Asn Ser Gly Ala Leu Thr Ser Gly Val His Thr Phe
                   155                 160                 165
   Pro Ala Val Leu Gln Ser Ser Gly Leu Tyr Ser Leu Ser Ser Val
                   170                 175                 180
   Val Thr Val Pro Ser Ser Ser Leu Gly Thr Gln Thr Tyr Ile Cys
                   185                 190                 195
   Asn Val Asn His Lys Pro Ser Asn Thr Lys Val Asp Lys Lys Val
                   200                 205                 210
   Glu Pro Lys Ser Cys Asp Lys Thr His Thr Cys Pro Pro Cys Pro
                   215                 220                 225
   Ala Pro Glu Leu Leu Gly Gly Pro Ser Val Phe Leu Phe Pro Pro
                   230                 235                 240
   Lys Pro Lys Asp Thr Leu Met Ile Ser Arg Thr Pro Glu Val Thr
                   245                 250                 255
   Cys Val Val Val Asp Val Ser His Glu Asp Pro Glu Val Lys Phe
                   260                 265                 270
   Asn Trp Tyr Val Asp Gly Val Glu Val His Asn Ala Lys Thr Lys
                   275                 280                 285
   Pro Arg Glu Glu Gln Tyr Asn Ser Thr Tyr Arg Val Val Ser Val
                   290                 295                 300
   Leu Thr Val Leu His Gln Asp Trp Leu Asn Gly Lys Glu Tyr Lys
                   305                 310                 315
   Cys Lys Val Ser Asn Lys Ala Leu Pro Ala Pro Ile Glu Lys Thr
                   320                 325                 330
   Ile Ser Lys Ala Lys Gly Gln Pro Arg Glu Pro Gln Val Tyr Thr
                   335                 340                 345
   Leu Pro Pro Ser Arg Glu Glu Met Thr Lys Asn Gln Val Ser Leu
                   350                 355                 360
   Thr Cys Leu Val Lys Gly Phe Tyr Pro Ser Asp Ile Ala Val Glu
                   365                 370                 375
   Trp Glu Ser Asn Gly Gln Pro Glu Asn Asn Tyr Lys Thr Thr Pro
                   380                 385                 390
   Pro Val Leu Asp Ser Asp Gly Ser Phe Phe Leu Tyr Ser Lys Leu
   395 400 405
   Thr Val Asp Lys Ser Arg Trp Gln Gln Gly Asn Val Phe Ser Cys
   410 415 420
   Ser Val Met His Glu Ala Leu His Asn His Tyr Thr Gln Lys Ser
   425 430 435
   Leu Ser Leu Ser Pro Gly
   440
<210> 83
   <211> 10
   <212> PRT
   <213> Artificial sequence
<220>
   <223> Thio-huMA79b HC-variant
<400> 83
   Glu Val Gln Leu Cys Glu Ser Gly Gly Gly
   5 10
<210> 84
   <211> 11
   <212> PRT
   <213> Artificial sequence
<220>
   <223> Thio-huMA79b HC-variant
<400> 84
   Leu Arg Leu Ser Cys Cys Ala Ser Gly Tyr Thr
   5 10
<210> 85
   <211> 11
   <212> PRT
   <213> Artificial sequence
<220>
   <223> Thio-huMA79b HC-variant
<400> 85
   Met Asn Ser Leu Arg Cys Glu Asp Thr Ala Val
   5 10
<210> 86
   <211> 11
   <212> PRT
   <213> Artificial sequence
<220>
   <223> Thio-huMA79b HC-variant
<400> 86
   Thr Leu Val Thr Val Cys Ser Ala Ser Thr Lys
   5 10
<210> 87
   <211> 11
   <212> PRT
   <213> Artificial sequence
<220>
   <223> Thio-huMA79b HC-variant
<400> 87
   Val Thr Val Ser Ser Cys Ser Thr Lys Gly Pro
   5 10
<210> 88
   <211> 11
   <212> PRT
   <213> Artificial sequence
<220>
   <223> Thio-huMA79b HC-variant
<400> 88
   Val Ser Ser Ala Ser Cys Lys Gly Pro Ser Val
   5 10
<210> 89
   <211> 11
   <212> PRT
   <213> Artificial sequence
<220>
   <223> Thio-huMA79b HC-variant
<400> 89
   Trp Tyr Val Asp Gly Cys Glu Val His Asn Ala
   5 10
<210> 90
   <211> 11
   <212> PRT
   <213> Artificial sequence
<220>
   <223> Thio-huMA79b HC-variant
<400> 90
   Lys Gly Phe Tyr Pro Cys Asp Ile Ala Val Glu
   5 10
<210> 91
   <211> 11
   <212> PRT
   <213> Artificial sequence
<220>
   <223> Thio-chMA79b HC-variant
<400> 91
   Pro Pro Val Leu Asp Cys Asp Gly Ser Phe Phe
                       5                   10
<210> 92
   <211> 10
   <212> PRT
   <213> Artificial sequence
<220>
   <223> Thio-chMA79b HC-variant
<400> 92
   Glu Val Gln Leu Cys Gln Ser Gly Ala Glu
                       5                   10
<210> 93
   <211> 11
   <212> PRT
   <213> Artificial sequence
<220>
   <223> Thio-chMA79b HC-variant
<400> 93
   Val Lys Ile Ser Cys Cys Ala Thr Gly Tyr Thr
                       5                   10
<210> 94
   <211> 11
   <212> PRT
   <213> Artificial sequence
<220>
   <223> Thio-chMA79b HC-variant
<400> 94
   Leu Ser Ser Leu Thr Cys Glu Asp Ser Ala Val
                       5                   10
<210> 95
   <211> 11
   <212> PRT
   <213> Artificial sequence
<220>
   <223> Thio-chMA79b HC-variant
<400> 95
   Thr Ser Val Thr Val Cys Ser Ala Ser Thr Lys
   5 10
<210> 96
   <211> 11
   <212> PRT
   <213> Artificial sequence
<220>
   <223> Thio-chMA79b HC-variant
<400> 96
   Val Thr Val Ser Ser Cys Ser Thr Lys Gly Pro
   5 10
<210> 97
   <211> 11
   <212> PRT
   <213> Artificial sequence
<220>
   <223> Thio-chMA79b HC-variant
<400> 97
   Val Ser Ser Ala Ser Cys Lys Gly Pro Ser Val
   5 10
<210> 98
   <211> 11
   <212> PRT
   <213> Artificial sequence
<220>
   <223> Thio-chMA79b HC-variant
<400> 98
   Trp Tyr Val Asp Gly Cys Glu Val His Asn Ala
   5 10
<210> 99
   <211> 11
   <212> PRT
   <213> Artificial sequence
<220>
   <223> Thio-chMA79b HC-variant
<400> 99
   Lys Gly Phe Tyr Pro Cys Asp Ile Ala Val Glu
   5 10
<210> 100
   <211> 11
   <212> PRT
   <213> Artificial sequence
<220>
   <223> Thio-chMA79b HC-variant
<400> 100
   Pro Pro Val Leu Asp Cys Asp Gly Ser Phe Phe
                       5                   10
<210> 101
   <211> 10
   <212> PRT
   <213> Artificial sequence
<220>
   <223> Thio anti-cynoCD79b(ch10D10)-HC-variant
<400> 101
   Glu Val Gln Leu Cys Glu Ser Gly Pro Gly
                   5                   10
<210> 102
   <211> 11
   <212> PRT
   <213> Artificial sequence
<220>
   <223> Thio anti-cynoCD79b(ch10D10)-HC-variant
<400> 102
   Leu Ser Leu Thr Cys Cys Val Thr Gly Tyr Ser
                       5                   10
<210> 103
   <211> 11
   <212> PRT
   <213> Artificial sequence
<220>
   <223> Thio anti-cynoCD79b(ch10D10)-HC-variant
<400> 103
   Leu Asn Ser Val Thr Cys Glu Asp Thr Ala Thr
                       5                   10
<210> 104
   <211> 11
   <212> PRT
   <213> Artificial sequence
<220>
   <223> Thio anti-cynoCD79b(ch10D10)-HC-variant
<400> 104
   Thr Thr Leu Thr Val Cys Ser Ala Ser Thr Lys
   1               5                   10
<210> 15
   <211> 11
   <212> PRT
   <213> Artificial sequence
<220>
   <223> Thio anti-cynoCD79b(ch10D10)-HC-variant
<400> 105
<210> 106
   <211> 11
   <212> PRT
   <213> Artificial sequence
<220>
   <223> Thio anti-cynoCD79b(ch10D10)-HC-variant
<400> 106
   Val Ser Ser Ala Ser Cys Lys Gly Pro Ser Val
   1               5                   10
<210> 107
   <211> 11
   <212> PRT
   <213> Artificial sequence
<220>
   <223> Thio-anti-cynoCD79b (ch10D10)-HC-variant
<400> 107
   Trp Tyr Val Asp Gly Cys Glu Val His Asn Ala
   1               5                   10
<210> 108
   <211> 11
   <212> PRT
   <213> Artificial sequence
<220>
   <223> Thio anti-cynoCD79b(ch10D10)-HC-variant
<400> 108
   Lys Gly Phe Tyr Pro Cys Asp Ile Ala Val Glu
   1               5                   10
<210> 109
   <211> 11
   <212> PRT
   <213> Artificial sequence
<220>
   <223> Thio anti-cynoCD79b(ch10D10)-HC-variant
<400> 109
   Pro Pro Val Leu Asp Cys Asp Gly Ser Phe Phe
                   5                   10
<210> 110
   <211> 11
   <212> PRT
   <213> Artificial sequence
<220>
   <223> Thio-huMA79b LC-variant
<400> 110
   Ser Leu Ser Ala Ser Cys Gly Asp Arg Val Thr
                   5                   10
<210> 111
   <211> 11
   <212> PRT
   <213> Artificial sequence
<220>
   <223> Thio-huMA79b LC-variant
<400> 111
   Glu Ile Lys Arg Thr Cys Ala Ala Pro Ser Val
                   5                   10
<210> 112
   <211> 11
   <212> PRT
   <213> Artificial sequence
<220>
   <223> Thio-huMA79b LC-variant
<400> 112
   Thr Val Ala Ala Pro Cys Val Phe Ile Phe Pro
                   5                   10
<210> 113
   <211> 11
   <212> PRT
   <213> Artificial sequence
<220>
   <223> Thio-huMA79b LC-variant
<400> 113
   Phe Ile Phe Pro Pro Cys Asp Glu Gln Leu Lys
   1               5                   10
<210> 114
   <211> 11
   <212> PRT
   <213> Artificial sequence
<220>
   <223> Thio-huMA79b LC-variant
<400> 114
   Asp Glu Gln Leu Lys Cys Gly Thr Ala Ser Val
                       5                   10
<210> 115
   <211> 11
   <212> PRT
   <213> Artificial sequence
<220>
   <223> Thio-huMA79b LC-variant
<400> 115
   Val Thr Glu Gln Asp Cys Lys Asp Ser Thr Tyr
                       5                   10
<210> 116
   <211> 11
   <212> PRT
   <213> Artificial sequence
<220>
   <223> Thio-huMA79b LC-variant
<400> 116
   Gly Leu Ser Ser Pro Cys Thr Lys Ser Phe Asn
                       5                   10
<210> 117
   <211> 11
   <212> PRT
   <213> Artificial sequence
<220>
   <223> Thio-chMA79b LC-variant
<400> 117
   Ser Leu Ala Val Ser Cys Gly Gln Arg Ala Thr
                       5                   10
<210> 118
   <211> 11
   <212> PRT
   <213> Artificial sequence
<220>
   <223> Thio-chMA79b LC-variant
<400> 118
   Glu Leu Lys Arg Thr Cys Ala Ala Pro Ser Val
                   5                   10
<210> 119
   <211> 11
   <212> PRT
   <213> Artificial sequence
<220>
   <223> Thio-chMA79b LC-variant
<400> 119
   Thr Val Ala Ala Pro Cys Val Phe Ile Phe Pro
                   5                   10
<210> 120
   <211> 11
   <212> PRT
   <213> Artificial sequence
<220>
   <223> Thio-chMA79b LC-variant
<400> 120
   Phe Ile Phe Pro Pro Cys Asp Glu Gln Leu Lys
                   5                   10
<210> 121
   <211> 11
   <212> PRT
   <213> Artificial sequence
<220>
   <223> Thio-chMA79b LC-variant
<400> 121
   Asp Glu Gln Leu Lys Cys Gly Thr Ala Ser Val
                   5                   10
<210> 122
   <211> 11
   <212> PRT
   <213> Artificial sequence
<220>
   <223> Thio-chMA79b LC-variant
<400> 122
   Val Thr Glu Gln Asp Cys Lys Asp Ser Thr Tyr
   1               5                   10
<210> 123
   <211> 11
   <212> PRT
   <213> Artificial sequence
<220>
   <223> Thio-chMA79b LC-variant
<400> 123
   Gly Leu Ser Ser Pro Cys Thr Lys Ser Phe Asn
                   5                   10
<210> 124
   <211> 11
   <212> PRT
   <213> Artificial sequence
<220>
   <223> Thio anti-cynoCD79b(ch10D10)-HC-variant
<400> 124
   Ser Leu Ala Val Ser Cys Gly Gln Arg Ala Thr
                   5                   10
<210> 125
   <211> 11
   <212> PRT
   <213> Artificial sequence
<220>
   <223> Thio anti-cynoCD79b(ch10D10)-HC-variant
<400> 125
   Glu Ile Lys Arg Thr Cys Ala Ala Pro Ser Val
                   5                   10
<210> 126
   <211> 11
   <212> PRT
   <213> Artificial sequence
<220>
   <223> Thio anti-cynoCD79b(ch10D10)-HC-variant
<400> 126
   Thr Val Ala Ala Pro Cys Val Phe Ile Phe Pro
                   5                   10
<210> 127
   <211> 11
   <212> PRT
   <213> Artificial sequence
<220>
   <223> Thio anti-cynoCD79b(ch10D10)-HC-variant
<400> 127
   Phe Ile Phe Pro Pro Cys Asp Glu Gln Leu Lys
   1               5                   10
<210> 128
   <211> 11
   <212> PRT
   <213> Artificial sequence
<220>
   <223> Thio anti-cynoCD79b(ch10D10)-HC-variant
<400> 128
   Asp Glu Gln Leu Lys Cys Gly Thr Ala Ser Val
                       5                   10
<210> 129
   <211> 11
   <212> PRT
   <213> Artificial sequence
<220>
   <223> Thio anti-cynoCD79b(ch10D10)-HC-variant
<400> 129
   Val Thr Glu Gln Asp Cys Lys Asp Ser Thr Tyr
                       5                   10
<210> 130
   <211> 11
   <212> PRT
   <213> Artificial sequence
<220>
   <223> Thio anti-cynoCD79b(ch10D10)-HC-variant
<400> 130
   Gly Leu Ser Ser Pro Cys Thr Lys Ser Phe Asn
                       5                   10
<210> 131
   <211> 23
   <212> PRT
   <213> Artificial sequence
<220>
   <223> FR1-LC of Humanized Antibody Variant (huMA79b.v17)
<400> 131
   Asp Ile Gln Leu Thr Gln Ser Pro Ser Ser Leu Ser Ala Ser Val
   1               5                   10                  15
   Gly Asp Arg Val Thr Ile Thr Cys
               20
<210> 132
   <211> 15
   <212> PRT
   <213> Artificial sequence
<220>
   <223> FR2-LC Humanized Antibody variant huMA79b.v17)
<400> 132
   Trp Tyr Gln Gln Lys Pro Gly Lys Ala Pro Lys Leu Leu Ile Tyr
   1               5                   10                  15
<210> 133
   <211> 32
   <212> PRT
   <213> Artificial sequence
<220>
   <223> FR3-LC of Humanized Antibody variant (huMA79b.v17)
<400> 133
   Gly Val Pro Ser Arg Phe Ser Gly Ser Gly Ser Gly Thr Asp Phe
   1               5                   10                  15
   Thr Leu Thr Ile Ser Ser Leu Gln Pro Glu Asp Phe Ala Thr Tyr
               20                  25                  30
   Tyr Cys
<210> 134
   <211> 11
   <212> PRT
   <213> Artificial sequence
<220>
   <223> FR4-LC of Humanized Antibody Variant (huMA79b.v17)
<400> 134
   Phe Gly Gln Gly Thr Lys Val Glu Ile Lys Arg
                   5                   10
<210> 135
   <211> 106
   <212> PRT
   <213> Artificial sequence
<220>
   <223> CL1-LC of Humanized Antibody Variant (huMA79b.v17)
<400> 135
   Thr Val Ala Ala Pro Ser Val Phe Ile Phe Pro Pro Ser Asp Glu
   1               5                   10                  15
   Gln Leu Lys Ser Gly Thr Ala Ser Val Val Cys Leu Leu Asn Asn
                   20                  25                  30
   Phe Tyr Pro Arg Glu Ala Lys Val Gln Trp Lys Val Asp Asn Ala
                   35                  40                  45
   Leu Gln Ser Gly Asn Ser Gln Glu Ser Val Thr Glu Gln Asp Ser
                   50                  55                  60
   Lys Asp Ser Thr Tyr Ser Leu Ser Ser Thr Leu Thr Leu Ser Lys
                   65                  70                  75
   Ala Asp Tyr Glu Lys His Lys Val Tyr Ala Cys Glu Val Thr His
                   80                  85                  90
   Gln Gly Leu Ser Ser Pro Val Thr Lys Ser Phe Asn Arg Gly Glu
                   95                  100                 105
   Cys
<210> 136
   <211> 25
   <212> PRT
   <213> Artificial sequence
<220>
   <223> FR1-HC of Humanized Antibody Variant (huMA79b.v17)
<400> 136
<210> 137
   <211> 13
   <212> PRT
   <213> Artificial sequence
<220>
   <223> FR2-HC of Humanized Antibody Variant (huMA79b.v17)
<400> 137
   Trp Val Arg Gln Ala Pro Gly Lys Gly Leu Glu Trp Ile
                   5                   10
<210> 138
   <211> 30
   <212> PRT
   <213> Artificial sequence
<220>
   <223> FR3-HC of Humanized Antibody Variant (huMA79b.v17)
<400> 138
   Arg Ala Thr Phe Ser Ala Asp Thr Ser Lys Asn Thr Ala Tyr Leu
   1               5                   10                  15
   Gln Met Asn Ser Leu Arg Ala Glu Asp Thr Ala Val Tyr Tyr Cys
                   20                  25                  30
<210> 139
   <211> 11
   <212> PRT
   <213> Artificial sequence
<220>
   <223> FR4-HC of Humanized Antibody Variant (huMA79b.v17)
<400> 139
   Trp Gly Gln Gly Thr Leu Val Thr Val Ser Ser
                   5                   10
<210> 140
   <211> 108
   <212> PRT
   <213> Artificial Sequence
<220>
   <223> CH1-HC of Humanized Antibody Varient (huMA79b.v17)
<400> 140
   Ala Ser Thr Lys Gly Pro Ser Val Phe Pro Leu Ala Pro Ser Ser
   1               5                   10                  15
   Lys Ser Thr Ser Gly Gly Thr Ala Ala Leu Gly Cys Leu Val Lys
                   20                  25                  30
   Asp Tyr Phe Pro Glu Pro Val Thr Val Ser Trp Asn Ser Gly Ala
                   35                  40                  45
   Leu Thr Ser Gly Val His Thr Phe Pro Ala Val Leu Gln Ser Ser
                   50                  55                  60
   Gly Leu Tyr Ser Leu Ser Ser Val Val Thr Val Pro Ser Ser Ser
                   65                  70                  75
   Leu Gly Thr Gln Thr Tyr Ile Cys Asn Val Asn His Lys Pro Ser
   80 85 90
   Asn Thr Lys Val Asp Lys Lys Val Glu Pro Lys Ser Cys Asp Lys
   95 100 105
   Thr His Thr
<210> 141
   <211> 221
   <212> PRT
   <213> Artificial Sequence
<220>
   <223> Fc-HC of Humanized Antibody Variant (huMA79b.v17)
<400> 141
   Cys Pro Pro Cys Pro Ala Pro Glu Leu Leu Gly Gly Pro Ser Val
   1 5 10 15
   Phe Leu Phe Pro Pro Lys Pro Lys Asp Thr Leu Met Ile Ser Arg
   20 25 30
   Thr Pro Glu Val Thr Cys Val Val Val Asp Val Ser His Glu Asp
   35 40 45
   Pro Glu Val Lys Phe Asn Trp Tyr Val Asp Gly Val Glu Val His
   50 55 60
   Asn Ala Lys Thr Lys Pro Arg Glu Glu Gln Tyr Asn Ser Thr Tyr
   65 70 75
   Arg Val Val Ser Val Leu Thr Val Leu His Gln Asp Trp Leu Asn
   80 85 90
   Gly Lys Glu Tyr Lys Cys Lys Val Ser Asn Lys Ala Leu Pro Ala
   95 100 105
   Pro Ile Glu Lys Thr Ile Ser Lys Ala Lys Gly Gln Pro Arg Glu
   110 115 120
   Pro Gln Val Tyr Thr Leu Pro Pro Ser Arg Glu Glu Met Thr Lys
   125 130 135
   Asn Gln Val Ser Leu Thr Cys Leu Val Lys Gly Phe Tyr Pro Ser
   140 145 150
   Asp Ile Ala Val Glu Trp Glu Ser Asn Gly Gln Pro Glu Asn Asn
   155 160 165
   Tyr Lys Thr Thr Pro Pro Val Leu Asp Ser Asp Gly Ser Phe Phe
   170 175 180
   Leu Tyr Ser Lys Leu Thr Val Asp Lys Ser Arg Trp Gln Gln Gly
   185 190 195
   Asn Val Phe Ser Cys Ser Val Met His Glu Ala Leu His Asn His
   200 205 210
   Tyr Thr Gln Lys Ser Leu Ser Leu Ser Pro Gly
   215 220
<210> 142
   <211> 23
   <212> PRT
   <213> Artificial sequence
<220>
   <223> FR1-LC of Humanized Antibody Variant (huMA79b.v18)
<400> 142
   Asp Ile Gln Leu Thr Gln Ser Pro Ser Ser Leu Ser Ala Ser Val
   1 5 10 15
   Gly Asp Arg Val Thr Ile Thr Cys
   20
<210> 143
   <211> 15
   <212> PRT
   <213> Artificial sequence
<220>
   <223> FR2-LC of Humanized Antibody Variant (huMA79b.v18)
<400> 143
   Trp Tyr Gln Gln Lys Pro Gly Lys Ala Pro Lys Leu Leu Ile Tyr
   1               5                   10                  15
<210> 144
   <211> 32
   <212> PRT
   <213> Artificial sequence
<220>
   <223> FR3-LC of Humanized Antibody Variant (huMA79b.v18)
<400> 144
   Gly Val Pro Ser Arg Phe Ser Gly Ser Gly Ser Gly Thr Asp Phe
   1               5                   10                  15
   Thr Leu Thr Ile Ser Ser Leu Gln Pro Glu Asp Phe Ala Thr Tyr
                   20                  25                  30
   Tyr Cys
<210> 145
   <211> 11
   <212> PRT
   <213> Artificial sequence
<220>
   <223> FR4-LC of Humanized Antibody Variant (huMA79b.v18)
<400> 145
   Phe Gly Gln Gly Thr Lys Val Glu Ile Lys Arg
                   5                   10
<210> 146
   <211> 446
   <212> PRT
   <213> Artificial sequence
<220>
   <223> Heavy chain of huMA79bv.32
<400> 146
   Glu Val Gln Leu Val Glu Ser Gly Gly Gly Leu Val Gln Pro Gly
   1 5 10 15
   Gly Ser Leu Arg Leu Ser Cys Ala Ala Ser Gly Tyr Thr Phe Ser
   20 25 30
   Ser Tyr Trp Ile Glu Trp Val Arg Gln Ala Pro Gly Lys Gly Leu
   35 40 45
   Glu Trp Ile Gly Glu Ile Leu Pro Gly Gly Gly Asp Thr Asn Tyr
   50 55 60
   Asn Glu Ile Phe Lys Gly Arg Ala Thr Phe Ser Ala Asp Thr Ser
   65 70 75
   Lys Asn Thr Ala Tyr Leu Gln Met Asn Ser Leu Arg Ala Glu Asp
   80 85 90
   Thr Ala Val Tyr Tyr Cys Thr Arg Arg Val Pro Ile Arg Leu Asp
   95 100 105
   Tyr Trp Gly Gln Gly Thr Leu Val Thr Val Ser Ser Ala Ser Thr
   110 115 120
   Lys Gly Pro Ser Val Phe Pro Leu Ala Pro Ser Ser Lys Ser Thr
   125 130 135
   Ser Gly Gly Thr Ala Ala Leu Gly Cys Leu Val Lys Asp Tyr Phe
   140 145 150
   Pro Glu Pro Val Thr Val Ser Trp Asn Ser Gly Ala Leu Thr Ser
   155 160 165
   Gly Val His Thr Phe Pro Ala Val Leu Gln Ser Ser Gly Leu Tyr
   170 175 180
   Ser Leu Ser Ser Val Val Thr Val Pro Ser Ser Ser Leu Gly Thr
   185 190 195
   Gln Thr Tyr Ile Cys Asn Val Asn His Lys Pro Ser Asn Thr Lys
   200 205 210
   Val Asp Lys Lys Val Glu Pro Lys Ser Cys Asp Lys Thr His Thr
   215 220 225
   Cys Pro Pro Cys Pro Ala Pro Glu Leu Leu Gly Gly Pro Ser Val
   230 235 240
   Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp
   260 265 270
   Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn
                   305                 310                 315
   Gly Lys Glu Tyr Lys Cys Lys Val Ser Asn Lys Ala Leu Pro Ala
                   320                 325                 330
   Pro Ile Glu Lys Thr Ile Ser Lys Ala Lys Gly Gln Pro Arg Glu
                   335                 340                 345
   Pro Gln Val Tyr Thr Leu Pro Pro Ser Arg Glu Glu Met Thr Lys
                   350                 355                 360
   Asn Gln Val Ser Leu Thr Cys Leu Val Lys Gly Phe Tyr Pro Ser
                   365                 370                 375
   Asp Ile Ala Val Glu Trp Glu Ser Asn Gly Gln Pro Glu Asn Asn
                   380                 385                 390
   Tyr Lys Thr Thr Pro Pro Val Leu Asp Ser Asp Gly Ser Phe Phe
                   395                 400                 405
   Leu Tyr Ser Lys Leu Thr Val Asp Lys Ser Arg Trp Gln Gln Gly
                   410                 415                 420
   Asn Val Phe Ser Cys Ser Val Met His Glu Ala Leu His Asn His
                   425                 430                 435
   Tyr Thr Gln Lys Ser Leu Ser Leu Ser Pro Gly
                   440                 445
<210> 147
   <211> 106
   <212> PRT
   <213> Artificial Sequence
<220>
   <223> CL1-LC of Humanized Antibody Variant (huMA79b.v18)
<400> 147
   Thr Val Ala Ala Pro Ser Val Phe Ile Phe Pro Pro Ser Asp Glu
   1               5                   10                  15
   Gln Leu Lys Ser Gly Thr Ala Ser Val Val Cys Leu Leu Asn Asn
                   20                  25                  30
   Phe Tyr Pro Arg Glu Ala Lys Val Gln Trp Lys Val Asp Asn Ala
                   35                  40                  45
   Leu Gln Ser Gly Asn Ser Gln Glu Ser Val Thr Glu Gln Asp Ser
                   50                  55                  60
   Lys Asp Ser Thr Tyr Ser Leu Ser Ser Thr Leu Thr Leu Ser Lys
                   65                  70                  75
   Ala Asp Tyr Glu Lys His Lys Val Tyr Ala Cys Glu Val Thr His
                   80                  85                  90
   Gln Gly Leu Ser Ser Pro Val Thr Lys Ser Phe Asn Arg Gly Glu
                   95                  100                 105
   Cys
<210> 148
   <211> 25
   <212> PRT
   <213> Artificial sequence
<220>
   <223> FR1-HC of Humanized Antibody Variant (huMA79b.v18)
<400> 148
<210> 149
   <211> 13
   <212> PRT
   <213> Artificial sequence
<220>
   <223> FR2-HC of Humanized Antibody Variant (huMA79b.v18)
<400> 149
   Trp Val Arg Gln Ala Pro Gly Lys Gly Leu Glu Trp Ile
                   5                   10
<210> 150
   <211> 30
   <212> PRT
   <213> Artificial sequence
<220>
   <223> FR3-HC of Humanized Antibody Variant (huMA79b.v18)
<400> 150
   Arg Ala Thr Phe Ser Ala Asp Thr Ser Lys Asn Thr Ala Tyr Leu
    1               5                  10                  15
   Gln Met Asn Ser Leu Arg Ala Glu Asp Thr Ala Val Tyr Tyr Cys
                   20                  25                  30
<210> 151
   <211> 11
   <212> PRT
   <213> Artificial sequence
<220>
   <223> FR4-HC of Humanized Antibody Variant (huMA79b.v18)
<400> 151
   Trp Gly Gln Gly Thr Leu Val Thr Val Ser Ser
                    5                  10
<210> 152
   <211> 108
   <212> PRT
   <213> Artificial Sequence
<220>
   <223> CH1-HC of Humanized Antibody Variant (huMA79b.v18)
<400> 152
   Ala Ser Thr Lys Gly Pro Ser Val Phe Pro Leu Ala Pro Ser Ser
    1               5                  10                  15
   Lys Ser Thr Ser Gly Gly Thr Ala Ala Leu Gly Cys Leu Val Lys
                   20                  25                  30
   Asp Tyr Phe Pro Glu Pro Val Thr Val Ser Trp Asn Ser Gly Ala
                   35                  40                  45
   Leu Thr Ser Gly Val His Thr Phe Pro Ala Val Leu Gln Ser Ser
                   50                  55                  60
   Gly Leu Tyr Ser Leu Ser Ser Val Val Thr Val Pro Ser Ser Ser
                   65                  70                  75
   Leu Gly Thr Gln Thr Tyr Ile Cys Asn Val Asn His Lys Pro Ser
                   80                  85                  90
   Asn Thr Lys Val Asp Lys Lys Val Glu Pro Lys Ser Cys Asp Lys
                   95                  100                 105
   Thr His Thr
<210> 153
   <211> 221
   <212> PRT
   <213> Artificial Sequence
<220>
   <223> Fc-HC of Humanized Antibody Variant (huMA79b.v18)
<400> 153
   Cys Pro Pro Cys Pro Ala Pro Glu Leu Leu Gly Gly Pro Ser Val
   1               5                   10                  15
   Phe Leu Phe Pro Pro Lys Pro Lys Asp Thr Leu Met Ile Ser Arg
               20                  25                  30
   Thr Pro Glu Val Thr Cys Val Val Val Asp Val Ser His Glu Asp
               35                  40                  45
   Pro Glu Val Lys Phe Asn Trp Tyr Val Asp Gly Val Glu Val His
               50                  55                  60
   Asn Ala Lys Thr Lys Pro Arg Glu Glu Gln Tyr Asn Ser Thr Tyr
               65                  70                  75
   Arg Val Val Ser Val Leu Thr Val Leu His Gln Asp Trp Leu Asn
               80                  85                  90
   Gly Lys Glu Tyr Lys Cys Lys Val Ser Asn Lys Ala Leu Pro Ala
               95                  100                 105
   Pro Ile Glu Lys Thr Ile Ser Lys Ala Lys Gly Gln Pro Arg Glu
               110                 115                 120
   Pro Gln Val Tyr Thr Leu Pro Pro Ser Arg Glu Glu Met Thr Lys
               125                 130                 135
   Asn Gln Val Ser Leu Thr Cys Leu Val Lys Gly Phe Tyr Pro Ser
               140                 145                 150
   Asp Ile Ala Val Glu Trp Glu Ser Asn Gly Gln Pro Glu Asn Asn
               155                 160                 165
   Tyr Lys Thr Thr Pro Pro Val Leu Asp Ser Asp Gly Ser Phe Phe
               170                 175                 180
   Leu Tyr Ser Lys Leu Thr Val Asp Lys Ser Arg Trp Gln Gln Gly
               185                 190                 195
   Asn Val Phe Ser Cys Ser Val Met His Glu Ala Leu His Asn His
               200                 205                 210
   Tyr Thr Gln Lys Ser Leu Ser Leu Ser Pro Gly
               215                 220
<210> 154
   <211> 23
   <212> PRT
   <213> Artificial sequence
<220>
   <223> FR1-LC of Humanized Antibody Variant (huMA79b.v28)
<400> 154
   Asp Ile Gln Leu Thr Gln Ser Pro Ser Ser Leu Ser Ala Ser Val
   1 5 10 15
   Gly Asp Arg Val Thr Ile Thr Cys
   20
<210> 155
   <211> 15
   <212> PRT
   <213> Artificial sequence
<220>
   <223> FR2-LC of Humanized Antibody Variant (huMA79b.v28)
<400> 155
   Trp Tyr Gln Gln Lys Pro Gly Lys Ala Pro Lys Leu Leu Ile Tyr
   1 5 10 15
<210> 156
   <211> 32
   <212> PRT
   <213> Artificial sequence
<220>
   <223> FR3-LC of Humanized Antibody Variant (huMA79b.v28)
<400> 156
   Gly Val Pro Ser Arg Phe Ser Gly Ser Gly Ser Gly Thr Asp Phe
   1 5 10 15
   Thr Leu Thr Ile Ser Ser Leu Gln Pro Glu Asp Phe Ala Thr Tyr
   20 25 30
   Tyr Cys
<210> 157
   <211> 11
   <212> PRT
   <213> Artificial sequence
<220>
   <223> FR4-LC of Humanized Antibody Variant (huMA79b.v28)
<400> 157
   Phe Gly Gln Gly Thr Lys Val Glu Ile Lys Arg
   5 10
<210> 158
   <211> 106
   <212> PRT
   <213> Artificial Sequence
<220>
   <223> CL1-LC of Humanized Antibody Variant (huMA79b.v28)
<400> 158
   Thr Val Ala Ala Pro Ser Val Phe Ile Phe Pro Pro Ser Asp Glu
   1               5                   10                  15
   Gln Leu Lys Ser Gly Thr Ala Ser Val Val Cys Leu Leu Asn Asn
                   20                  25                  30
   Phe Tyr Pro Arg Glu Ala Lys Val Gln Trp Lys Val Asp Asn Ala
                   35                  40                  45
   Leu Gln Ser Gly Asn Ser Gln Glu Ser Val Thr Glu Gln Asp Ser
                   50                  55                  60
   Lys Asp Ser Thr Tyr Ser Leu Ser Ser Thr Leu Thr Leu Ser Lys
                   65                  70                  75
   Ala Asp Tyr Glu Lys His Lys Val Tyr Ala Cys Glu Val Thr His
                   80                  85                  90
   Gln Gly Leu Ser Ser Pro Val Thr Lys Ser Phe Asn Arg Gly Glu
                   95                  100                 105
   Cys
<210> 159
   <211> 25
   <212> PRT
   <213> Artificial sequence
<220>
   <223> FR1-HC of Humanized Antibody Variant (huMA79b.v28)
<400> 159
<210> 160
   <211> 13
   <212> PRT
   <213> Artificial sequence
<220>
   <223> FR2-HC of Humanized Antibody Variant (huMA79b.v28)
<400> 160
   Trp Val Arg Gln Ala Pro Gly Lys Gly Leu Glu Trp Ile
                   5                   10
<210> 161
   <211> 30
   <212> PRT
   <213> Artificial sequence
<220>
   <223> FR3-HC of Humanized Antibody Variant (huMA79b.v28)
<400> 161
   Arg Ala Thr Phe Ser Ala Asp Thr Ser Lys Asn Thr Ala Tyr Leu
   1 5 10 15
   Gln Met Asn Ser Leu Arg Ala Glu Asp Thr Ala Val Tyr Tyr Cys
   20 25 30
<210> 162
   <211> 11
   <212> PRT
   <213> Artificial sequence
<220>
   <223> FR4-HC of Humanized Antibody Variant (huMA79b.v28)
<400> 162
   Trp Gly Gln Gly Thr Leu Val Thr Val Ser Ser
   5 10
<210> 163
   <211> 108
   <212> PRT
   <213> Artificial Sequence
<220>
   <223> CH1-HC of Humanized Antibody Variant (huMA79b.v28)
<400> 163
   Ala Ser Thr Lys Gly Pro Ser Val Phe Pro Leu Ala Pro Ser Ser
   1 5 10 15
   Lys Ser Thr Ser Gly Gly Thr Ala Ala Leu Gly Cys Leu Val Lys
   20 25 30
   Asp Tyr Phe Pro Glu Pro Val Thr Val Ser Trp Asn Ser Gly Ala
   35 40 45
   Leu Thr Ser Gly Val His Thr Phe Pro Ala Val Leu Gln Ser Ser
   50 55 60
   Gly Leu Tyr Ser Leu Ser Ser Val Val Thr Val Pro Ser Ser Ser
   65 70 75
   Leu Gly Thr Gln Thr Tyr Ile Cys Asn Val Asn His Lys Pro Ser
   80 85 90
   Asn Thr Lys Val Asp Lys Lys Val Glu Pro Lys Ser Cys Asp Lys
   95 100 105
   Thr His Thr
<210> 164
   <211> 221
   <212> PRT
   <213> Artificial Sequence
<220>
   <223> Fc-HC of Humanized Antibody Variant (huMA79b.V28)
<400> 164
   Cys Pro Pro Cys Pro Ala Pro Glu Leu Leu Gly Gly Pro Ser Val
   1               5                   10                  15
   Phe Leu Phe Pro Pro Lys Pro Lys Asp Thr Leu Met Ile Ser Arg
                   20                  25                  30
   Thr Pro Glu Val Thr Cys Val Val Val Asp Val Ser His Glu Asp
                   35                  40                  45
   Pro Glu Val Lys Phe Asn Trp Tyr Val Asp Gly Val Glu Val His
                   50                  55                  60
   Asn Ala Lys Thr Lys Pro Arg Glu Glu Gln Tyr Asn Ser Thr Tyr
                   65                  70                  75
   Arg Val Val Ser Val Leu Thr Val Leu His Gln Asp Trp Leu Asn
                   80                  85                  90
   Gly Lys Glu Tyr Lys Cys Lys Val Ser Asn Lys Ala Leu Pro Ala
                   95                  100                 105
   Pro Ile Glu Lys Thr Ile Ser Lys Ala Lys Gly Gln Pro Arg Glu
                   110                 115                 120
   Pro Gln Val Tyr Thr Leu Pro Pro Ser Arg Glu Glu Met Thr Lys
                   125                 130                 135
   Asn Gln Val Ser Leu Thr Cys Leu Val Lys Gly Phe Tyr Pro Ser
                   140                 145                 150
   Asp Ile Ala Val Glu Trp Glu Ser Asn Gly Gln Pro Glu Asn Asn
                   155                 160                 165
   Tyr Lys Thr Thr Pro Pro Val Leu Asp Ser Asp Gly Ser Phe Phe
                   170                 175                 180
   Leu Tyr Ser Lys Leu Thr Val Asp Lys Ser Arg Trp Gln Gln Gly
                   185                 190                 195
   Asn Val Phe Ser Cys Ser Val Met His Glu Ala Leu His Asn His
                   200                 205                 210
   Tyr Thr Gln Lys Ser Leu Ser Leu Ser Pro Gly
                   215                 220
<210> 165
   <211> 23
   <212> PRT
   <213> Artificial sequence
<220>
   <223> FR1-LC of Humanized Antibody Variant (huMA79b.v32)
<400> 165
   Asp Ile Gln Leu Thr Gln Ser Pro Ser Ser Leu Ser Ala Ser Val
   1 5 10 15
   Gly Asp Arg Val Thr Ile Thr Cys
   20
<210> 166
   <211> 15
   <212> PRT
   <213> Artificial sequence
<220>
   <223> FR2-LC of Humanized Antibody Variant (huMA79b.v32)
<400> 166
   Trp Tyr Gln Gln Lys Pro Gly Lys Ala Pro Lys Leu Phe Ile Tyr
   1 5 10 15
<210> 167
   <211> 32
   <212> PRT
   <213> Artificial sequence
<220>
   <223> FR3-LC of Humanized Antibody Variant (huMA79b.v32)
<400> 167
   Gly Val Pro Ser Arg Phe Ser Gly Ser Gly Ser Gly Thr Asp Phe
   1 5 10 15
   Thr Leu Thr Ile Ser Ser Leu Gln Pro Glu Asp Phe Ala Thr Tyr
   20 25 30
   Tyr Cys
<210> 168
   <211> 11
   <212> PRT
   <213> Artificial sequence
<220>
   <223> FR4-LC of Humanized Antibody Variant (huMA79b.v32)
<400> 168
   Phe Gly Gln Gly Thr Lys Val Glu Ile Lys Arg
   5 10
<210> 169
   <211> 106
   <212> PRT
   <213> Artificial Sequence
<220>
   <223> CL1-LC of Humanized Antibody Variant (huMA79b.v32)
<400> 169
   Thr Val Ala Ala Pro Ser Val Phe Ile Phe Pro Pro Ser Asp Glu
   1               5                   10                  15
   Gln Leu Lys Ser Gly Thr Ala Ser Val Val Cys Leu Leu Asn Asn
                   20                  25                  30
   Phe Tyr Pro Arg Glu Ala Lys Val Gln Trp Lys Val Asp Asn Ala
                   35                  40                  45
   Leu Gln Ser Gly Asn Ser Gln Glu Ser Val Thr Glu Gln Asp Ser
                   50                  55                  60
   Lys Asp Ser Thr Tyr Ser Leu Ser Ser Thr Leu Thr Leu Ser Lys
                   65                  70                  75
   Ala Asp Tyr Glu Lys His Lys Val Tyr Ala Cys Glu Val Thr His
                   80                  85                  90
   Gln Gly Leu Ser Ser Pro Val Thr Lys Ser Phe Asn Arg Gly Glu
                   95                  100                 105
   Cys
<210> 170
   <211> 25
   <212> PRT
   <213> Artificial sequence
<220>
   <223> FR1-HC of Humanized Antibody Variant (huMA79b.v32)
<400> 170
<210> 171
   <211> 13
   <212> PRT
   <213> Artificial sequence
<220>
   <223> FR2-HC of Humanized Antibody Variant (huMA79b.v32)
<400> 171
   Trp Val Arg Gln Ala Pro Gly Lys Gly Leu Glu Trp Ile
                   5                   10
<210> 172
   <211> 30
   <212> PRT
   <213> Artificial sequence
<220>
   <223> FR3-HC of Humanized Antibody Variant (huMA79b.v32)
<400> 172
   Arg Ala Thr Phe Ser Ala Asp Thr Ser Lys Asn Thr Ala Tyr Leu
   1               5                   10                  15
   Gln Met Asn Ser Leu Arg Ala Glu Asp Thr Ala Val Tyr Tyr Cys
                   20                  25                  30
<210> 173
   <211> 11
   <212> PRT
   <213> Artificial sequence
<220>
   <223> FR4-HC of Humanized Antibody Variant (huMA79b.v32)
<400> 173
   Trp Gly Gln Gly Thr Leu Val Thr Val Ser Ser
                   5                   10
<210> 174
   <211> 108
   <212> PRT
   <213> Artificial Sequence
<220>
   <223> CH1-HC of Humanized Antibody Variant (huMA79b.v32)
<400> 174
   Ala Ser Thr Lys Gly Pro Ser Val Phe Pro Leu Ala Pro Ser Ser
   1               5                   10                  15
   Lys Ser Thr Ser Gly Gly Thr Ala Ala Leu Gly Cys Leu Val Lys
                   20                  25                  30
   Asp Tyr Phe Pro Glu Pro Val Thr Val Ser Trp Asn Ser Gly Ala
                   35                  40                  45
   Leu Thr Ser Gly Val His Thr Phe Pro Ala Val Leu Gln Ser Ser
                   50                  55                  60
   Gly Leu Tyr Ser Leu Ser Ser Val Val Thr Val Pro Ser Ser Ser
                   65                  70                  75
   Leu Gly Thr Gln Thr Tyr Ile Cys Asn Val Asn His Lys Pro Ser
                   80                  85                  90
   Asn Thr Lys Val Asp Lys Lys Val Glu Pro Lys Ser Cys Asp Lys
                   95                  100                 105
   Thr His Thr
<210> 175
   <211> 221
   <212> PRT
   <213> Artificial Sequence
<220>
   <223> Fc-HC of Humanized Antibody Variant (huMA79b.v32)
<400> 175
   Cys Pro Pro Cys Pro Ala Pro Glu Leu Leu Gly Gly Pro Ser Val
   1               5                   10                  15
   Phe Leu Phe Pro Pro Lys Pro Lys Asp Thr Leu Met Ile Ser Arg
                   20                  25                  30
   Thr Pro Glu Val Thr Cys Val Val Val Asp Val Ser His Glu Asp
                   35                  40                  45
   Pro Glu Val Lys Phe Asn Trp Tyr Val Asp Gly Val Glu Val His
                   50                  55                  60
   Asn Ala Lys Thr Lys Pro Arg Glu Glu Gln Tyr Asn Ser Thr Tyr
                   65                  70                  75
   Arg Val Val Ser Val Leu Thr Val Leu His Gln Asp Trp Leu Asn
                   80                  85                  90
   Gly Lys Glu Tyr Lys Cys Lys Val Ser Asn Lys Ala Leu Pro Ala
                   95                  100                 105
   Pro Ile Glu Lys Thr Ile Ser Lys Ala Lys Gly Gln Pro Arg Glu
                   110                 115                 120
   Pro Gln Val Tyr Thr Leu Pro Pro Ser Arg Glu Glu Met Thr Lys
                   125                 130                 135
   Asn Gln Val Ser Leu Thr Cys Leu Val Lys Gly Phe Tyr Pro Ser
                   140                 145                 150
   Asp Ile Ala Val Glu Trp Glu Ser Asn Gly Gln Pro Glu Asn Asn
                   155                 160                 165
   Tyr Lys Thr Thr Pro Pro Val Leu Asp Ser Asp Gly Ser Phe Phe
                   170                 175                 180
   Leu Tyr Ser Lys Leu Thr Val Asp Lys Ser Arg Trp Gln Gln Gly
                   185                 190                 195
   Asn Val Phe Ser Cys Ser Val Met His Glu Ala Leu His Asn His
                   200                 205                 210
   Tyr Thr Gln Lys Ser Leu Ser Leu Ser Pro Gly
                   215                 220
<210> 176
   <211> 218
   <212> PRT
   <213> Artificial sequence
<220>
   <223> Light chain of huMA79bv.17
<400> 176
   Asp Ile Gln Leu Thr Gln Ser Pro Ser Ser Leu Ser Ala Ser Val
   1               5                   10                  15
   Gly Asp Arg Val Thr Ile Thr Cys Lys Ala Ser Gln Ser Val Asp
                   20                  25                  30
   Tyr Asp Gly Asp Ser Phe Leu Asn Trp Tyr Gln Gln Lys Pro Gly
                   35                  40                  45
   Lys Ala Pro Lys Leu Leu Ile Tyr Ala Ala Ser Asn Leu Glu Ser
                   50                  55                  60
   Gly Val Pro Ser Arg Phe Ser Gly Ser Gly Ser Gly Thr Asp Phe
                   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
                   95                  100                 105
   Thr Lys Val Glu Ile Lys Arg Thr Val Ala Ala Pro Ser Val Phe
                   110                 115                 120
   Ile Phe Pro Pro Ser Asp Glu Gln Leu Lys Ser Gly Thr Ala Ser
                   125                 130                 135
   Val Val Cys Leu Leu Asn Asn Phe Tyr Pro Arg Glu Ala Lys Val
                   140                 145                 150
   Gln Trp Lys Val Asp Asn Ala Leu Gln Ser Gly Asn Ser Gln Glu
                   155                 160                 165
   Ser Val Thr Glu Gln Asp Ser Lys Asp Ser Thr Tyr Ser Leu Ser
                   170                 175                 180
   Ser Thr Leu Thr Leu Ser Lys Ala Asp Tyr Glu Lys His Lys Val
                   185                 190                 195
   Tyr Ala Cys Glu Val Thr His Gln Gly Leu Ser Ser Pro Val Thr
                   200                 205                 210
   Lys Ser Phe Asn Arg Gly Glu Cys
                   215
<210> 177
   <211> 446
   <212> PRT
   <213> Artificial sequence
<220>
   <223> Heavy chain of huMA79bv.17
<400> 177
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Tyr Thr Phe Ser
                   20                  25                  30
   Ser Tyr Trp Ile Glu Trp Val Arg Gln Ala Pro Gly Lys Gly Leu
                   35                  40                  45
   Glu Trp Ile Gly Glu Ile Leu Pro Gly Gly Gly Asp Thr Asn Tyr
                   50                  55                  60
   Asn Glu Ile Phe Lys Gly Arg Ala Thr Phe Ser Ala Asp Thr Ser
                   65                  70                  75
   Lys Asn Thr Ala Tyr Leu Gln Met Asn Ser Leu Arg Ala Glu Asp
                   80                  85                  90
   Thr Ala Val Tyr Tyr Cys Thr Arg Arg Val Pro Val Tyr Phe Asp
                   95                  100                 105
   Tyr Trp Gly Gln Gly Thr Leu Val Thr Val Ser Ser Ala Ser Thr
                   110                 115                 120
   Lys Gly Pro Ser Val Phe Pro Leu Ala Pro Ser Ser Lys Ser Thr
                   125                 130                 135
   Ser Gly Gly Thr Ala Ala Leu Gly Cys Leu Val Lys Asp Tyr Phe
   140 145 150
   Pro Glu Pro Val Thr Val Ser Trp Asn Ser Gly Ala Leu Thr Ser
   155 160 165
   Gly Val His Thr Phe Pro Ala Val Leu Gln Ser Ser Gly Leu Tyr
   170 175 180
   Ser Leu Ser Ser Val Val Thr Val Pro Ser Ser Ser Leu Gly Thr
   185 190 195
   Gln Thr Tyr Ile Cys Asn Val Asn His Lys Pro Ser Asn Thr Lys
   200 205 210
   Val Asp Lys Lys Val Glu Pro Lys Ser Cys Asp Lys Thr His Thr
   215 220 225
   Cys Pro Pro Cys Pro Ala Pro Glu Leu Leu Gly Gly Pro Ser Val
   230 235 240
   Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp
   260 265 270
   Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn
   305 310 315
   Gly Lys Glu Tyr Lys Cys Lys Val Ser Asn Lys Ala Leu Pro Ala
   320 325 330
   Pro Ile Glu Lys Thr Ile Ser Lys Ala Lys Gly Gln Pro Arg Glu
   335 340 345
   Pro Gln Val Tyr Thr Leu Pro Pro Ser Arg Glu Glu Met Thr Lys
   350 355 360
   Asn Gln Val Ser Leu Thr Cys Leu Val Lys Gly Phe Tyr Pro Ser
   365 370 375
   Asp Ile Ala Val Glu Trp Glu Ser Asn Gly Gln Pro Glu Asn Asn
   380 385 390
   Tyr Lys Thr Thr Pro Pro Val Leu Asp Ser Asp Gly Ser Phe Phe
   395 400 405
   Leu Tyr Ser Lys Leu Thr Val Asp Lys Ser Arg Trp Gln Gln Gly
   410 415 420
   Asn Val Phe Ser Cys Ser Val Met His Glu Ala Leu His Asn His
   425 430 435
   Tyr Thr Gln Lys Ser Leu Ser Leu Ser Pro Gly
   440 445
<210> 178
   <211> 218
   <212> PRT
   <213> Artificial sequence
<220>
   <223> Light chain of huMA79bv.18
<400> 178
   Asp Ile Gln Leu Thr Gln Ser Pro Ser Ser Leu Ser Ala Ser Val
   1               5                   10                  15
   Gly Asp Arg Val Thr Ile Thr Cys Lys Ala Ser Gln Ser Val Asp
                   20                  25                  30
   Tyr Asp Gly Asp Ser Phe Leu Asn Trp Tyr Gln Gln Lys Pro Gly
                   35                  40                  45
   Lys Ala Pro Lys Leu Leu Ile Tyr Ala Ala Ser Asn Leu Glu Ser
                   50                  55                  60
   Gly Val Pro Ser Arg Phe Ser Gly Ser Gly Ser Gly Thr Asp Phe
                   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
                   95                  100                 105
   Thr Lys Val Glu Ile Lys Arg Thr Val Ala Ala Pro Ser Val Phe
                   110                 115                 120
   Ile Phe Pro Pro Ser Asp Glu Gln Leu Lys Ser Gly Thr Ala Ser
                   125                 130                 135
   Val Val Cys Leu Leu Asn Asn Phe Tyr Pro Arg Glu Ala Lys Val
                   140                 145                 150
   Gln Trp Lys Val Asp Asn Ala Leu Gln Ser Gly Asn Ser Gln Glu
                   155                 160                 165
   Ser Val Thr Glu Gln Asp Ser Lys Asp Ser Thr Tyr Ser Leu Ser
                   170                 175                 180
   Ser Thr Leu Thr Leu Ser Lys Ala Asp Tyr Glu Lys His Lys Val
                   185                 190                 195
   Tyr Ala Cys Glu Val Thr His Gln Gly Leu Ser Ser Pro Val Thr
                   200                 205                 210
   Lys Ser Phe Asn Arg Gly Glu Cys
                   215
<210> 179
   <211> 446
   <212> PRT
   <213> Artificial sequence
<220>
   <223> Heavy chain of huMA79bv.18
<400> 179
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Tyr Thr Phe Ser
                   20                  25                  30
   Ser Tyr Trp Ile Glu Trp Val Arg Gln Ala Pro Gly Lys Gly Leu
                   35                  40                  45
   Glu Trp Ile Gly Glu Ile Leu Pro Gly Gly Gly Asp Thr Asn Tyr
                   50                  55                  60
   Asn Glu Ile Phe Lys Gly Arg Ala Thr Phe Ser Ala Asp Thr Ser
                   65                  70                  75
   Lys Asn Thr Ala Tyr Leu Gln Met Asn Ser Leu Arg Ala Glu Asp
                   80                  85                  90
   Thr Ala Val Tyr Tyr Cys Thr Arg Arg Val Pro Ile Arg Leu Asp
                   95                  100                 105
   Tyr Trp Gly Gln Gly Thr Leu Val Thr Val Ser Ser Ala Ser Thr
                   110                 115                 120
   Lys Gly Pro Ser Val Phe Pro Leu Ala Pro Ser Ser Lys Ser Thr
                   125                 130                 135
   Ser Gly Gly Thr Ala Ala Leu Gly Cys Leu Val Lys Asp Tyr Phe
                   140                 145                 150
   Pro Glu Pro Val Thr Val Ser Trp Asn Ser Gly Ala Leu Thr Ser
                   155                 160                 165
   Gly Val His Thr Phe Pro Ala Val Leu Gln Ser Ser Gly Leu Tyr
                   170                 175                 180
   Ser Leu Ser Ser Val Val Thr Val Pro Ser Ser Ser Leu Gly Thr
                   185                 190                 195
   Gln Thr Tyr Ile Cys Asn Val Asn His Lys Pro Ser Asn Thr Lys
                   200                 205                 210
   Val Asp Lys Lys Val Glu Pro Lys Ser Cys Asp Lys Thr His Thr
                   215                 220                 225
   Cys Pro Pro Cys Pro Ala Pro Glu Leu Leu Gly Gly Pro Ser Val
                   230                 235                 240
   Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp
                   260                 265                 270
   Pro Glu Val Lys Phe Asn Trp Tyr Val Asp Gly Val Glu Val His
                   275                 280                 285
   Asn Ala Lys Thr Lys Pro Arg Glu Glu Gln Tyr Asn Ser Thr Tyr
                   290                 295                 300
   Arg Val Val Ser Val Leu Thr Val Leu His Gln Asp Trp Leu Asn
                   305                 310                 315
   Gly Lys Glu Tyr Lys Cys Lys Val Ser Asn Lys Ala Leu Pro Ala
                   320                 325                 330
   Pro Ile Glu Lys Thr Ile Ser Lys Ala Lys Gly Gln Pro Arg Glu
                   335                 340                 345
   Pro Gln Val Tyr Thr Leu Pro Pro Ser Arg Glu Glu Met Thr Lys
                   350                 355                 360
   Asn Gln Val Ser Leu Thr Cys Leu Val Lys Gly Phe Tyr Pro Ser
                   365                 370                 375
   Asp Ile Ala Val Glu Trp Glu Ser Asn Gly Gln Pro Glu Asn Asn
                   380                 385                 390
   Tyr Lys Thr Thr Pro Pro Val Leu Asp Ser Asp Gly Ser Phe Phe
                   395                 400                 405
   Leu Tyr Ser Lys Leu Thr Val Asp Lys Ser Arg Trp Gln Gln Gly
                   410                 415                 420
   Asn Val Phe Ser Cys Ser Val Met His Glu Ala Leu His Asn His
                   425                 430                 435
   Tyr Thr Gln Lys Ser Leu Ser Leu Ser Pro Gly
                   440                 445
<210> 180
   <211> 218
   <212> PRT
   <213> Artificial sequence
<220>
   <223> Light chain of huMA79bv.28
<400> 180
   Asp Ile Gln Leu Thr Gln Ser Pro Ser Ser Leu Ser Ala Ser Val
   1               5                   10                  15
   Gly Asp Arg Val Thr Ile Thr Cys Lys Ala Ser Gln Ser Val Asp
                   20                  25                  30
   Tyr Glu Gly Asp Ser Phe Leu Asn Trp Tyr Gln Gln Lys Pro Gly
                   35                  40                  45
   Lys Ala Pro Lys Leu Leu Ile Tyr Ala Ala Ser Asn Leu Glu Ser
                   50                  55                  60
   Gly Val Pro Ser Arg Phe Ser Gly Ser Gly Ser Gly Thr Asp Phe
                   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
                   95                  100                 105
   Thr Lys Val Glu Ile Lys Arg Thr Val Ala Ala Pro Ser Val Phe
                   110                 115                 120
   Ile Phe Pro Pro Ser Asp Glu Gln Leu Lys Ser Gly Thr Ala Ser
                   125                 130                 135
   Val Val Cys Leu Leu Asn Asn Phe Tyr Pro Arg Glu Ala Lys Val
                   140                 145                 150
   Gln Trp Lys Val Asp Asn Ala Leu Gln Ser Gly Asn Ser Gln Glu
                   155                 160                 165
   Ser Val Thr Glu Gln Asp Ser Lys Asp Ser Thr Tyr Ser Leu Ser
                   170                 175                 180
   Ser Thr Leu Thr Leu Ser Lys Ala Asp Tyr Glu Lys His Lys Val
                   185                 190                 195
   Tyr Ala Cys Glu Val Thr His Gln Gly Leu Ser Ser Pro Val Thr
                   200                 205                 210
   Lys Ser Phe Asn Arg Gly Glu Cys
                   215
<210> 181
   <211> 446
   <212> PRT
   <213> Artificial sequence
<220>
   <223> Heavy chain of huMA79bv.28
<400> 181
   Glu Val Gln Leu Val Glu Ser Gly Gly Gly Leu Val Gln Pro Gly
   1               5                   10                  15
   Gly Ser Leu Arg Leu Ser Cys Ala Ala Ser Gly Tyr Thr Phe Ser
                   20                  25                  30
   Ser Tyr Trp Ile Glu Trp Val Arg Gln Ala Pro Gly Lys Gly Leu
                   35                  40                  45
   Glu Trp Ile Gly Glu Ile Leu Pro Gly Gly Gly Asp Thr Asn Tyr
                   50                  55                  60
   Asn Glu Ile Phe Lys Gly Arg Ala Thr Phe Ser Ala Asp Thr Ser
                   65                  70                  75
   Lys Asn Thr Ala Tyr Leu Gln Met Asn Ser Leu Arg Ala Glu Asp
                   80                  85                  90
   Thr Ala Val Tyr Tyr Cys Thr Arg Arg Val Pro Ile Arg Leu Asp
                   95                  100                 105
   Tyr Trp Gly Gln Gly Thr Leu Val Thr Val Ser Ser Ala Ser Thr
                   110                 115                 120
   Lys Gly Pro Ser Val Phe Pro Leu Ala Pro Ser Ser Lys Ser Thr
                   125                 130                 135
   Ser Gly Gly Thr Ala Ala Leu Gly Cys Leu Val Lys Asp Tyr Phe
                   140                 145                 150
   Pro Glu Pro Val Thr Val Ser Trp Asn Ser Gly Ala Leu Thr Ser
                   155                 160                 165
   Gly Val His Thr Phe Pro Ala Val Leu Gln Ser Ser Gly Leu Tyr
                   170                 175                 180
   Ser Leu Ser Ser Val Val Thr Val Pro Ser Ser Ser Leu Gly Thr
   185 190 195
   Gln Thr Tyr Ile Cys Asn Val Asn His Lys Pro Ser Asn Thr Lys
   200 205 210
   Val Asp Lys Lys Val Glu Pro Lys Ser Cys Asp Lys Thr His Thr
   215 220 225
   Cys Pro Pro Cys Pro Ala Pro Glu Leu Leu Gly Gly Pro Ser Val
   230 235 240
   Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp
   260 265 270
   Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn
   305 310 315
   Gly Lys Glu Tyr Lys Cys Lys Val Ser Asn Lys Ala Leu Pro Ala
   320 325 330
   Pro Ile Glu Lys Thr Ile Ser Lys Ala Lys Gly Gln Pro Arg Glu
   335 340 345
   Pro Gln Val Tyr Thr Leu Pro Pro Ser Arg Glu Glu Met Thr Lys
   350 355 360
   Asn Gln Val Ser Leu Thr Cys Leu Val Lys Gly Phe Tyr Pro Ser
   365 370 375
   Asp Ile Ala Val Glu Trp Glu Ser Asn Gly Gln Pro Glu Asn Asn
   380 385 390
   Tyr Lys Thr Thr Pro Pro Val Leu Asp Ser Asp Gly Ser Phe Phe
   395 400 405
   Leu Tyr Ser Lys Leu Thr Val Asp Lys Ser Arg Trp Gln Gln Gly
   410 415 420
   Asn Val Phe Ser Cys Ser Val Met His Glu Ala Leu His Asn His
   425 430 435
   Tyr Thr Gln Lys Ser Leu Ser Leu Ser Pro Gly
   440 445
<210> 182
   <211> 218
   <212> PRT
   <213> Artificial sequence
<220>
   <223> Light chain of huMA79bv.32
<400> 182
   Asp Ile Gln Leu Thr Gln Ser Pro Ser Ser Leu Ser Ala Ser Val
   1               5                   10                  15
   Gly Asp Arg Val Thr Ile Thr Cys Lys Ala Ser Gln Ser Val Asp
                   20                  25                  30
   Tyr Ser Gly Asp Ser Phe Leu Asn Trp Tyr Gln Gln Lys Pro Gly
                   35                  40                  45
   Lys Ala Pro Lys Leu Phe Ile Tyr Ala Ala Ser Asn Leu Glu Ser
                   50                  55                  60
   Gly Val Pro Ser Arg Phe Ser Gly Ser Gly Ser Gly Thr Asp Phe
                   65                  70                  75
   Thr Leu Thr Ile Ser Ser Leu Gln Pro Glu Asp Phe Ala Thr Tyr
                   80                  85                  90
   Tyr Cys Gln Gln Ser Asn Glu Asp Pro Leu Thr Phe Gly Gln Gly
                   95                  100                 105
   Thr Lys Val Glu Ile Lys Arg Thr Val Ala Ala Pro Ser Val Phe
                   110                 115                 120
   Ile Phe Pro Pro Ser Asp Glu Gln Leu Lys Ser Gly Thr Ala Ser
                   125                 130                 135
   Val Val Cys Leu Leu Asn Asn Phe Tyr Pro Arg Glu Ala Lys Val
                   140                 145                 150
   Gln Trp Lys Val Asp Asn Ala Leu Gln Ser Gly Asn Ser Gln Glu
                   155                 160                 165
   Ser Val Thr Glu Gln Asp Ser Lys Asp Ser Thr Tyr Ser Leu Ser
                   170                 175                 180
   Ser Thr Leu Thr Leu Ser Lys Ala Asp Tyr Glu Lys His Lys Val
                   185                 190                 195
   Tyr Ala Cys Glu Val Thr His Gln Gly Leu Ser Ser Pro Val Thr
                   200                 205                 210
   Lys Ser Phe Asn Arg Gly Glu Cys
                   215